# EUROPEAN PATENT SPECIFICATION

(11) **EP 3 083 659 B1**
(45) Date of publication and mention of the grant of the patent: **28.07.2021**
(21) Application number: 14824566.5
(22) Date of filing: 19.12.2014
(51) Int. Cl.: C07K 14/005, A61K 39/21

(54) **VIRAL PEPTIDES**
VIRALE PEPTIDE
PEPTIDES VIRAUX

(30) Priority: 19.12.2013 GB 201322574
(43) Date of publication of application: 26.10.2016
(73) Proprietor: Equigerminal SA, 3060-197 Cantanhede (PT)
(72) Inventor: CARVALHO, Isabel Maria Fidalgo dos Santos Silva, P-3000-235 Coimbra (PT); PIRES, Alexandre Simão Vieira, P-3000-235 Coimbra (PT)
(74) Representative: D Young & Co LLP
(86) International application number: PCT/PT2014/000077
(87) International publication number: WO 2015/094001

(56) References cited:
- EP-A2- 0 743 364
- WO-A1-01/24810
- WO-A1-98/32771
- WO-A1-2004/083418
- WO-A1-2009/097692
- WO-A1-2012/102963
- WO-A1-2013/151669
- WO-A1-2013/151671
- WO-A2-02/48345
- WO-A2-02/054081
- WO-A2-02/069691
- WO-A2-2004/081186
- WO-A2-2005/015206
- WO-A2-2005/032495
- WO-A2-2005/100399
- WO-A2-2006/034005
- WO-A2-2006/086111
- WO-A2-2008/054514
- WO-A2-2009/074350
- WO-A2-2012/088513
- JP-A- 2004 361 227
- US-A- 5 693 325
- US-A1- 2005 271 676
- US-A1- 2005 282 744
- US-A1- 2008 263 730
- US-A1- 2011 065 756
- DATABASE EMBL [Online] 9 August 2007 (2007-08-09), XP002736682, retrieved from EBI Database accession no. AC207823
- DATABASE EMBL [Online] 20 October 2006 (2006-10-20), XP002736721, retrieved from EBI Database accession no. CU197709
- DATABASE EMBL [Online] 31 October 2009 (2009-10-31), XP002736722, retrieved from EBI Database accession no. CP001220
- DATABASE EMBL [Online] 6 September 2002 (2002-09-06), XP002736723, retrieved from EBI Database accession no. AC129777
- DATABASE EMBL [Online] 31 January 2013 (2013-01-31), XP002736724, retrieved from EBI Database accession no. JR948040
- DATABASE EMBL [Online] 31 January 2013 (2013-01-31), XP002736725, retrieved from EBI Database accession no. JR949410
- DATABASE EMBL [Online] 23 October 2003 (2003-10-23), XP002736726, retrieved from EBI Database accession no. CG714943
- DATABASE EMBL [Online] 31 January 2013 (2013-01-31), XP002736727, retrieved from EBI Database accession no. JR948186
- DATABASE EMBL [Online] 23 July 2010 (2010-07-23), XP002736728, retrieved from EBI Database accession no. FN566618
- DATABASE UniProt [Online] 1 November 1999 (1999-11-01), XP002736729, Database accession no. Q9W7Z6
- None

## Description

### FIELD OF THE INVENTION

Various embodiments of the present invention generally relate to biotechnology. Further, various embodiments relate to veterinary medicine. In particular, the invention relates to antigenic peptides of equine viruses and uses thereof.

### BACKGROUND OF THE INVENTION

Equine infectious anemia (EIA) or swamp fever is an ancient lentiviral disease of Equids. The Swamp fever or equine infectious anemia is the only retroviral disease known to affect *Equidae.*

The EIAV (Equine infectious anemia virus) retrovirus is known, historically, to be the first viral agent responsible for an animal disease, the Swamp fever in horses.

The disease was first reported in Europe, in France, in 1843 (Lignee, 1843). At the present it is considered to be distributed worldwide. In the last years, several clinical cases of swamp fever were reported by the World Organization for Animal Health (OIE) in the European horse population. However, knowledge about the EIAV European primary isolates sequences is scarce. EIAV sequences are limited to long terminal repeats or gag sequences reported in Greece (Spyrou et al., 2003), Ireland (Mooney J et al., 2006, Quinlivan M et al., 2007), Italy, Romania and Slovenia (Cappeli et al., 2011, Cappomacio et al., 2012, Kuhar et al., 2014). EIAV infections have been reported in the United Kingdom (UK), France, Belgium, Romania, Slovenia, Austria and Germany, but very little is know about the EIAV sequences responsible for these infections. The full length EIAV genome of European strains is restricted to Ireland strains isolated from a 2006 outbreak (Quinlivan M et al., 2013). Nevertheless, even in the continents where the disease is prevalent, reports of EIAV in naturally infected horses or viral sequence variations among primary isolates are limited. Recently some EIAV isolates from Pennsylvania, US and Japan were recovered by *'in vivo'* viral transfer experiments between EIAV seropositive and naïve horses. The retrieved viral sequences showed more diverse and divergent EIAV strains. The identified EIAV strains were detected in horses determined as seropositive by the Coggins test (also known as the AGID or Agar Gel Diffusion test). The AGID test detects antibodies against the major core protein of EIAV (p26).

The ancient origin of this virus, geographical dispersion, persistence over time in countries with monitoring plans, and the ability of EIAV to be highly variable suggest that much remains to be learned about this virus. Means and methods for diagnosis and treatment are thus needed.

Currently, there is no known cure for the disease. Diagnosis, quarantine/isolation or elimination of seropositive animals is the only way to control the disease. The current immunodiagnostic tests for the detection of anti-EIAV antibodies in the serum of horses infected with EIAV utilize the whole virus as an antigen, or viral recombinant proteins.

The OIE official test to diagnose the presence of EIA has been the presence of antibodies specific for the disease in the serum of affected animals using the Coggins or agar gel diffusion test (AGID) (described in U.S. Patent No. 3.929.982 and U.S. Patent No.3.932.601).

In the Coggins test, prepared antigens are used to test serum for the presence of anti-EIAV antibodies. In these assays the used antigens are whole EIAV viral proteins produced in cell lines or p26 recombinant proteins. Although they brought great progress, these immunoassays still do not permit detection of all the serums of subjects infected with EIAV divergent viral strains.

Fidalgo-Carvalho (2008; PhD thesis, University of Porto, Portugal) identified 33 horses that tested positive for EIAV in the immunoblot test, but that tested negative in the Coggins test and commercial ELISAs directed to the EIAV core protein.

Recently Issel and colleagues (2013) have shown the fragilities of the Coggins test, and documented the immunoblot as the more reliable test for determining EIAV seropositiveness. When first deployed, the immunoblot test was recommended as a useful adjunct for diagnosis because samples from EIAV- infected equids with low levels of anti-p26 antibodies (that may be falsely reported as negative on the AGID test) generally had higher levels of reactivity against the envelope proteins.

The immunoblot test uses gradient purified EIAV viral particles obtained from cell culture supernatants of equine cell lines infected with EIAV_{PV}, a biological EIAV isolate. In the immunoblot test linear epitopes of all EIAV viral proteins, gag derived (p26, p15 and p11), envelope proteins (gp90 and gp45), and accessory proteins can be detected by specific sera antibodies.

The available ELISA tests have been validated by comparing results on reference and field samples with those in AGID tests, with cut-off points adjusted so that results are in agreement to a high degree of significant statistical accuracy. The majority of commercially available test kits in AGID and ELISA formats use the p26 antigen only. Two ELISA kits also incorporate a synthetic antigen determinant of the transmembrane protein gp45.

### STATEMENT OF INVENTION

The present invention is based on the use of peptides for therapeutic and/or diagnostics purposes of an EIAV and/or NEV infection of horses. The inventors used viral peptides (identified by mass spectrometry) from EIAV seropositive horses. The viral peptides were obtained from viral supernatants which originated from macrophage cell cultures isolated from EIAV seropositive horses. EIAV seropositive horses were identified by immunoblot techniques, previously these horses were considered to be EIAV negative in commercially available ELISAs and in the Coggins test. The present invention provides peptide-based immunodiagnostics techniques with the similar sensitivity and specificity of immunoblot techniques. Further, the invention provides tools for the detection of divergent EIAV and/or NEV strains or subtypes.

Advantageously, the diagnostic methods of the present invention are capable of identifying EIAV and/or NEV infection in animals which current diagnostic methods do not detect an infection. Advantageously, this means that more animals (e.g. horses) with EIAV and/or NEV infection (i.e. seropositive animals) can be identified.

The present invention provides a composition comprising one or more peptides selected from the group consisting of:
SRLLESRGPTTSEK (SEQ ID NO: 1);
TLKEVLGGEAAVR (SEQ ID NO: 2);
PCRSKNILPV (SEQ ID NO: 3);
DPCVHSVDVLLR (SEQ ID NO: 4);
supernatants of equine cell lines infected with EIAV_{PV}, a biological EIAV isolate. In the immunoblot test linear epitopes of all EIAV viral proteins, gag derived (p26, p15 and p11), envelope proteins (gp90 and gp45), and accessory proteins can be detected by specific sera antibodies.

The available ELISA tests have been validated by comparing results on reference and field samples with those in AGID tests, with cut-off points adjusted so that results are in agreement to a high degree of significant statistical accuracy. The majority of commercially available test kits in AGID and ELISA formats use the p26 antigen only. Two ELISA kits also incorporate a synthetic antigen determinant of the transmembrane protein gp45.

### STATEMENT OF INVENTION

In one aspect, the present invention is composition comprising one or more peptides selected from the group consisting of SEQ ID NOs: 18 and 20 and variants thereof having at least 80% identity to SEQ ID NO: 20; and/or comprising one or more polynucleotide sequences selected from the group consisting of SEQ ID NOs: 95 and 152, and variants thereof having at least 80% identity to SEQ ID NO: 95.

In a further aspect, the present invention is a polynucleotide sequence encoding the fusion protein of the composition of the present invention.

In a further aspect the present invention is a vector that encodes one or more peptides of the composition of the present invention, or a vector comprising one or more polynucleotide sequences of the present invention or of the composition of the present invention.

In a further aspect the present invention is an antibody that binds to SEQ ID NO: 20.

In a further aspect the present invention is a pharmaceutical composition comprising: one or more peptides of the composition of the present invention; or one or more polynucleotide sequences of the present invention or of the composition of the present invention; or one or more vectors of the present invention; or one or more antibodies of the present invention; and a pharmaceutically acceptable carrier, vehicle, diluent or excipient.

In a further aspect the present invention is a kit comprising: a composition of the present invention; and/or a vector of the present invention; and/or an antibody of the present invention; and/or a pharmaceutical composition of the present invention; and optionally instructions for administration to an animal.

In a further aspect the present invention is a diagnostic method comprising providing a sample obtained from an animal and determining the presence or absence in said sample of antibodies that bind to one or more peptides selected from the group consisting of SEQ ID NOs: 18 and 20; and/or determining the presence or absence in said sample of one or more of said peptides; and/or determining the presence or absence in said sample of one or more polynucleotide sequences of the present invention or of the composition of the present invention, preferably wherein: said method determines the presence or absence of an EIAV and/or NEV in said animal, and/or the diagnostic method determines the viral titre of an EIAV and/or NEV in said animal; wherein NEV is deposited under ECAAC accession number 14120201; preferably wherein said animal is an equine, more preferably a horse.

In a further aspect the present invention is the use of:
(a) a composition comprising one or more peptides selected from the group consisting of SEQ ID NOs: 18 and 20 and variants thereof having at least 80% identity to SEQ ID NO: 20; and/or comprising one or more polynucleotide sequences selected from the group consisting of SEQ ID NOs: 95 and 152, and variants thereof having at least 80% identity to SEQ ID NO: 95, preferably wherein said one or more peptides selected from the group consisting of SEQ ID Nos: 18 and 20 are linked together as a fusion protein; and/or
(b) a vector encoding said one or more peptides or said fusion protein; or comprising said one or more polynucleotide sequences; and/or
(c) a pharmaceutical composition comprising said one or more peptides and/or said fusion protein and/or said one or more polynucleotide sequences and/or said vector and/or an antibody that binds to said one or more peptides and a pharmaceutically acceptable carrier, vehicle, diluent or excipient; and/or
(d) an antibody that binds to said one or more peptides
in a kit or assay for the in vitro or ex vivo diagnosis an infection by an EIAV and/or NEV in an animal; wherein NEV is deposited under ECAAC accession number 14120201.

The present invention is based on the use of peptides for therapeutic and/or diagnostics purposes of an EIAV and/or NEV infection of horses. The inventors used viral peptides (identified by mass spectrometry) from EIAV seropositive horses. The viral peptides were obtained from viral supernatants which originated from macrophage cell cultures isolated from EIAV seropositive horses. EIAV seropositive horses were identified by immunoblot techniques, previously these horses were considered to be EIAV negative in commercially available ELISAs and in the Coggins test. Described herein are provides peptide-based immunodiagnostics techniques with the similar sensitivity and specificity of immunoblot techniques. Further, described herein are tools for the detection of divergent EIAV and/or NEV strains or subtypes.

Advantageously, the diagnostic methods of the present invention are capable of identifying EIAV and/or NEV infection in animals which current diagnostic methods do not detect an infection. Advantageously, this means that more animals (e.g. horses) with EIAV and/or NEV infection (i.e. seropositive animals) can be identified.

As described herein are a composition comprising one or more peptides selected from the group consisting of:
SRLLESRGPTTSEK (SEQ ID NO: 1);
TLKEVLGGEAAVR (SEQ ID NO: 2);
PCRSKNILPV (SEQ ID NO: 3);
DPCVHSVDVLLR (SEQ ID NO: 4);
STFPPTPV (SEQ ID NO: 5);
NAPLLSKVSP (SEQ ID NO: 6);
MAQCIVNR (SEQ ID NO: 7);
KPNVEGRYGLSRSETNK (SEQ ID NO: 8);
QQGPEAPLPSLQVAEVPK (SEQ ID NO: 9);
PHAGSTQTEWPK (SEQ ID NO: 10);
PIQINACK (SEQ ID NO: 11);
GGMRESWDGGQV (SEQ ID NO: 12);
ESLVSKGFR (SEQ ID NO: 13);
CSLVLGEMQIKRIR (SEQ ID NO: 14);
LDKWEKIR (SEQ ID NO 15);
QMMIAASEK (SEQ ID NO 16);
QPSLPTGSEELK (SEQ ID NO 17);
EALDKIEEIQNKNKQK (SEQ ID NO 18);
PPIPVGGIYK (SEQ ID NO 19);
QEQNPPPSVSLRSLFGNDPL(SEQ ID NO 20);
EKIEQLR (SEQ ID NO 21);
DLLLIVAR (SEQ ID NO 22);
IVELLGR (SEQ ID NO 23);
IWGNVTWMEWER (SEQ ID NO 24);
LKDLFPNK(SEQ ID NO 25);
AKLEESFPGK (SEQ ID NO 26);
NGSLAGESIIIR (SEQ ID NO 27);
NITFNSSAGGDLEIT (SEQ ID NO 28);
AVILLLDRLR(SEQ ID NO 29);
GPGIHIGKR (SEQ ID NO 30);
VIICSASK (SEQ ID NO 31);
ESFPNK (SEQ ID NO 32);
FGNKTTIIFTK (SEQ ID NO 33);
LLNGSLAGESIIIR (SEQ ID NO 34);
VNVSVTNNNTTTNV (SEQ ID NO 35);
AILHILRR (SEQ ID NO 36);
DLLALDK (SEQ ID NO 37);
IGCQHSRIGITLPR (SEQ ID NO 38);
TLQYLALTALVTPK (SEQ ID NO 39);
PTTPVTPAPGVGEISKELAQGK (SEQ ID NO 40);
WSSALQYLIPR (SEQ ID NO 41);
PVWAEPVK (SEQ ID NO 42);
VKGNDLLK (SEQ ID NO 43);
EDLNLQDWK (SEQ ID NO 44);
LFVSVLQR (SEQ ID NO 45);
AAEQKASPPSLTPK (SEQ ID NO 46);
PLSLPLKI (SEQ ID NO 47);
FPSIVGRPR (SEQ ID NO 48);
IWHHTFYNELR (SEQ ID NO 49);
MTQIMFETF (SEQ ID NO 50);
EEEVAALVIDNGSGMCK (SEQ ID NO 51);
VAPEEHPVLLTEAPLNPK (SEQ ID NO 52);
AVFPSIVGRPR (SEQ ID NO 53);
YPIEHGIVTNWDDMEK (SEQ ID NO 54);
AVFPSIVGR (SEQ ID NO 55);
IGRKDAERQLLSPGNAR (SEQ ID NO 56);
DSYVGDEAQSKR (SEQ ID NO 57);
RGILTLK (SEQ ID NO 58);
WGIAHTTGIPGNSQGQAMVER (SEQ ID NO 59);
HLVDQLIRDLK (SEQ ID NO 60);
YEQLQLQAR (SEQ ID NO 61);
TITLEVEPSDTIENVK (SEQ ID NO 62);
INRELLK (SEQ ID NO 63);
QTIIPTNKDVDEK (SEQ ID NO 64);
KNYGKLDK (SEQ ID NO 65);
TTISFSK (SEQ ID NO 66);
QDRTTISFSK (SEQ ID NO 67);
AQGRAIAHK (SEQ ID NO 68);
MAQTQLVPVK (SEQ ID NO 69);
QELIPPCK (SEQ ID NO 70);
DIVLLENGK (SEQ ID NO 71);
LPPLSILK (SEQ ID NO 72);
IFLINLAFLIK (SEQ ID NO 73);
NRLEILK (SEQ ID NO 74);
ADDVAVLQDALGR (SEQ ID NO 75);
LNKSLEQLR (SEQ ID NO 76); and
variants, homologues, fragments or derivatives thereof having at least 75%, 80%, 85%, 90%, 95%, 97%, 98%, or 99% identity to any one of SEQ ID NOs 1 to 76; and/or comprising one or more polynucleotide sequences encoding one or more of the peptides;
and/or comprising one or more polynucleotide sequences selected from the group consisting of:
AGCAGGCTGCTGGAGAGCAGGGGCCCCACCACCAGCGAGAAG (SEQ ID NO 77);
ACCCTGAAAGAGGTTCTTGGTGGTGAAGCAGCCGTGCGC (SEQ ID NO 78);
CCCTGCCGGAGCAAGAACATCCTGCCCGTG (SEQ ID NO 79);
GACCCCTGCGTGCACAGCGTGGACGTGCTGCTGAGG (SEQ ID NO 80);
TCCACATTTCCTCCCACTCCCGTC (SEQ ID NO 81);
AATGCCCCATTACTCTCAAAAGTGTCCCCA (SEQ ID NO 82);
ATGGCACAGTGTATCGTTAACCGC (SEQ ID NO 83);
AAGCCCAACGTGGAGGGCAGGTACGGCCTGAGCAGGAGCGAGACCAACAAG(S EQ ID NO 84); 
CAGCAAGGCCCAGAGGCGCCACTGCCCAGCCTGCAGGTTGCAGAAGTGCCTA
AA (SEQ ID NO 85);
CCCCATGCGGGCTCCACTCAGACAGAGTGGCCCAAA (SEQ ID NO 86);
CCAATACAGATCAATGCTTGCAAA (SEQ ID NO 87);
GGTGGAATGCGTGAGTCATGGGATGGAGGACAAGTT (SEQ ID NO 88);
GAATCTCTGGTGTCAAAAGGGTTTCGG (SEQ ID NO 89);
TGCTCATTAGTCCTTGGGGAGATGCAAATCAAA (SEQ ID NO 90);
CTGGACAAGTGGGAGAAGATCAGG (SEQ ID NO 91);
CAGATGATGATCGCCGCCAGCGAGAAG(SEQ ID NO 92);
CAGCCCAGCCTGCCCACCGGCAGCGAGGAGCTGAAG (SEQ ID NO 93);
GAGGCCCTGGACAAGATCGAGGAGATCCAGAACAAGAACAAGCAGAAG (SEQ ID NO 152);
CCCCCCATCCCCGTGGGCGGCATCTACAAG (SEQ ID NO 94); 
CAGGAGCAGAACCCCCCCCCAGCGTGAGCCTGAGGAGCCTGTTCGGCAACG
ACCCCCTG(SEQ ID NO 95);
GAGAAGATCGAGCAGCTGAGG (SEQ ID NO 96);
GACCTGCTGCTGATCGTGGCCAGG (SEQ ID NO 97);
ATCGTGGAGCTGCTGGGCAGG (SEQ ID NO 98);
ATCTGGGGCAACGTGACCTGGATGGAGTGGGAGAGG (SEQ ID NO 99);
CTGAAGGACCTGTTCCCCAACAAG (SEQ ID NO 100);
GCCAAGCTGGAGGAGAGCTTCCCCGGCAAG (SEQ ID NO 101);
AACGGCAGCCTGGCCGGCGAGAGCATCATCATCAGG (SEQ ID NO 102);
AACATCACCTTCAACAGCAGCGCCGGCGGCGACCTGGAGATCACC (SEQ ID NO 103);
GCCGTGATCCTGCTGCTGGACAGGCTGAGG (SEQ ID NO 104);
GGCCCCGGCATCCACATCGGCAAGAGG (SEQ ID NO 105);
GTGATCATCTGCAGCGCCAGCAAG (SEQ ID NO 106);
GAGAGCTTCCCCAACAAG (SEQ ID NO 107);
TTCGGCAACAAGACCACCATCATCTTCACCAAG (SEQ ID NO 108);
CTGCTGAACGGCAGCCTGGCCGGCGAGAGCATCATCATCAGG (SEQ ID NO 109);
GTGAACGTGAGCGTGACCAACAACAACACCACCACCAACGTG (SEQ ID NO 110);
GCCATCCTGCACATCCTGAGGAGG (SEQ ID NO 111);
GACCTGCTGGCCCTGGACAAG (SEQ ID NO 112);
ATCGGCTGCCAGCACAGCAGGATCGGCATCACCCTGCCCAGG (SEQ ID NO 113);
ACCCTGCAGTACCTGGCCCTGACCGCCCTGGTGACCCCCAAG (SEQ ID NO 114); 
CCCACCACCCCCGTGACCCCCGCCCCCGGCGTGGGCGAGATCAGCAAGGAGC
TGGCCCAGGGCAAG (SEQ ID NO 115);
GTGGTGAGCAGCGCCCTGCAGTACCTGATCCCCAGG (SEQ ID NO 116);
CCCGTGTGGGCCGAGCCCGTGAAG (SEQ ID NO 117);
GTGAAGGGCAACGACCTGCTGAAG (SEQ ID NO 118);
GAGGACCTGAACCTGCAGGACTGGAAG (SEQ ID NO 119);
CTGTTCGTGAGCGTGCTGCAGAGG (SEQ ID NO 120);
GCCGCCGAGCAGAAGGCCAGCCCCCCCAGCCTGACCCCCAAG (SEQ ID NO 121);
CCCCTGAGCCTGCCCCTGAAGATC (SEQ ID NO 122);
TTCCCCAGCATCGTGGGCAGGCCCAGG (SEQ ID NO 123);
ATCTGGCACCACACCTTCTACAACGAGCTGAGG (SEQ ID NO 124);
ATGACCCAGATCATGTTCGAGACCTTC (SEQ ID NO 125);
GAGGAGGAGGTGGCCGCCCTGGTGATCGACAACGGCAGCGGCATGTGCAAG (SEQ ID NO 126); 
GTGGCCCCCGAGGAGCACCCCGTGCTGCTGACCGAGGCCCCCCTGAACCCCA
AG (SEQ ID NO 127);
GCCGTGTTCCCCAGCATCGTGGGCAGGCCCAGG (SEQ ID NO 128);
TACCCCATCGAGCACGGCATCGTGACCAACTGGGACGACATGGAGAAG (SEQ ID NO 129);
GCCGTGTTCCCCAGCATCGTGGGCAGG(SEQ ID NO 130);
ATCGGCAGGAAGGACGCCGAGAGGCAGCTGCTGAGCCCCGGCAACGCCAGG (SEQ ID NO 131);
GACAGCTACGTGGGCGACGAGGCCCAGAGCAAGAGG (SEQ ID NO 132);
AGGGGCATCCTGACCCTGAAG (SEQ ID NO 133); 
TGGGGCATCGCCCACACCACCGGCATCCCCGGCAACAGCCAGGGCCAGGCCA
TGGTGGAGAGG (SEQ ID NO 134);
CACCTGGTGGACCAGCTGATCAGGGACCTGAAG (SEQ ID NO 135);
TACGAGCAGCTGCAGCTGCAGGCCAGG (SEQ ID NO 136);
ACCATCACCCTGGAGGTGGAGCCCAGCGACACCATCGAGAACGTGAAG (SEQ ID NO 137);
ATCAACAGGGAGCTGCTGAAG (SEQ ID NO 138);
CAGACCATCATCCCCACCAACAAGGACGTGGACGAGAAG (SEQ ID NO 139);
AAGAACTACGGCAAGCTGGACAAG (SEQ ID NO 140);
ACCACCATCAGCTTCAGCAAG (SEQ ID NO 141);
CAGGACAGGACCACCATCAGCTTCAGCAAG (SEQ ID NO 142);
GCCCAGGGCAGGGCCATCGCCCACAAG (SEQ ID NO 143);
ATGGCCCAGACCCAGCTGGTGCCCGTGAAG (SEQ ID NO 144);
CAGGAGCTGATCCCCCCCTGCAAG (SEQ ID NO 145);
GACATCGTGCTGCTGGAGAACGGCAAG (SEQ ID NO 146);
CTGCCCCCCCTGAGCATCCTGAAG (SEQ ID NO 147);
ATCTTCCTGATCAACCTGGCCTTCCTGATCAAG (SEQ ID NO 148);
AACAGGCTGGAGATCCTGAAG (SEQ ID NO 149);
GCCGACGACGTGGCCGTGCTGCAGGACGCCCTGGGCAGG (SEQ ID NO 150);
CTGAACAAGAGCCTGGAGCAGCTGAGG (SEQ ID NO 151); and
variants, homologues, fragments or derivatives thereof having at least 75%, 80%, 85%, 90%, 95%, 97%, 98%, or 99% identity to any one of SEQ ID NOs 77 to 152.

Described herein is a vector capable of encoding one or more peptides selected from the group consisting of SEQ ID NOs: 1 to 76 and variants, homologues, fragments or derivatives thereof having at least 75%, 80%, 85%, 90%, 95%, 97%, 98%, or 99% identity to any one of SEQ ID NOs 1 to 76.

Described herein is a vector comprising one or more polynucleotide sequences encoding one or more of the peptides selected from the group consisting of SEQ ID NOs: 1 to 76 and variants, homologues, fragments or derivatives thereof having at least 75%, 80%, 85%, 90%, 95%, 97%, 98%, or 99% identity to any one of SEQ ID NOs 1 to 76; and/or comprising one or more polynucleotide sequences selected from the group consisting of SEQ ID NOs 77 to 152 and variants, homologues, fragments or derivatives thereof having at least 75%, 80%, 85%, 90%, 95%, 97%, 98%, or 99% identity to any one of SEQ ID NOs 77 to 152.

Described herein is an antibody capable of binding to one or more peptides selected from the group consisting of SEQ ID NOs: 1 to 76 and variants, homologues, fragments or derivatives thereof having at least 75%, 80%, 85%, 90%, 95%, 97%, 98%, or 99% identity to any one of SEQ ID NOs 1 to 76.

The present invention provides in another aspect, a pharmaceutical composition comprising:
one or more peptides according to the present invention; and/or
one or more polynucleotide sequences according to the present invention; and/or
one or more vectors according to the present invention; and/or
one or more antibodies according to the present invention;
and a pharmaceutically acceptable carrier, vehicle, diluent or excipient.

Described herein is a composition according to the present invention, and/or a vector according to the present invention, and/or antibody according to the present invention, and/or a pharmaceutical composition according to the present invention for use in the treatment or prevention of equine infectious anaemia and/or the treatment or prevention of an infection by an EIAV and/or NEV in an animal.

The present invention provides, in a further aspect, a kit comprising:
a composition according to the present invention; and/or
a vector according to the present invention; and/or
one or more antibodies according to the present invention; and/or
a pharmaceutical composition according to the present invention;
and optionally instructions for administration to an animal.

Described herein is a diagnostic method comprising obtaining a sample from an animal and determining the presence or absence in said sample of antibodies which are capable of binding to one or more peptides selected from the group consisting of SEQ ID NOs: 1 to 76 and variants, homologues, fragments or derivatives thereof having at least 75%, 80%, 85%, 90%, 95%, 97%, 98%, or 99% identity to any one of SEQ ID NOs 1 to 76
and/or determining the presence or absence in said sample of one or more of peptides of the present invention;
and/or determining the presence or absence in said sample of one or more polynucleotide sequence of the present invention.

Described herein is a kit comprising one or more peptides selected from the group consisting of SEQ ID NOs: 1 to 76 and variants, homologues, fragments or derivatives thereof having at least 75%, 80%, 85%, 90%, 95%, 97%, 98%, or 99% identity to any one of SEQ ID NOs 1 to 76 and optionally instructions for determining the presence or absence in an animal sample of antibodies capable of binding to one or more said peptides.

The present invention provides, in a further aspect, a kit comprising one or more antibodies according to the present invention and optionally instructions for determining the presence or absence in an animal sample of peptides capable of binding to said antibodies.

Described herein is an oligonucleotide sequence (e.g. a primer or a probe) comprising or consisting of a polynucleotide sequence capable of encoding one or more of the peptides selected from the group consisting of SEQ ID NOs: 1 to 76 and variants, homologues, fragments or derivatives thereof having at least 75%, 80%, 85%, 90%, 95%, 97%, 98%, or 99% identity to any one of SEQ ID NOs 1 to 76 and/or comprising or consisting of one or more polynucleotide sequences selected from the group consisting of SEQ ID NOs: 77 to 152 and variants, homologues, fragments or derivatives thereof having at least 75%, 80%, 85%, 90%, 95%, 97%, 98%, or 99% identity to any one of SEQ ID NOs 77 to 152.

Described herein is a kit comprising one or more oligonucleotide sequences according to the present invention and optionally instructions for determining the presence or absence in an animal sample of said polynucleotide sequence.

Described herein is a method for controlling EIAV and/or NEV disease in a group of animals comprising the identification of EIAV and/or NEV infection in an animal using the diagnostic method according to the present invention.

Described herein is a method for controlling EIAV and/or NEV disease in a group of animals comprising the identification of EIAV and/or NEV infection in an animal using the diagnostic method according to the present invention and the isolation of an EIAV and/or NEV infected animal from other animals.

Described herein is the use of a composition according to the present invention, and/or a vector according to the present invention, and/or an antibody according to the present invention, and/or a pharmaceutical composition according to the present invention, for the manufacture of a medicament for the treatment or prevention of equine infectious anaemia and/or the treatment or prevention of an infection by an EIAV and/or NEV in an animal.

Described herein is a method for the treatment or prevention of equine infectious anaemia and/or the treatment or prevention of an infection by an EIAV and/or NEV in an animal, wherein said method comprising administering to an animal one or more compositions according to the present invention, and/or one or more vectors according to the present invention, and/or one or more antibodies according to the present invention, and/or one or more pharmaceutical compositions according to the present invention.

### BRIEF DESCRIPTION OF FIGURES

Figure 1. Immunoblot determining equine infectious anemia (EIAV) seropositiveness.
Figure 2. NEV cell lines established from AGID negative horses showed positive reverse transcriptase activity, cytopathic effects and syncytia cell formation.

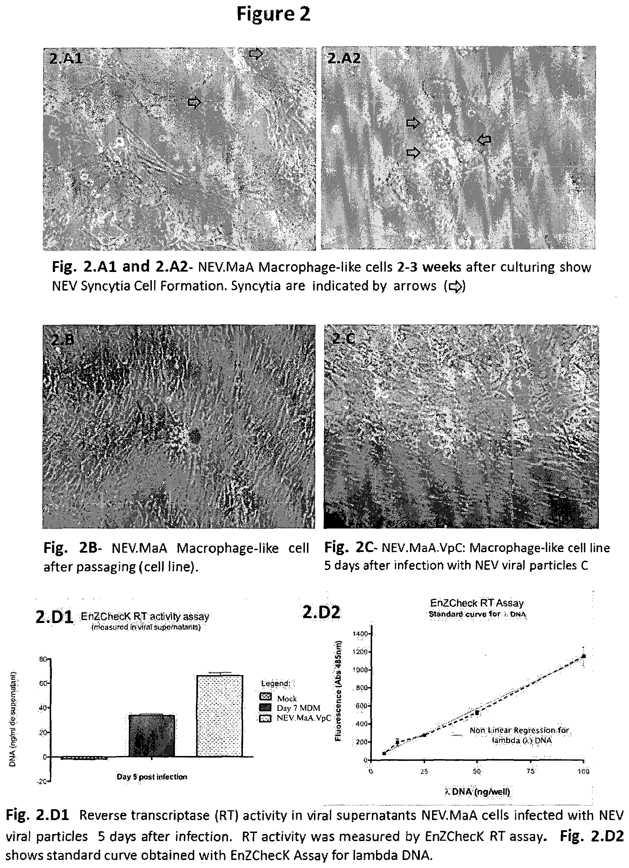

Fig.2.A1 and 2.A2 - NEV.MaA Macrophage-like cells 2,3 weeks after culturing show Syncytia Cell Formation. Syncytia are indicated by arrows. Fig. 2.B - NEV.MaA Macrophage-like cell after passaging (cell line). Fig. 2.C - NEV.MaA.VpC: Macrophage-like cell line 5 days after infection with NEV viral particles C. Fig.2.D1 Reverse transcriptase (RT) activity in viral supernatants NEV.MaA cells infected with NEV viral particles 5 days after infection. RT activity was measured by EnZCheck RT assay. Fig.2.D2 shows standard curve obtained with EnZCheck Assay for lambda DNA.

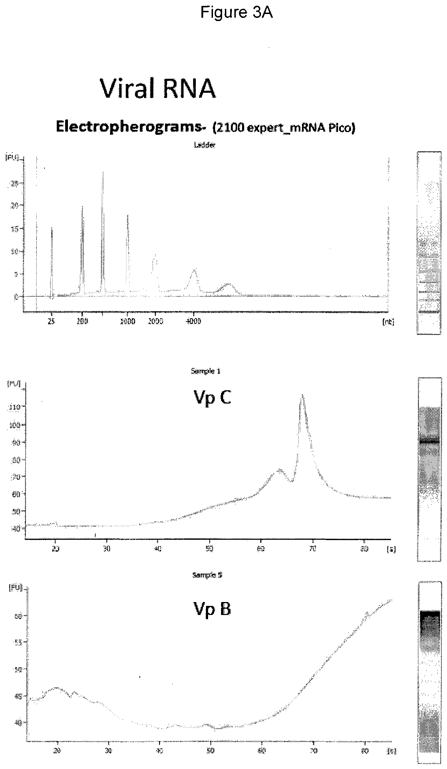

Figure 3A. NEV purified viral particles: viral RNA.

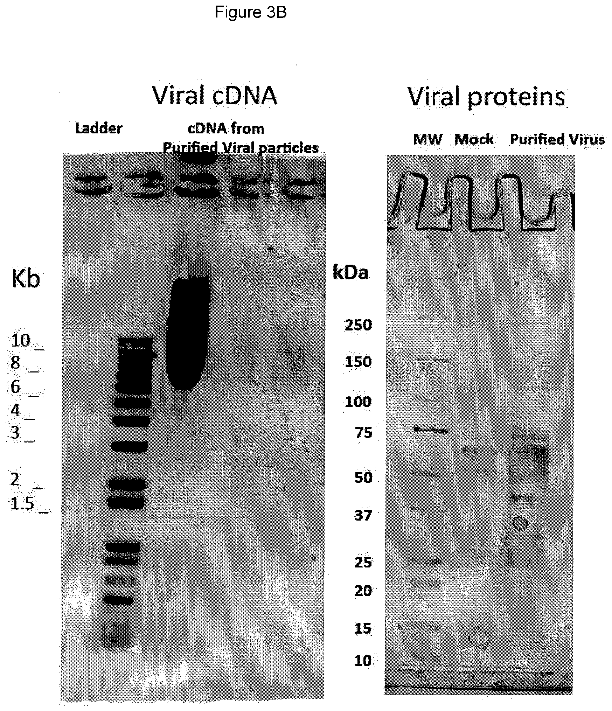

Figure 3B. NEV purified viral particles: cDNA and viral proteins.

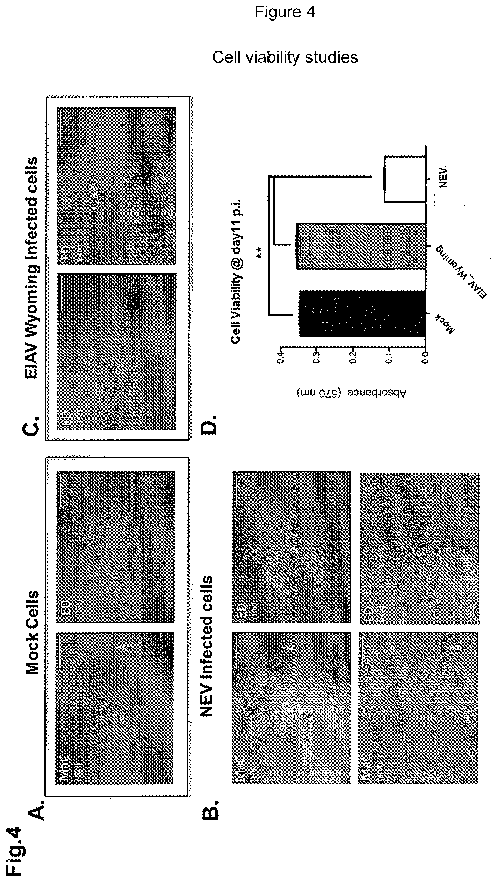

Figure 4. Cytopathic effects (CPE) and viability of infected cells. MaC and ED mock cells (Fig. 4A), MaC or ED cells infected with NEV virus (Fig. 4B) and ED cells infected with EIAV-Wyoming (Fig. 4C) after 7 days of infection. Presto blue cell viability assays of infected ED cells at day 11 post infection (Fig. 4D).

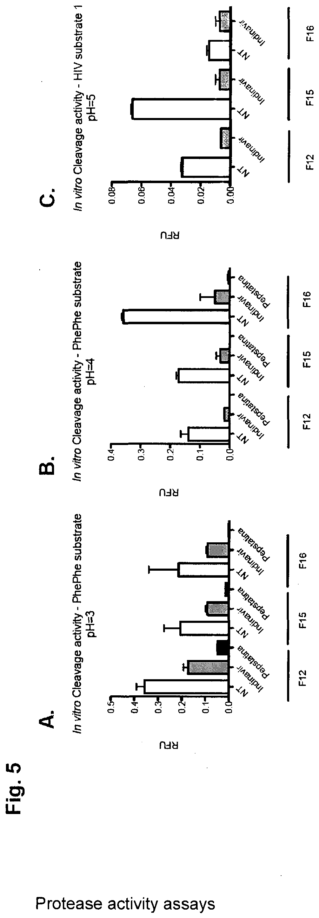

Figure 5. *In vitro* cleavage activity of viral protein fractions analyzed by size-exclusion chromatography. Cleavage of Phe-Phe substrate at pH=3 (Fig. 5A), pH=4 (Fig. 5B) and its inhibition by Indinavir or pepstatin (10 µM). Cleavage of the specific HIV Protease Substrate 1 Arg-Glu(EDANS)-Ser-Gln-Asn-Tyr-Pro-Ile-Val-Gln-Lys(DABCYL)-Arg (SEQ ID NO: 160) and its inhibition by Indinavir (10 µM) (Fig. 5C).

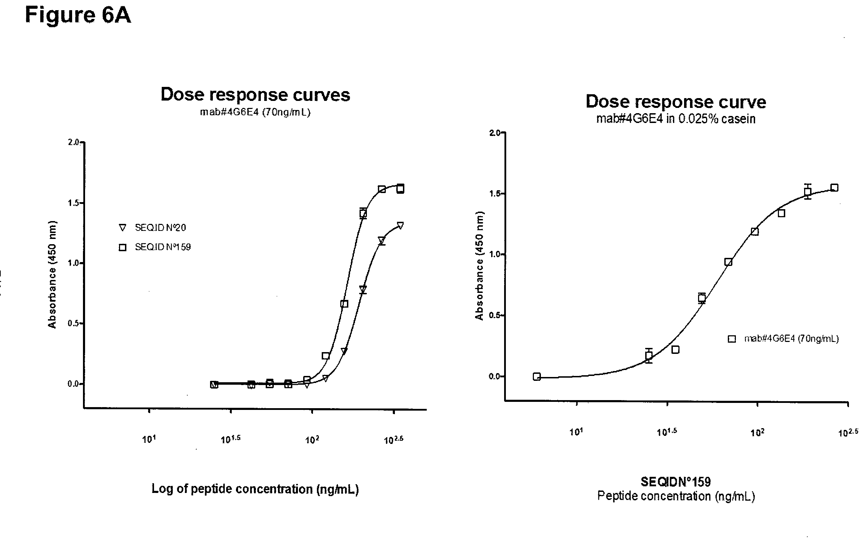

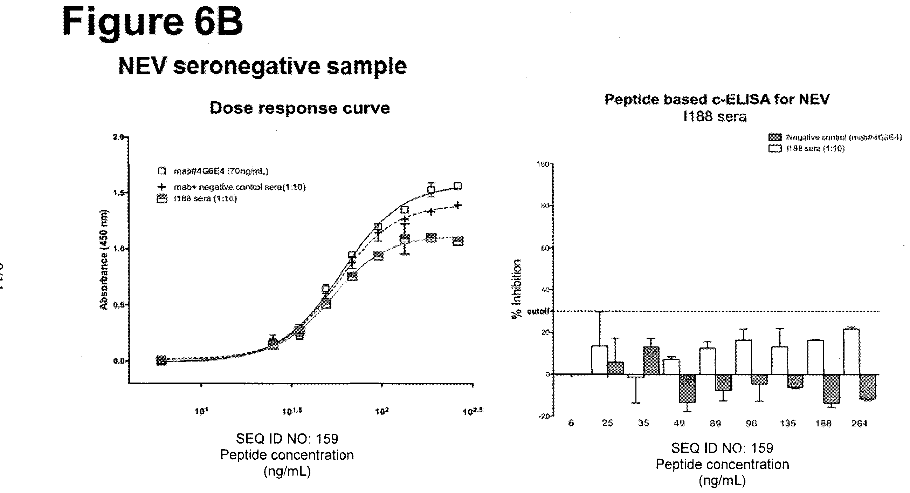

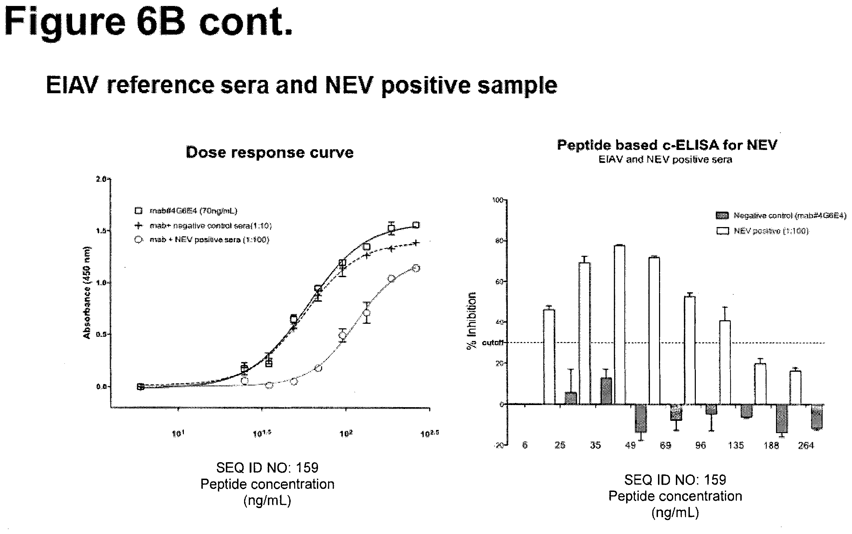

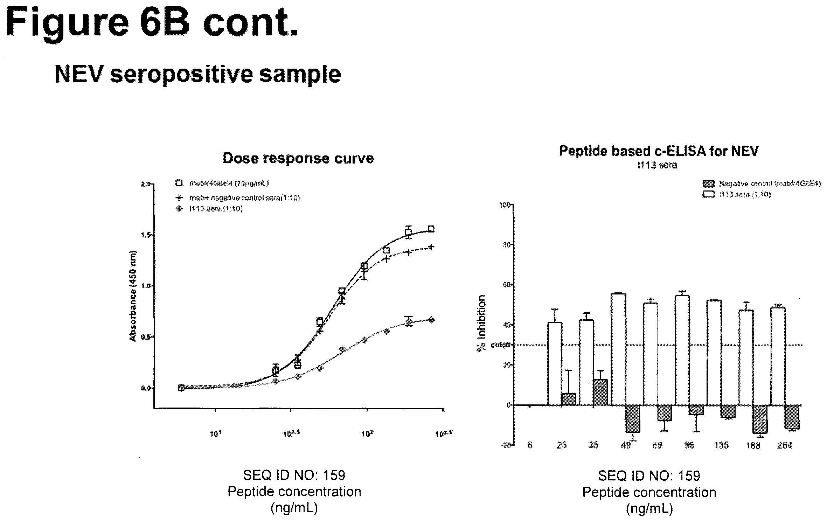

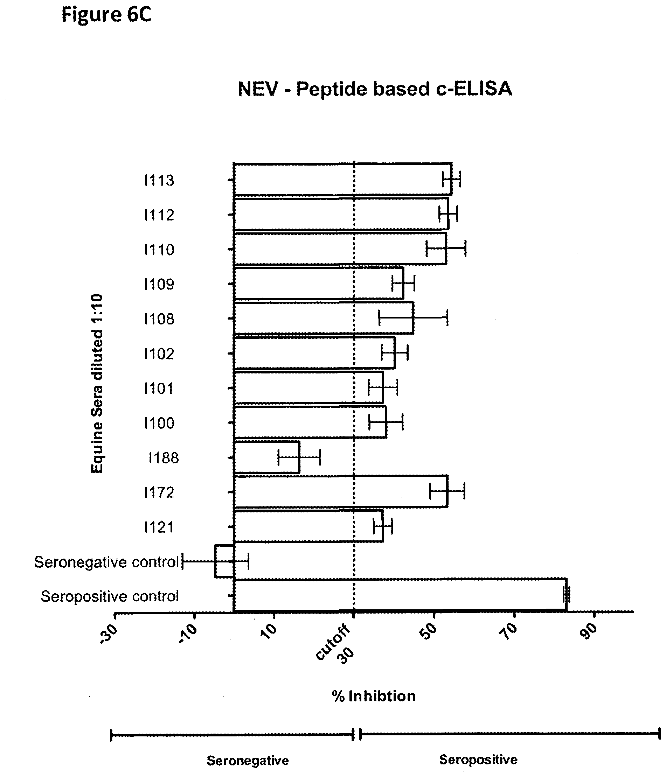

Figure 6. (A) Dose response curves for mab#4G6E4. (B) Dose responses curve and inhibition rates of NEV seronegative and seropositive samples. (C) Representative inhibition rates (%I) of NEV seropositive and one seronegative. Inhibition rates were obtained for a SEQ ID NO: 159 peptide concentration of 96 ng/mL of peptide and mab#4G6E4 IgG concentration of 70ng/mL.

### DETAILED DESCRIPTION

### EIAV

EIAV (equine infectious anaemia virus) is a retrovirus.

EIAV causes equine infectious anaemia (EIA) in equines. Equine infectious anaemia is also known as swamp fever in horses. In addition to horses (Equus *caballus)* EIAV can infect donkeys (Equus *asinus)* (Cook et al., 2001) and mules (Spyrou et al., 2003). However, a wide range of host susceptibility to disease expression is exhibited among these species (Cook et al., 2001; Hammond et al., 2000; Spyrou et al., 2003). EIAV infected horses can present three different disease states during infection: acute/sub-acute, chronic and inapparent.

EIAV is transmitted by bloodsucking insects. The virus (EIAV) is endemic in the Americas, parts of Europe, the Middle and Far East, Russia, and South Africa. The virus is a lentivirus. EIAV can be transmitted through blood, saliva, milk, and body secretions. Transmission is primarily through biting flies, such as the horse-fly and deer-fly. The virus can survive up to 4 hours in the carrier. Contaminated surgical equipment and recycled needles and syringes, and bits can transmit EIAV. Further, mares can transmit EIAV to their foals via the placenta. The risk of transmitting the disease is greatest when an infected horse is ill, as the blood levels of the virus are then high.

The EIA incubation period lasts usually one to three weeks, but may be as long as three months.

The most notable of the signs of disease are the concurrent development of febrile episodes (defined as rectal temperatures above 39°C), thrombocytopenia (defined as platelets levels bellow 105000/µl of blood), that are typically accompanied by viremia at least of 10⁵ copies of EIAV RNA/mL plasma.

The acute form of EIA is a sudden onset of the disease at full-force. Clinical signs include high fever, anemia (due to the breakdown of red blood cells), thrombocytopenia, weakness, swelling of the lower abdomen and legs, weak pulse, irregular heartbeat, tachypneia, petechiae on the mucous membrane, diarrhoea and blood stained feces. Thrombocytopenia is a consistent hematological finding and one of the earliest hematological abnormalities detected in acutely infected horses (Clabough et al., 1991; Crawford et al., 1996). Neurological signs are also reported in EIA infected horses (Oaks et al., 2004). Occasionally, death occurs during the acute infection, and the equine may die suddenly. After the initial bout, the majority of the horses may become asymptomatic.

The subacute form of EIA is a slower, less severe progression of the disease. Symptoms include recurrent fever, weight loss, an enlarged spleen (felt during a rectal examination), anemia, and swelling of the lower chest, abdominal wall, penile sheath, scrotum, and legs.

Some develop chronic recurring EIA signs that vary from mild illness and failure to thrive to fever, depression, petechial hemorrhages on the mucous membranes, weight loss, edema, and sometimes death. The chronic form of EIA is where an equine tires easily and is unsuitable for work. The equine may have a recurrent fever and anemia; the equine may relapse to the subacute or acute form even several years after the original attack. The majority of infected horses become life long inapparent carriers with no overt clinical abnormalities as a result of infection (Coggins, 1984; Leroux et al., 2004; McGuire et al., 1990), yet still tests positive for EIA antibodies. Such an equine can still pass on the virus.

In contrast to the pathogenesis observed in infected horses, no evident clinical signs result from EIAV in *in vivo* experimental infections of donkeys and mules. Indeed these Equids behave as inapparent carriers from the onset of infection (Cook et al., 2001; Spyrou et al., 2003).

EIA may cause abortion in pregnant mares. This may occur at any time during the pregnancy if there is a relapse when the virus enters the blood. Most infected mares will abort, however some give birth to healthy foals. The foals are not necessarily infected.

The present inventors have discovered a new and previously uncharacterised virus, which was obtained from horses with discordant results for EIAV testing i.e. the horses were positive for EIAV in an immunoblot but negative for EIAV in both the AGID test and an ELISA.

The present inventors have named this new uncharacterised virus New Equine Virus (NEV).

NEV may be obtainable from or may be similar to a virus obtainable from the deposit made at the European Collection of Cell Cultures (ECAAC), Culture Collections, under accession number 14120201.

Other workers in the field may refer to NEV as EIAV or EIAV-like.

The present inventors have identified a number of NEV peptides which find use in the present invention.

Thus, the NEV virus as described herein may comprise or contain any of SEQ ID NOS: 1-76.

Thus, the NEV virus as described herein may comprise or contain any of SEQ ID NOS: 77-152.

Examples of NEV peptides as described herein include:
SRLLESRGPTTSEK (SEQ ID NO: 1);
TLKEVLGGEAAVR (SEQ ID NO: 2);
PCRSKNILPV (SEQ ID NO: 3);
DPCVHSVDVLLR (SEQ ID NO: 4);
STFPPTPV (SEQ ID NO: 5);
NAPLLSKVSP (SEQ ID NO: 6);
MAQCIVNR (SEQ ID NO: 7);
KPNVEGRYGLSRSETNK (SEQ ID NO: 8);
QQGPEAPLPSLQVAEVPK (SEQ ID NO: 9);
PHAGSTQTEWPK (SEQ ID NO: 10);
PIQINACK (SEQ ID NO: 11);
GGMRESWDGGQV (SEQ ID NO: 12);
ESLVSKGFR (SEQ ID NO: 13);
CSLVLGEMQIKRIR (SEQ ID NO: 14);
LDKWEKIR (SEQ ID NO 15);
QMMIAASEK (SEQ ID NO 16);
QPSLPTGSEELK (SEQ ID NO 17);
EALDKIEEIQNKNKQK (SEQ ID NO 18);
PPIPVGGIYK (SEQ ID NO 19);
QEQNPPPSVSLRSLFGNDPL (SEQ ID NO 20);
EKIEQLR (SEQ ID NO 21);
DLLLIVAR (SEQ ID NO 22);
IVELLGR (SEQ ID NO 23);
IWGNVTWMEWER (SEQ ID NO 24);
LKDLFPNK (SEQ ID NO 25);
AKLEESFPGK (SEQ ID NO 26);
NGSLAGESIIIR (SEQ ID NO 27);
NITFNSSAGGDLEIT (SEQ ID NO 28);
AVILLLDRLR (SEQ ID NO 29);
GPGIHIGKR (SEQ ID NO 30);
VIICSASK (SEQ ID NO 31);
ESFPNK (SEQ ID NO 32);
FGNKTTIIFTK (SEQ ID NO 33);
LLNGSLAGESIIIR (SEQ ID NO 34);
VNVSVTNNNTTTNV (SEQ ID NO 35);
AILHILRR (SEQ ID NO 36);
DLLALDK (SEQ ID NO 37);
IGCQHSRIGITLPR (SEQ ID NO 38);
TLQYLALTALVTPK (SEQ ID NO 39);
PTTPVTPAPGVGEISKELAQGK (SEQ ID NO 40);
WSSALQYLIPR (SEQ ID NO 41);
PVWAEPVK (SEQ ID NO 42);
VKGNDLLK (SEQ ID NO 43);
EDLNLQDWK (SEQ ID NO 44);
LFVSVLQR (SEQ ID NO 45);
AAEQKASPPSLTPK (SEQ ID NO 46);
PLSLPLKI (SEQ ID NO 47);
FPSIVGRPR (SEQ ID NO 48);
IWHHTFYNELR (SEQ ID NO 49);
MTQIMFETF (SEQ ID NO 50);
EEEVAALVIDNGSGMCK (SEQ ID NO 51);
VAPEEHPVLLTEAPLNPK (SEQ ID NO 52);
AVFPSIVGRPR (SEQ ID NO 53);
YPIEHGIVTNWDDMEK (SEQ ID NO 54);
AVFPSIVGR (SEQ ID NO 55);
IGRKDAERQLLSPGNAR (SEQ ID NO 56);
DSYVGDEAQSKR (SEQ ID NO 57);
RGILTLK (SEQ ID NO 58);
WGIAHTTGIPGNSQGQAMVER (SEQ ID NO 59);
HLVDQLIRDLK (SEQ ID NO 60);
YEQLQLQAR (SEQ ID NO 61);
TITLEVEPSDTIENVK (SEQ ID NO 62);
INRELLK (SEQ ID NO 63);
QTIIPTNKDVDEK (SEQ ID NO 64);
KNYGKLDK (SEQ ID NO 65);
TTISFSK (SEQ ID NO 66);
QDRTTISFSK (SEQ ID NO 67);
AQGRAIAHK (SEQ ID NO 68);
MAQTQLVPVK (SEQ ID NO 69);
QELIPPCK (SEQ ID NO 70);
DIVLLENGK (SEQ ID NO 71);
LPPLSILK (SEQ ID NO 72);
IFLINLAFLIK (SEQ ID NO 73);
NRLEILK (SEQ ID NO 74);
ADDVAVLQDALGR (SEQ ID NO 75);
LNKSLEQLR (SEQ ID NO 76); and
variants, homologues, fragments or derivatives thereof having at least 75%, 80%, 85%, 90%, 95%, 97%, 98%, or 99% identity to any one of SEQ ID NOs 1 to 76.

NEV peptides as described herein are selected from the group consisting of SEQ ID NOs 1 to 14.

Examples of nucleotide sequences encoding NEV peptides as described herein include polynucleotide sequences encoding:
SRLLESRGPTTSEK (SEQ ID NO: 1);
TLKEVLGGEAAVR (SEQ ID NO: 2);
PCRSKNILPV (SEQ ID NO: 3);
DPCVHSVDVLLR (SEQ ID NO: 4);
STFPPTPV (SEQ ID NO: 5);
NAPLLSKVSP (SEQ ID NO: 6);
MAQCIVNR (SEQ ID NO: 7);
KPNVEGRYGLSRSETNK (SEQ ID NO: 8);
QQGPEAPLPSLQVAEVPK (SEQ ID NO: 9);
PHAGSTQTEWPK (SEQ ID NO: 10);
PIQINACK (SEQ ID NO: 11);
GGMRESWDGGQV (SEQ ID NO: 12);
ESLVSKGFR (SEQ ID NO: 13);
CSLVLGEMQIKRIR (SEQ ID NO: 14);
LDKWEKIR (SEQ ID NO 15);
QMMIAASEK (SEQ ID NO 16);
QPSLPTGSEELK (SEQ ID NO 17);
EALDKIEEIQNKNKQK (SEQ ID NO 18);
PPIPVGGIYK (SEQ ID NO 19);
QEQNPPPSVSLRSLFGNDPL(SEQ ID NO 20);
EKIEQLR (SEQ ID NO 21);
DLLLIVAR (SEQ ID NO 22);
IVELLGR (SEQ ID NO 23);
IWGNVTWMEWER (SEQ ID NO 24);
LKDLFPNK(SEQ ID NO 25);
AKLEESFPGK (SEQ ID NO 26);
NGSLAGESIIIR (SEQ ID NO 27);
NITFNSSAGGDLEIT (SEQ ID NO 28);
AVILLLDRLR(SEQ ID NO 29);
GPGIHIGKR (SEQ ID NO 30);
VIICSASK (SEQ ID NO 31);
ESFPNK (SEQ ID NO 32);
FGNKTTIIFTK (SEQ ID NO 33);
LLNGSLAGESIIIR (SEQ ID NO 34);
VNVSVTNNNTTTNV (SEQ ID NO 35);
AILHILRR (SEQ ID NO 36);
DLLALDK (SEQ ID NO 37);
IGCQHSRIGITLPR (SEQ ID NO 38);
TLQYLALTALVTPK (SEQ ID NO 39);
PTTPVTPAPGVGEISKELAQGK (SEQ ID NO 40);
WSSALQYLIPR (SEQ ID NO 41);
PVWAEPVK (SEQ ID NO 42);
VKGNDLLK (SEQ ID NO 43);
EDLNLQDWK (SEQ ID NO 44);
LFVSVLQR (SEQ ID NO 45);
AAEQKASPPSLTPK (SEQ ID NO 46);
PLSLPLKI (SEQ ID NO 47);
FPSIVGRPR (SEQ ID NO 48);
IWHHTFYNELR (SEQ ID NO 49);
MTQIMFETF (SEQ ID NO 50);
EEEVAALVIDNGSGMCK (SEQ ID NO 51);
VAPEEHPVLLTEAPLNPK (SEQ ID NO 52);
AVFPSIVGRPR (SEQ ID NO 53);
YPIEHGIVTNWDDMEK (SEQ ID NO 54);
AVFPSIVGR (SEQ ID NO 55);
IGRKDAERQLLSPGNAR (SEQ ID NO 56);
DSYVGDEAQSKR (SEQ ID NO 57);
RGILTLK (SEQ ID NO 58);
WGIAHTTGIPGNSQGQAMVER (SEQ ID NO 59);
HLVDQLIRDLK (SEQ ID NO 60);
YEQLQLQAR (SEQ ID NO 61);
TITLEVEPSDTIENVK (SEQ ID NO 62);
INRELLK (SEQ ID NO 63);
QTIIPTNKDVDEK (SEQ ID NO 64);
KNYGKLDK (SEQ ID NO 65);
TTISFSK (SEQ ID NO 66);
QDRTTISFSK (SEQ ID NO 67);
AQGRAIAHK (SEQ ID NO 68);
MAQTQLVPVK (SEQ ID NO 69);
QELIPPCK (SEQ ID NO 70);
DIVLLENGK (SEQ ID NO 71);
LPPLSILK (SEQ ID NO 72);
IFLINLAFLIK (SEQ ID NO 73);
NRLEILK (SEQ ID NO 74);
ADDVAVLQDALGR (SEQ ID NO 75);
LNKSLEQLR (SEQ ID NO 76); and
variants, homologues, fragments or derivatives thereof having at least 75%, 80%, 85%, 90%, 95%, 97%, 98%, or 99% identity to any one of SEQ ID NOs 1 to 76.

Nucleotide sequences encoding NEV peptides as described herein are selected from the group consisting of polynucleotide sequences encoding any one of SEQ ID NOs 1 to 14.

Further examples of nucleotide sequences encoding NEV peptides as described herein include:
AGCAGGCTGCTGGAGAGCAGGGGCCCCACCACCAGCGAGAAG (SEQ ID NO 77);
ACCCTGAAAGAGGTTCTTGGTGGTGAAGCAGCCGTGCGC (SEQ ID NO 78);
CCCTGCCGGAGCAAGAACATCCTGCCCGTG (SEQ ID NO 79);
GACCCCTGCGTGCACAGCGTGGACGTGCTGCTGAGG (SEQ ID NO 80);
TCCACATTTCCTCCCACTCCCGTC (SEQ ID NO 81);
AATGCCCCATTACTCTCAAAAGTGTCCCCA (SEQ ID NO 82);
ATGGCACAGTGTATCGTTAACCGC (SEQ ID NO 83);
AAGCCCAACGTGGAGGGCAGGTACGGCCTGAGCAGGAGCGAGACCAACAAG(S EQ ID NO 84); 
CAGCAAGGCCCAGAGGCGCCACTGCCCAGCCTGCAGGTTGCAGAAGTGCCTA
AA (SEQ ID NO 85);
CCCCATGCGGGCTCCACTCAGACAGAGTGGCCCAAA (SEQ ID NO 86);
CCAATACAGATCAATGCTTGCAAA (SEQ ID NO 87);
GGTGGAATGCGTGAGTCATGGGATGGAGGACAAGTT (SEQ ID NO 88);
GAATCTCTGGTGTCAAAAGGGTTTCGG (SEQ ID NO 89);
TGCTCATTAGTCCTTGGGGAGATGCAAATCAAA (SEQ ID NO 90);
CTGGACAAGTGGGAGAAGATCAGG (SEQ ID NO 91);
CAGATGATGATCGCCGCCAGCGAGAAG(SEQ ID NO 92);
CAGCCCAGCCTGCCCACCGGCAGCGAGGAGCTGAAG (SEQ ID NO 93);
GAGGCCCTGGACAAGATCGAGGAGATCCAGAACAAGAACAAGCAGAAG (SEQ ID NO 152); CCCCCCATCCCCGTGGGCGGCATCTACAAG (SEQ ID NO 94); CAGGAGCAGAACCCCCCCCCCAGCGTGAGCCTGAGGAGCCTGTTCGGCAACG ACCCCCTG(SEQ ID NO 95);
GAGAAGATCGAGCAGCTGAGG (SEQ ID NO 96);
GACCTGCTGCTGATCGTGGCCAGG (SEQ ID NO 97);
ATCGTGGAGCTGCTGGGCAGG (SEQ ID NO 98);
ATCTGGGGCAACGTGACCTGGATGGAGTGGGAGAGG (SEQ ID NO 99);
CTGAAGGACCTGTTCCCCAACAAG (SEQ ID NO 100);
GCCAAGCTGGAGGAGAGCTTCCCCGGCAAG (SEQ ID NO 101);
AACGGCAGCCTGGCCGGCGAGAGCATCATCATCAGG (SEQ ID NO 102);
AACATCACCTTCAACAGCAGCGCCGGCGGCGACCTGGAGATCACC (SEQ ID NO 103);
GCCGTGATCCTGCTGCTGGACAGGCTGAGG (SEQ ID NO 104);
GGCCCCGGCATCCACATCGGCAAGAGG (SEQ ID NO 105);
GTGATCATCTGCAGCGCCAGCAAG (SEQ ID NO 106);
GAGAGCTTCCCCAACAAG (SEQ ID NO 107);
TTCGGCAACAAGACCACCATCATCTTCACCAAG (SEQ ID NO 108);
CTGCTGAACGGCAGCCTGGCCGGCGAGAGCATCATCATCAGG (SEQ ID NO 109);
GTGAACGTGAGCGTGACCAACAACAACACCACCACCAACGTG (SEQ ID NO 110);
GCCATCCTGCACATCCTGAGGAGG (SEQ ID NO 111);
GACCTGCTGGCCCTGGACAAG (SEQ ID NO 112);
ATCGGCTGCCAGCACAGCAGGATCGGCATCACCCTGCCCAGG (SEQ ID NO 113);
ACCCTGCAGTACCTGGCCCTGACCGCCCTGGTGACCCCCAAG (SEQ ID NO 114); 
CCCACCACCCCCGTGACCCCCGCCCCCGGCGTGGGCGAGATCAGCAAGGAGC
TGGCCCAGGGCAAG (SEQ ID NO 115);
GTGGTGAGCAGCGCCCTGCAGTACCTGATCCCCAGG (SEQ ID NO 116);
CCCGTGTGGGCCGAGCCCGTGAAG (SEQ ID NO 117);
GTGAAGGGCAACGACCTGCTGAAG (SEQ ID NO 118);
GAGGACCTGAACCTGCAGGACTGGAAG (SEQ ID NO 119);
CTGTTCGTGAGCGTGCTGCAGAGG (SEQ ID NO 120);
GCCGCCGAGCAGAAGGCCAGCCCCCCCAGCCTGACCCCCAAG (SEQ ID NO 121);
CCCCTGAGCCTGCCCCTGAAGATC (SEQ ID NO 122);
TTCCCCAGCATCGTGGGCAGGCCCAGG (SEQ ID NO 123);
ATCTGGCACCACACCTTCTACAACGAGCTGAGG (SEQ ID NO 124);
ATGACCCAGATCATGTTCGAGACCTTC (SEQ ID NO 125);
GAGGAGGAGGTGGCCGCCCTGGTGATCGACAACGGCAGCGGCATGTGCAAG (SEQ ID NO 126); 
GTGGCCCCCGAGGAGCACCCCGTGCTGCTGACCGAGGCCCCCCTGAACCCCA
AG (SEQ ID NO 127);
GCCGTGTTCCCCAGCATCGTGGGCAGGCCCAGG (SEQ ID NO 128);
TACCCCATCGAGCACGGCATCGTGACCAACTGGGACGACATGGAGAAG (SEQ ID NO 129);
GCCGTGTTCCCCAGCATCGTGGGCAGG(SEQ ID NO 130);
ATCGGCAGGAAGGACGCCGAGAGGCAGCTGCTGAGCCCCGGCAACGCCAGG (SEQ ID NO 131);
GACAGCTACGTGGGCGACGAGGCCCAGAGCAAGAGG (SEQ ID NO 132);
AGGGGCATCCTGACCCTGAAG (SEQ ID NO 133); 
TGGGGCATCGCCCACACCACCGGCATCCCCGGCAACAGCCAGGGCCAGGCCA
TGGTGGAGAGG (SEQ ID NO 134);
CACCTGGTGGACCAGCTGATCAGGGACCTGAAG (SEQ ID NO 135);
TACGAGCAGCTGCAGCTGCAGGCCAGG (SEQ ID NO 136);
ACCATCACCCTGGAGGTGGAGCCCAGCGACACCATCGAGAACGTGAAG (SEQ ID NO 137);
ATCAACAGGGAGCTGCTGAAG (SEQ ID NO 138);
CAGACCATCATCCCCACCAACAAGGACGTGGACGAGAAG (SEQ ID NO 139);
AAGAACTACGGCAAGCTGGACAAG (SEQ ID NO 140);
ACCACCATCAGCTTCAGCAAG (SEQ ID NO 141);
CAGGACAGGACCACCATCAGCTTCAGCAAG (SEQ ID NO 142);
GCCCAGGGCAGGGCCATCGCCCACAAG (SEQ ID NO 143);
ATGGCCCAGACCCAGCTGGTGCCCGTGAAG (SEQ ID NO 144);
CAGGAGCTGATCCCCCCCTGCAAG (SEQ ID NO 145);
GACATCGTGCTGCTGGAGAACGGCAAG (SEQ ID NO 146);
CTGCCCCCCCTGAGCATCCTGAAG (SEQ ID NO 147);
ATCTTCCTGATCAACCTGGCCTTCCTGATCAAG (SEQ ID NO 148);
AACAGGCTGGAGATCCTGAAG (SEQ ID NO 149);
GCCGACGACGTGGCCGTGCTGCAGGACGCCCTGGGCAGG (SEQ ID NO 150);
CTGAACAAGAGCCTGGAGCAGCTGAGG (SEQ ID NO 151); and
variants, homologues, fragments or derivatives thereof having at least 75%, 80%, 85%, 90%, 95%, 97%, 98%, or 99% identity to any one of SEQ ID NOs 77 to 152.

Nucleotide sequences encoding NEV peptides as described herein are selected from the group consisting of SEQ ID NOs 77 to 89.

The peptides of the present invention and the nucleotide sequences of the present invention are derived from a novel virus, NEV. Without wishing to be bound by theory, NEV identified by the present inventors represents a completely new and uncharacterised virus.

In some embodiments, the peptides of the invention and/or the novel viral strain or variant as described herein may be denoted as "EIAV-like". By "EIAV-Iike" it is meant that the virus and/or peptides were derived from horses with discordant results obtained from EIAV testing i.e. the horses were positive using an EIAV immunoblot but negative using the AGID test and an ELISA. None of the identified peptides belonged to the known EIAV proteome. Some of the peptides were found to have sequence identity with retroviral peptides. Some of the peptides were found to have sequence identity to part of the HIV-1 proteome.

Table 2 shows the viral proteins which have the highest sequence identity (similarity) to SEQ ID NOs 1-76 as described herein.

**Table 2. Similarity of peptides as described herein to known viral peptides (if any)**

| **SEQ ID NO:** | **Peptide** | **Protein-Virus (Highest similarity)** | **Taxonomy** | **Host** | **Swissprot Accession number** | **NCBI accession number** |
|---|---|---|---|---|---|---|
| 1 | SRLLESRGPTTSEK | Not available | | | | |
| 2 | TLKEVLGGEAAVR | Not available | | | | |
| 3 | PCRSKNILPV | Not available | | | | |
| 4 | DPCVHSVDVLLR | Not available | | | | |
| 5 | STFPPTPV | Not available | | | | |
| 6 | NAPLLSKVSP | Not available | | | | |
| 7 | MAQCIVNR | Not available | | | | |
| 8 | KPNVEGRYGLSRSETNK | Not available | | | | |
| 9 | QQGPEAPLPSLQVAEVPK | Not available | | | | |
| 10 | PHAGSTQTEWPK | Not available | | | | |
| 11 | PIQINACK | Not available | | | | |
| 12 | GGMRESWDGGQV | Not available | | | | |
| 13 | ESLVSKGFR | Not available | | | | |
| 14 | CSLVLGEMQIK | Not available | | | | |
| 15 | LDKWEKIR | GAG/MA HIV-1 | Retroviridae | Mammals_Human | gi\|407732641 | gi\|86753472\|ABD14908.1 |
| 16 | QMMIAASEK | GAG HIV-1 | Retroviridae | Mammals_Human | RRRRRtr\|Q5U6V4\|Q5U6V4_9HIV1 | |
| 17 | QPSLPTGSEELK | GAG/MA HIV-1 | Retroviridae | Mammals_Human | gi\|407732641 | gi\|222531787\|BAH21078.1 |
| 18 | EALDKIEEIQNKNKQK | GAG/MA HIV-1 | Retroviridae | Mammals_Human | tr\|C9DWN6\|C9DWN6_9HIV1 | gi\|7767365\|AAF69092.1 |
| 19 | PPIPVGGIYK | GAG HIV-1 | Retroviridae | Mammals_Human | tr\|C4TMF7\|C4TMF7_9HIV1 | gi\|12746078\|AAK07335.1 |
| 20 | QEQNPPPSVSLRSLFGND PL | GAG HIV-1 | Retroviridae | Mammals_Human | tr\|C3VAJ3\|C3VAJ3_9HIV1 | gi\|227058002\|ACP18955.1 |
| 21 | EKIEQLR | RT HIV-1 | Retroviridae | Mammals_Human | gi\|407233110 | gi\|429524088\|AFZ98436.1 |
| 22 | DLLLIVAR | ENV-HIV-1 | Retroviridae | Mammals_Human | tr\|B5AJS5\|B5AJS5_9HIV1 | gi\|12740\|AAA82861.1 |
| 23 | IVELLGR | ENV-HIV-1 | Retroviridae | Mammals_Human | tr\|B5AJS5\|B5AJS5_9HIV1 | gi\|381343663\|AFG23872.1 |
| 24 | IWGNVTWMEWER | ENV-HIV-1 | Retroviridae | Mammals_Human | tr\|B5AJS5\|B5AJS5_9HIV1 | gi\|342891379\|AEL75499.1 |
| 25 | LKDLFPNK | ENV-HIV-1 | Retroviridae | Mammals_Human | tr\|B5AJS5\|B5AJS5_9HIV1 | gi\|194476065\|ACF74764.1 |
| 26 | AKLEESFPGK | ENV-HIV-1 | Retroviridae | Mammals_Human | tr\|QSQB22\|QSQB22_9HIV1 | gi\|55979593\|AAV69232.1 |
| 27 | NGSLAGESIIIR | ENV-HIV-1 | Retroviridae | Mammals_Human | tr\|Q5QB22\|Q5QB22_9HIV1 | gi\|55979314\|AAV69098.1 |
| 28 | NITFNSSAGGDLEIT | ENV-HIV-1 | Retroviridae | Mammals_Human | tr\|Q5QB22\|Q5QB22_9HIV1 | gi\|2109329\|AAB58180.1 |
| 29 | AVILLLDRLR | ENV-HIV-1 | Retroviridae | Mammals_Human | RRRRRtr\|B3UES1\|B3UES1_9H IV1 | |
| 30 | GPGIHIGKR | ENV-HIV-1 | Retroviridae | Mammals_Human | RRRRRtr\|B3UES1\|B3UES1_9H IV1; | |
| 31 | VIICSASK | ENV/VPU-HIV-1 | Retroviridae | Mammals_Human | tr\|Q1HBI3\|Q1HBI3_9HIV1 | gi\|210039295\|CAQ06400.1 |
| 32 | ESFPNK | ENV-HIV-1 | Retroviridae | Mammals_Human | tr\|Q5QBB1\|Q5QBB1_9HIV1 | gi\|209864285\|ACI89043.1 |
| 33 | FGNKTTIIFTK | ENV-HIV-1 | Retroviridae | Mammals_Human | tr\|Q7ZPZ1\|Q7ZPZ1_9HIV1; tr\|Q7ZPZ0\|Q7ZPZ0_9HIV1; tr\|Q7ZPY9\|Q7ZPY9_9HIV1 | gi\|27763543\|AAO20805.1 |
| 34 | LLNGSLAGESIIIR | ENV-HIV-1 | Retroviridae | Mammals_Human | tr\|Q5QB22\|Q5QB22_9HIV1 | gi\|55979286\|AAV69084.1 |
| 35 | VNVSVTNNNTTTNV | ENV-HIV-1 | Retroviridae | Mammals_Human | gi\|410815107 | gi\|410815107\|AFV82892.1 |
| 36 | AILHILRR | ENV-HIV-1 | Retroviridae | Mammals_Human | gi\|407734638 | gi\|62735303\|AAX97057.1 |
| 37 | DLLALDK | ENV-HIV-1 | Retroviridae | Mammals_Human | gi\|407734638 | gi\|468091\|AAA19140.1 |
| 38 | IGCQHSRIGITLPR | VPR_HIV | Retroviridae | Mammals_Human | tr\|Q8UT50\|Q8UT50_9HIV1; tr\|C5HAM4\|C5HAM4_9HIV1 | gi\|17046785\|AAL34799.1 |
| 39 | TLQYLALTALVTPK | VIF_HIV | Retroviridae | Mammals_Human | gi\|408719443; gi\|408719439; gi\|408719435; gi\|408719431 | gi\|408719439\|AFU83789.1 |
| 40 | PTTPVTPAPGVGEISKELA QGK | Nef_HIV-1 | Retroviridae | Mammals_Human | tr\|E5RDP6\|E5RDP6_9HIV1 | ADR03151.1 |
| 41 | VVSSALQYLIPR | TAX-HTLV3 | Retroviridae | Mammals_Human | gi\|120875 | gi\|194302003\|ACF40916.1 |
| 42 | PVWAEPVK | ENV-SFV | Retroviridae | Mammals-Simian | gi\|425856942 | gi\|425856942\|AFX98090.1 |
| 43 | VKGNDLLK | GAG/MA SRV2 | Retroviridae | Mammals-Simian | gi\|1730185 | gi\|5442109\|AAD43258.1 |
| 44 | EDLNLQDWK | GAG/MA_MMTVG | Retroviridae | Mammals-Mice | gi\|120875 | gi\|120875\|P03343.3 |
| 45 | LFVSVLQR | GAG/MA_MMTVG | Retroviridae | Mammals-Mice | gi\|120875 | gi\|40796112\|NP955565.1 |
| 46 | AAEQKASPPSLTPK | FGR_ABL | Retroviridae | Mammals-Mice | gi\|120875 | gi\|40796142\|NP955595.1 |
| 47 | PLSLPLKI | ENV-FLV | Retroviridae | Mammals-Feline | gi\|399433 | gi\|396077157\|BAM33591.1 |
| 48 | FPSIVGRPR | FGR_FSVGR | Retroviridae | Mammals-Feline | gi\|302393735 | gi\|125357\|P00544.1 |
| 49 | IWHHTFYNELR | FGR_FSVGR | Retroviridae | Mammals-Feline | gi\|117940173 | gi\|125357\|P00544.1 |
| 50 | MTQIMFETF | FGR_FSVGR | Retroviridae | Mammals-Feline | gi\|125136 | gi\|125357\|P00544.1 |
| 51 | EEEVAALVIDNGSGMCK | FGR_FSVGR | Retroviridae | Mammals-Feline | gi\|125357 | gi\|125357\|P00544.1 |
| 52 | VAPEEHPVLLTEAPLNPK | FGR_FSVGR | Retroviridae | Mammals-Feline | gi\|125357 | gi\|125357\|P00544.1 |
| 53 | AVFPSIVGRPR | FGR_FSVGR | Retroviridae | Mammals-Feline | gi\|125357 | gi\|125357\|P00544.1 |
| 54 | YPIEHGIVTNWDDMEK | FGR_FSVGR | Retroviridae | Mammals-Feline | gi\|125357 | gi\|125357\|P00544.1 |
| 55 | AVFPSIVGR | FGR_FSVGR | Retroviridae | Mammals-Feline | gi\|125357 | gi\|125357\|P00544.1 |
| 56 | IGRKDAERQLLSPGNAR | FGR_FSVGR | Retroviridae | Mammals-Feline | gi\|125357 | gi\|125357\|P00544.1 |
| 57 | DSYVGDEAQSKR | FGR_FSVGR | Retroviridae | Mammals-Feline | gi\|125357 | gi\|125357\|P00544.1 |
| 58 | RGILTLK | FGR_FSVGR | Retroviridae | Mammals-Feline | gi\|125357 | P00544.1 |
| 59 | WGIAHTTGIPGNSQGQAM VER | IN-ALV | Retroviridae | Avian | gi\|1730185 | gi\|6729948\|1VSMA |
| 60 | HLVDQLIRDLK | Pol_BVD | Flaviviridae | Mammals_Bovine | tr\|Q65815\|Q65815_BVDV | AAA02769.1 |
| 61 | YEQLQLQAR | Pol_BVD | Flaviviridae | Mammals_Bovine | tr\|Q65815\|Q65815_BVDV | AAC72518.1 |
| 62 | TITLEVEPSDTIENVK | Pol_BVD | Flaviviridae | Mammals_Bovine | tr\|Q65815\|Q65815_BVDV | AAD44038.1 |
| 63 | INRELLK | NSP1 | Reoviridae | Mammals-Equine | sp\|O39822\|NSP1_ROTEL | AEH96574.1 |
| 64 | QTIIPTNKDVDEK | NS3 | Reoviridae | Insects | sp\|Q91ID4\|NS3_LDCPR | NP149153.1 |
| 65 | KNYGKLDK | UNCP | Iridoviridade | Insects | sp\|Q91FR3\|VF259-IIV6 | NP149722.1 |
| 66 | TTISFSK | POL | Secoviridae | Plant | sp\|P31630\|POL2_CPSMV | NP734064.1 |
| 67 | QDRTTISFSK | POL | Secoviridae | Plant | sp\|P31630\|POL2_CPSMV | NP734064.1 |
| 68 | AQGRAIAHK | POL | Tombusviridae | Plant | sp\|P22956\|RDRP_RCNMV | AAA47456.1 |
| 69 | MAQTQLVPVK | ABVP | Ampullaviridae | Plant | sp\|A4ZUA5\|GT315_ABVP | YP 001210323.1 |
| 70 | QELIPPCK | MG360_16R/ASFV_K5 | Asfarviridae | Mammals_Swine | Mammals_Swin sp\|P0C9R3\|36016_ASFK5 | P0CA17.1 |
| 71 | DIVLLENGK | E4/PPV47 | Papillomaviridae | Mammals_Human | sp\|P22421\|VE4_HPV47 | P22421.1 |
| 72 | LPPLSILK | FIBP_ADE4 | Adenoviridae | Mammals_Human | sp\|P368441F\|BP_ADE04 | P36844.1 |
| 73 | IFLINLAFLIK | YMTV5 | Poxviridae | Mammals_Monkey | sp\|Q6TUY0\|PAP1_YMTV5 | NP938288.1 |
| 74 | NRLEILK | Pro/CA | Herpesviridae | Mammals-Equine | sp\|P52369\|PPR_EHV2 | NP042612.1 |
| 75 | ADDVAVLQDALGR | Helicase- primase/HHV-2 | Herpesviridae | Mammals_Human | sp\|P89471\|PRIM_HHV2H | AFM93871.1 |
| 76 | LNKSLEQLR | UL113/HCMV | Herpesviridae | Mammals_Human | sp\|Q96896\|U79_HHV6H; sp\|P52530\|U79_HHV6Z | AAA68471.1 |

Both EIAV and NEV can infect animals such as equines.

In one embodiment the animal is an equid.

Examples of equids include horses, donkeys, mules, hinnys and zebras.

In some embodiments, the terms "equid" and "equine" are used interchangeably.

In another embodiment, the animal is a horse.

In one embodiment a peptide as described herein, and/or a nucleotide sequence according to the present invention, and/or a vector according to the present invention, and/or an antibody according to the present invention, and/or a pharmaceutical composition according to the present invention is capable of inducing an immune response against EIAV and/or NEV in an animal. In another embodiment, a peptide according as described herein, and/or a nucleotide sequence according to the present invention, and/or a vector according to the present invention, and/or an antibody according to the present invention, and/or a pharmaceutical composition according to the present invention is capable of inducing a protective immune response against EIAV and/or NEV in an animal.

As used herein the term "treatment of equine infectious anaemia" refers to a reduction in EIA symptoms in the treated animal. For example, the animal may no longer be classified as having an acute form of EIA but now has the subacute form, or the chronic form, or the inapparent form and no longer shows any EIA symptoms - the virus may, however, still be detected in the animal but at levels below, for example, 10⁵ copies of EIAV RNA/mL plasma. For instance, after treatment, the EIAV (e.g. the EIAV of the present description) viral titre in the treated animal may be reduced by at least 10%, 20%, 30%, 40% or 50% when compared to the EIAV viral titre of the animal before treatment. In another example, thrombocytopenia in the treated animal may be reduced by at least 10%, 20%, 30%, 40% or 50% when compared to the thrombocytopenia of the animal before treatment. In a further example, the peaks of the fever in the treated animal may be reduced by at least 10%, 20%, 30%, 40% or 50% when compared to the temperature of the animal before treatment. Without wishing to be bound by theory, the treatment of equine infectious anaemia occurs by a reduction in the level of an EAIV virus in an animal.

As used herein the term "treatment of NEV" refers to a reduction in NEV symptoms in the treated animal. For example, the animal may no longer be classified as having an acute form of NEV but now has the subacute form, or the chronic form, or the inapparent form and no longer shows any NEV symptoms - the virus may, however, still be detected in the animal but at levels below, for example, 10⁵ copies of NEV RNA/mL plasma. For instance, after treatment, the NEV (e.g. the NEV of the present description) viral titre in the treated animal may be reduced by at least 10%, 20%, 30%, 40% or 50% when compared to the NEV viral titre of the animal before treatment. In another example, thrombocytopenia in the treated animal may be reduced by at least 10%, 20%, 30%, 40% or 50% when compared to the thrombocytopenia of the animal before treatment. In a further example, the peaks of the fever in the treated animal may be reduced by at least 10%, 20%, 30%, 40% or 50% when compared to the temperature of the animal before treatment. Without wishing to be bound by theory, the treatment of NEV occurs by a reduction in the level of an NEV virus in an animal.

The viral titre may be determined in a sample from an animal such as a blood sample, a blood serum sample, a plasma sample, a saliva sample, a sputum sample, a urine sample, a semen sample, a fecal sample, a milk sample, a biopsy sample, a lymph node biopsy sample, and/or a sweat sample.

As used herein the term "prevention of equine infectious anaemia" refers to a treated animal not developing EIA symptoms if the treated animal is exposed to the equine infectious anaemia virus, for example by exposure to a bloodsucking insect carrying the virus. The treated animal did not have any EIA symptoms before treatment and/or did not have any detectable EIAV in a sample such as a blood sample before treatment. For example, commercially available tests such as the Coggins test and/or the diagnostic test of the present invention may be used to determine (i.e. detect) if there is an EIAV titre in an animal.

As used herein the term "prevention of NEV" refers to a treated animal not developing NEV symptoms if the treated animal is exposed to the NEV virus, for example by exposure to a bloodsucking insect carrying the virus. The treated animal did not have any NEV symptoms before treatment and/or did not have any detectable NEV in a sample such as a blood sample before treatment.

As used herein the term "treatment of an infection by an EIAV" refers to a reduction in EIAV (e.g. the EIAV of the present description) viral titre in a sample from the treated animal when compared to the EIAV viral titre of the animal before treatment. For instance, after treatment, the EIAV (e.g. the EIAV of the present description) viral titre in the treated animal may be reduced by at least 10%, 20%, 30%, 40% or 50% when compared to the viral titre of the animal before treatment. Without wishing to be bound by theory, a reduction in the level of an EIAV virus in an animal may prevent the development of EIA symptoms.

As used herein the term "treatment of an infection by a NEV" refers to a reduction in NEV (e.g. the NEV of the present description) viral titre in a sample from the treated animal when compared to the NEV viral titre of the animal before treatment. For instance, after treatment, the NEV (e.g. the NEV of the present description) viral titre in the treated animal may be reduced by at least 10%, 20%, 30%, 40% or 50% when compared to the viral titre of the animal before treatment. Without wishing to be bound by theory, a reduction in the level of an NEV virus in an animal may prevent the development of NEV symptoms.

As used herein the term "prevention of an infection by an EIAV" refers to a treated animal not developing a detectable EIAV titre if the treated animal is exposed to the equine infectious anaemia virus, for example by exposure to a bloodsucking insect carrying the virus. The treated animal did not have any detectable EIAV in a sample such as a blood sample before treatment. For example, commercially available tests such as the Coggins test and/or the diagnostic test of the present invention may be used to determine (i.e. detect) if there is a EIAV titre in an animal.

As used herein the term "prevention of an infection by a NEV" refers to a treated animal not developing a detectable NEV titre if the treated animal is exposed to the NEV virus, for example by exposure to a bloodsucking insect carrying the virus. The treated animal did not have any detectable NEV in a sample such as a blood sample before treatment.

By using a kit according to the present invention or by using a diagnostic method of the present invention, animals which are infected with EIAV and/or NEV can be identified.

Animals with an EIAV and/or NEV infection may be isolated from other animals (e.g. animals which do not have the EIAV and/or NEV virus). Advantageously, this helps to prevent the spread of EIAV and/or NEV infection from infected animals to those which are not infected thereby controlling EIAV/NEV infection and/or EIAV/NEV within a group of animals.

Animals with an EIAV and/or NEV infection may be monitored (by using a kit according to the present invention or by using a diagnostic method of the present invention) to determine the progression of the EIAV/NEV infection and/or determine the progression of EIAV/NEV. Animals with an EIAV and/or NEV infection may be isolated from other animals (e.g. animals which do not have the EIAV and/or NEV virus) once the level of infection and/or the progression of EIAV and/or NEV has reached a critical point. Typically animals should be isolated during febrile episodes when rectal temperatures are above 39ºC, platelets levels are below 105000/µl of blood and viremia is at least of 10⁵ copies of EIAV and/or NEV RNA/mL plasma.

In addition or alternatively, by identifying animals with an EIAV and/or NEV infection (by using a kit according to the present invention or by using a diagnostic method of the present invention) care can be taken to ensure that medical equipment used on an EIAV and/or NEV infected animal is not used on an animal which does not have a EIAV and/or NEV infection. Advantageously, this helps to prevent the spread of EIAV and/or NEV infection from infected animals to those which are not infected thereby controlling EIAV and/or NEV disease with a group of animals.

In some embodiments, an animal identified as having EIAV and/or NEV is euthanized. Typically animals which are euthanized are those with frequent febrile episodes and animals which are lethargic or in lateral recumbence. An animal having EIAV and/or NEV may be euthanized when the viremia peaks are frequent.

Described herein is the use of NEV peptides in the discrimination of NEV-infected animals, and in the diagnosis, treatment and prevention of NEV infection in NEV-infected animals. Similarly, the present invention encompasses the use of NEV peptides in the discrimination of EIAV-infected animals, and in the diagnosis, treatment and prevention of EIAV infection in EIAV-infected animals. For example, the present inventors have identified EIAV reference sera (from horses) that are positive for an NEV fusion peptide of the invention.

### Deposit

The NEV virus described herein has been deposited by Equigerminal SA, Parque tecnologico de Cantanhede, nucleo 4 lote 4, Cantanhede, 3060-197 Portugal under the Budapest Treaty on the International Recognition of the Deposit of Microorganisms for the purposes of Patent Procedure at European Collection of Cell Cultures (ECAAC), Culture Collections, Public Health England, Porton Down, Salisbury, Wiltshire UK SP4 0JG on 2 December 2014 under accession number 14120201.

Other aspects of the present invention relate to the above viral deposit made at the ECAAC depository under accession number 14120201.

The present invention also related to peptide and nucleotide sequences obtainable from this deposit.

Described herein are nucleotide sequences capable of hybridising to nucleotide sequences obtainable from the deposit.

The NEV virus of the deposit may also be referred to as EIAV-like in the deposit.

### Vectors

As it is well known in the art, a vector is a tool that allows or facilitates the transfer of an entity from one environment to another. In accordance with the present invention, and by way of example, some vectors used in recombinant DNA techniques allow entities, such as a segment of DNA (such as a heterologous DNA segment, such as a heterologous cDNA segment), to be transferred into a host and/or a target cell for the purpose of replicating the vectors comprising the nucleotide sequences of the present invention and/or expressing the proteins of the invention encoded by the nucleotide sequences of the present invention. Examples of vectors used in recombinant DNA techniques include plasmids, chromosomes, artificial chromosomes or viruses.

The term "vector" includes expression vectors and/or transformation vectors.

The term "expression vector" means a construct capable of *in vivo* or *in vitro* or *ex vivo* expression.

As used herein, the term "expression vector" refers to a DNA construct containing a DNA coding sequence (e.g., gene sequence) that is operably linked to one or more suitable control sequence(s) capable of affecting expression of the coding sequence in a host. Such control sequences include a promoter to effect transcription, an optional operator sequence to control such transcription, a sequence encoding suitable mRNA ribosome binding sites, and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. The plasmid is the most commonly used form of expression vector. However, the description is intended to include such other forms of expression vectors that serve equivalent functions and which are, or become, known in the art.

The term "operably linked" refers to juxtaposition wherein the elements are in an arrangement allowing them to be functionally related. For example, a promoter is operably linked to a coding sequence if it controls the transcription of the coding sequence.

In one embodiment, the nucleotide sequence of the present invention is operably linked to a transcription unit.

The term "transcription unit(s)" as described herein are regions of nucleic acid containing coding sequences and the signals for achieving expression of those coding sequences independently of any other coding sequences. Thus, each transcription unit generally comprises at least a promoter, an optional enhancer and a polyadenylation signal.

The term "transformation vector" means a construct capable of being transferred from one species to another.

### "Naked DNA"

The vectors comprising nucleotide sequences encoding polypeptides of the present invention may be administered directly as "a naked nucleic acid construct"; in one embodiment the vector comprises flanking sequences homologous to the host cell genome. As used herein, the term "naked DNA" refers to a plasmid comprising a nucleotide sequence encoding a polypeptide of the present invention together with a short promoter region to control its production. It is called "naked" DNA because the plasmids are not carried in any delivery vehicle. When such a DNA plasmid enters a host cell, such as a eukaryotic cell, the proteins it encodes (such as NEV peptides of the present invention) are transcribed and translated within the cell.

### Non-viral delivery

Alternatively, the vectors comprising nucleotide sequences of the present invention may be introduced into suitable host cells using a variety of non-viral techniques known in the art, such as transfection, transformation, electroporation and biolistic transformation.

As used herein, the term "transfection" refers to a process using a non-viral vector to deliver a gene to a target mammalian cell.

Typical transfection methods include electroporation, DNA biolistics, lipid-mediated transfection, compacted DNA-mediated transfection, liposomes, immunoliposomes, lipofectin, cationic agent-mediated, cationic facial amphiphiles (CFAs) (Nature Biotechnology 1996 14; 556), multivalent cations such as spermine, cationic lipids or polylysine, 1, 2,-bis (oleoyloxy)-3-(trimethylammonio) propane (DOTAP)-cholesterol complexes (Wolff and Trubetskoy 1998 Nature Biotechnology 16: 421) and combinations thereof.

Uptake of naked nucleic acid constructs by mammalian cells is enhanced by several known transfection techniques for example those including the use of transfection agents. Examples of these agents include cationic agents (for example calcium phosphate and DEAE-dextran) and lipofectants (for example lipofectam™ and transfectam™). Typically, nucleic acid constructs are mixed with the transfection agent to produce a composition.

### Viral vectors

Alternatively, the vectors comprising nucleotide sequences of the present invention may be introduced into suitable host cells using a variety of viral techniques which are known in the art, such as for example infection with recombinant viral vectors such as retroviruses, herpes simplex viruses and adenoviruses.

In one embodiment the vector of the present invention is a recombinant viral vectors. Suitable recombinant viral vectors include but are not limited to adenovirus vectors, adeno-associated viral (AAV) vectors, herpes-virus vectors, a retroviral vector, lentiviral vectors, baculoviral vectors, pox viral vectors or parvovirus vectors (see Kestler et a/1999 Human Gene Ther 10(10):1619-32). In the case of viral vectors, gene delivery is mediated by viral infection of a target cell.

NEV as described herein may be used as a vector to deliver a gene of interest (GOI). For example NEV as described herein, or an element thereof, may be used as a vector to deliver a gene of interest to a cell, part of a cell, a tissue or an organism. In such embodiments, the NEV viral vector may be an attenuated virus, or may have one or more relevant and/or functional parts deleted.

### Retroviral vectors

Examples of retroviruses include: murine leukemia virus (MLV), human immunodeficiency virus (HIV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV).

In one embodiment vectors of the present invention for use in accordance with the present invention are recombinant viral vectors, in particular recombinant retroviral vectors (RRV) such as lentiviral vectors.

The term "recombinant retroviral vector" (RRV) refers to a vector with sufficient retroviral genetic information to allow packaging of an RNA genome, in the presence of packaging components, into a viral particle capable of infecting a target cell. Infection of the target cell includes reverse transcription and integration into the target cell genome. The RRV carries non-viral coding sequences which are to be delivered by the vector to the target cell. An RRV is incapable of independent replication to produce infectious retroviral particles within the final target cell. Usually the RRV lacks a functional *gag-pol* and/or *env* gene and/or other genes essential for replication. The vector of the present invention may be configured as a split-intron vector. A split intron vector is described in PCT patent application WO 99/15683.

A detailed list of retroviruses may be found in Coffin et al ("Retroviruses" 1997 Cold Spring Harbour Laboratory Press Eds: JM Coffin, SM Hughes, HE Varmus pp 758-763).

### Lentiviral vectors

Lentiviruses can be divided into primate and non-primate groups. Examples of primate lentiviruses include: the human immunodeficiency virus (HIV), the causative agent of human auto-immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

A distinction between the lentivirus family and other types of retroviruses is that lentiviruses have the capability to infect both dividing and non-dividing cells (Lewis et al 1992 EMBO. J 11: 3053-3058; Lewis and Emerman 1994 J. Virol. 68: 510-516). In contrast, other retroviruses - such as MLV - are unable to infect non-dividing cells such as those that make up, for example, muscle, brain, lung and liver tissue.

### Adenoviruses

As described herein, the features of adenoviruses may be combined with the genetic stability of retroviruses/lentiviruses which can be used to transduce target cells to become transient retroviral producer cells capable of stably infect neighbouring cells. Such retroviral producer cells which are engineered to express a NEV peptide of the present invention can be implanted in organisms such as animals for use in the treatment or prevention of diseases such as EIA, NEV and/or for the treatment or prevention of EIAV and/or NEV infection.

### Pox viruses

In one embodiment, vectors of the present invention for use in accordance with the present invention are recombinant pox viral vectors such as fowl pox virus (FPV), entomopox virus, vaccinia virus such as NYVAC, canarypox virus, MVA or other non-replicating viral vector systems such as those described for example in WO 95/30018.

### Replication vectors

The nucleotide sequences encoding the NEV peptides of the present invention may be incorporated into a recombinant replicable vector. The vector may be used to replicate the nucleotide sequence in a compatible host cell. Thus as described herein there is provided a method of making the polypeptide of the present invention by introducing a nucleotide sequence of the present invention into a replicable vector, introducing the vector into a compatible host cell, and growing the host cell under conditions which bring about replication of the vector. The vector may be recovered from the host cell.

### Expression vector

In one embodiment, a nucleotide sequence of present invention which is inserted into a vector is operably linked to a control sequence that is capable of providing for the expression of the coding sequence, such as the coding sequence of the NEV peptide as described herein by the host cell, i.e. the vector is an expression vector. The polypeptide produced by a host recombinant cell may be secreted or may be contained intracellularly depending on the sequence and/or the vector used. As will be understood by those of skill in the art, expression vectors containing the polypeptide coding sequences can be designed with signal sequences which direct secretion of the polypeptide coding sequences through a particular prokaryotic or eukaryotic cell membrane.

### Expression in vitro

The vectors of the present invention may be transformed or transfected into a suitable host cell and/or a target cell as described below to provide for expression of a polypeptide of the present invention. This process may comprise culturing a host cell and/or target cell transformed with an expression vector under conditions to provide for expression by the vector of a coding sequence encoding the NEV peptide of the present invention and optionally recovering the expressed polypeptide. The vectors may be for example, plasmid or virus vectors provided with an origin of replication, optionally a promoter for the expression of the said polynucleotide and optionally a regulator of the promoter. The vectors may contain one or more selectable marker genes, for example an ampicillin resistance gene in the case of a bacterial plasmid or a neomycin resistance gene for a mammalian vector. The expression of the polypeptide of the invention may be constitutive such that they are continually produced, or inducible, requiring a stimulus to initiate expression. In the case of inducible expression, polypeptide production can be initiated when required by, for example, addition of an inducer substance to the culture medium, for example dexamethasone or IPTG.

### Host/target cells

Host and/or target cells comprising nucleotide sequences of the present invention may be used to express the polypeptides of the present invention under *in vitro, in vivo* and *ex vivo* conditions.

The term "host cell and/or target cell" includes any cell derivable from a suitable organism which a vector is capable of transfecting or transducing. Examples of host and/or target cells can include cells capable of expressing the polypeptides of the present invention under *in vitro, in vivo* and *ex vivo* conditions. Examples of such cells include white blood cells such as macrophages. Further examples include stem cells, progenitor cells, endothelial cells respiratory airway epithelial cells, hepatocytes, muscle cells, cardiac myocytes, synoviocytes, primary mammary epithelial cells and post-mitotically terminally differentiated non-replicating cells such as monocytes, macrophages and/or neurons.

As described herein, the cell is an animal cell.

As described herein, the cell is an equine cell.

The term "organism" includes any suitable organism. In one embodiment, the organism is an animal. In one embodiment, the organism is an equine.

Although the polypeptides of the invention may be produced using prokaryotic cells as host cells, in one embodiment eukaryotic cells are used, for example yeast, plant, insect or mammalian cells, in particular equine cells. Suitable host cells include bacteria such as *E*. *coli,* yeast, plant, mammalian cell lines and other eukaryotic cell lines, for example insect Sf9 cells.

The present description also provides a method comprising transforming a host and/or target cell with a or the nucleotide sequence(s) of the present invention.

The term "transformed cell" means a host cell and/or a target cell having a modified genetic structure. With the present description, a cell has a modified genetic structure when a vector according to the present invention has been introduced into the cell.

Host cells and/or a target cells may be cultured under suitable conditions which allow expression of the polypeptide of the invention.

There is also provided a method comprising culturing a transformed host cell - which cell has been transformed with a or the nucleotide sequence(s) according to the present invention under conditions suitable for the expression of the polypeptide encoded by said nucleotide sequence(s).

The present description also provides a method comprising culturing a transformed host cell - which cell has been transformed with a or the nucleotide sequence(s) according to the present invention or a homologue, or fragment thereof - under conditions suitable for the expression of the polypeptide encoded by said nucleotide sequence(s); and then recovering said polypeptide from the transformed host cell culture.

The polypeptide of the present invention can be extracted from host cells by a variety of techniques known in the art, including enzymatic, chemical and/or osmotic lysis and physical disruption. The polypeptide may be purified and isolated in a manner known *per se.*

### Regulation of expression in vivo or in vitro or ex vivo

The present description also encompasses gene therapy whereby the NEV-encoding nucleotide sequence(s) of the present invention is regulated *in vivo* or *in vitro* or *ex vivo.* For example, expression regulation may be accomplished by administering compounds that bind to the NEV-encoding nucleotide sequence(s) of the present invention, or control regions associated with the NEV-encoding nucleotide sequence of the present invention, or its corresponding RNA transcript to modify the rate of transcription or translation.

### Control sequences

Control sequences operably linked to sequences encoding the NEV peptide of the present invention include promoters/enhancers and other expression regulation signals. These control sequences may be selected to be compatible with the host cell and/or target cell in which the expression vector is designed to be used. The control sequences may be modified, for example by the addition of further transcriptional regulatory elements to make the level of transcription directed by the control sequences more responsive to transcriptional modulators.

### Promoters

The term promoter is well-known in the art and is used in the normal sense of the art, e.g. as an RNA polymerase binding site. The term encompasses nucleic acid regions ranging in size and complexity from minimal promoters to promoters including upstream elements and enhancers.

The promoter is typically selected from promoters which are functional in mammalian, cells, although prokaryotic promoters and promoters functional in other eukaryotic cells may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of α-actin, β-actin, tubulin) or, alternatively, a tissue-specific manner (such as promoters of the genes for pyruvate kinase).

### Tissue-specific promoters

The promoters referred to herein may be tissue-specific promoters. That is, they are capable of driving transcription of an NEV-encoding nucleotide sequence(s) in one tissue while remaining largely "silent" in other tissue types.

### Inducible promoters

The promoters as described herein may also be promoters that respond to specific stimuli, for example promoters that bind steroid hormone receptors. Viral promoters may also be used, for example the Moloney murine leukaemia virus long terminal repeat (MMLV LTR) promoter, the rous sarcoma virus (RSV) LTR promoter or the human cytomegalovirus (CMV) IE promoter.

It may also be advantageous for the promoters to be inducible so that the levels of expression of the heterologous gene can be regulated during the life-time of the cell.

Inducible means that the levels of expression obtained using the promoter can be regulated.

### Enhancer

In addition, any of these promoters may be modified by the addition of further regulatory sequences, for example enhancer sequences. Chimeric promoters may also be used comprising sequence elements from two or more different promoters described above.

The term "enhancer" includes a DNA sequence which binds to other protein components of the transcription initiation complex and thus facilitates the initiation of transcription directed by its associated promoter.

### Pharmaceutical composition

The pharmaceutical composition may be any pharmaceutical composition. In one aspect, the pharmaceutical composition is to be administered orally, enterally, rectally or parenterally. For example, the composition may be an edible composition. "Edible" means a material that is approved for human or animal consumption.

Typically in equine medicine, pharmaceutical compositions are administered orally in the form of an oral paste or a powder to be dissolved into the drinking water. Further in equine medicine, pharmaceutical compositions may also be administered by injectable routes such as subcutaneous, intramuscular or intravenous injection.

The pharmaceutical compositions may be for animal usage in veterinary medicine.

Examples of such suitable excipients for the various different forms of pharmaceutical compositions described herein may be found in the "Handbook of Pharmaceutical Excipients, 2nd Edition, (1994), Edited by A Wade and PJ Weller.

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol, sorbitol. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s).

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride.

Preservatives, stabilizers, dyes and even flavouring agents may be provided in the pharmaceutical composition. Examples of preservatives include sodium benzoate, sorbic acid and esters of p-hydroxybenzoic acid. Antioxidants and suspending agents may be also used.

### Administration

The compositions, pharmaceutical compositions, or vectors of the present invention may be adapted for oral, rectal, vaginal, intrauterine, parenteral, intramuscular, intraperitoneal, intraarterial, intra-articular, intra-hoof, intrathecal, intrabronchial, subcutaneous, intradermal, intravenous, nasal, buccal or sublingual routes of administration.

In one aspect, the compositions, pharmaceutical compositions, or vectors of the present invention are adapted for oral, rectal, vaginal, parenteral, nasal, buccal or sublingual routes of administration.

In a further aspect, the compositions, pharmaceutical compositions, or vectors of the present invention are adapted for oral administration.

For oral administration, particular use is made of compressed tablets, pills, tablets, gels, drops, pastes, and capsules.

Other forms of administration comprise solutions or emulsions which may be injected intravenously, intraarterially, intraarticular, intrauterine, intrahoof, intrathecally, subcutaneously, intradermally, intraperitoneally, intracutane, subcutane, peroral, muscosal or intramuscularly, and which are prepared from sterile or sterilisable solutions. The compositions, pharmaceutical compositions, or vectors of the present invention may also be in form of suppositories, pessaries, suspensions, emulsions, pastes, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. In another example, the active ingredient can also be incorporated into an ointment consisting of a white wax or white soft paraffin base together with such stabilisers and preservatives as may be required.

Compositions, pharmaceutical compositions, or vectors may be formulated in unit dosage form, i.e., in the form of discrete portions containing a unit dose, or a multiple or sub-unit of a unit dose.

### Dosage

A person of ordinary skill in the art can easily determine an appropriate dose of the polypeptide or a polypeptide sequence encoding said polypeptide or polynucleotide sequence to administer to a subject without undue experimentation. Typically, a veterinarian will determine the actual dosage which will be most suitable for an individual patient and it will depend on a variety of factors including the activity of the polypeptide, the stability and length of action of the polypeptide, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited as described herein.

### Combinations

As described herein, the composition or the pharmaceutical composition comprises a combination of different peptides selected from the group consisting of SEQ ID NOs 1 to 76 and variants, homologues, fragments or derivatives thereof having at least 75%, 80%, 85%, 90%, 95%, 97%, 98%, or 99% identity to any one of SEQ ID NOs 1 to 76.

An example of a fragment of SEQ ID No 20 is QEQNPPPSVSLRSLFG (SEQ ID No 153).

An example of a fragment of SEQ ID No 95 is
CAGGAGCAGAACCCCCCCCCCAGCGTGAGCCTGAGGAGCCTGTTCGGC (SEQ ID No 154). SEQ ID No 154 encodes QEQNPPPSVSLRSLFG (SEQ ID No 153).

An example of a fragment of SEQ ID No 59 is WGIAHTTGIPGNSQGQAM (SEQ ID No 155).

An example of a fragment of SEQ ID No 134 is
TGGGGCATCGCCCACACCACCGGCATCCCCGGCAACAGCCAGGGCCAGGCCATG (SEQ ID No 156). SEQ ID No 156 encodes WGIAHTTGIPGNSQGQAM (SEQ ID NO 155).

For example, the composition or the pharmaceutical composition comprises a combination of different peptides selected from the group consisting of SEQ ID NOs 1 to 14 and variants, homologues, fragments or derivatives thereof having at least 75%, 80%, 85%, 90%, 95%, 97%, 98%, or 99% identity to any one of SEQ ID NOs 1 to 14.

As described herein, the composition or the pharmaceutical composition comprises at least two, three, four, five or six peptides selected from the group consisting of SEQ ID NOs 1 to 76 and variants, homologues, fragments or derivatives thereof having at least 75%, 80%, 85%, 90%, 95%, 97%, 98%, or 99% identity to any one of SEQ ID NOs 1 to 76.

For Example, the composition or the pharmaceutical composition comprises at least two, three, four, or five peptides selected from the group consisting of SEQ ID NOs 18, 34, 17, 20 and 59 and homologues thereof having at least 75%, 80%, 85%, 90%, 95%, 97%, 98%, or 99% identity to any one of SEQ ID NOs 18, 34, 17, 153 and 154.

As described herein, the composition or the pharmaceutical composition comprises at least two, three, four, or five peptides selected from the group consisting of SEQ ID NOs 18, 34, 17, 153 and 155.

In some embodiments, the compositions or the pharmaceutical composition further comprise at least one known EIAV peptide.

Examples of combinations of peptides include the composition or the pharmaceutical composition comprising (i) QEQNPPPSVSLRSLFG (from SEQ ID NO 20), (ii) EALDKIEEIQNKNKQK (SEQ ID NO 18) and (iii) LLNGSLAGESIIIR (SEQ ID NO 34). In another example, the composition or pharmaceutical composition comprises (i) QEQNPPPSVSLRSLFG (from SEQ ID NO 20), (ii) EALDKIEEIQNKNKQK (SEQ ID NO 18), (iii) LLNGSLAGESIIIR (SEQ ID NO 34), and (iv) QPSLPTGSEELK (SEQ ID NO 17) and/or (v) WGIAHTTGIPGNSQGQAM (from SEQ ID NO 59).

As described herein, the composition or the pharmaceutical composition comprises a combination of different polynucleotide sequences encoding different peptides selected from the group consisting of SEQ ID NOs 1 to 76 and variants, homologues, fragments or derivatives thereof having at least 75%, 80%, 85%, 90%, 95%, 97%, 98%, or 99% identity to any one of SEQ ID NOs 1 to 76.

As described herein, the composition or the pharmaceutical composition comprises at least two, three, four, five or six polynucleotide sequences encoding peptides selected from the group consisting of SEQ ID NOs 1 to 76 and variants, homologues, fragments or derivatives thereof having at least 75%, 80%, 85%, 90%, 95%, 97%, 98%, or 99% identity to any one of SEQ ID NOs 1 to 76.

For example, the composition or the pharmaceutical composition comprises at least two, three, four, or five polynucleotide sequences encoding peptides selected from the group consisting of SEQ ID NOs 18, 34, 17, 20 and 59 and homologues thereof having at least 75%, 80%, 85%, 90%, 95%, 97%, 98%, or 99% identity to any one of SEQ ID NOs 18, 34, 17, 20 and 59.

In one example, the composition or the pharmaceutical composition comprises at least two, three, four, or five peptides polynucleotide sequences encoding selected from the group consisting of SEQ ID NOs 18, 34, 17, 153 and 155.

As described herein, the composition or the pharmaceutical composition comprises a combination of different polynucleotide sequences selected from the group consisting of SEQ ID NOs 77 to 152 and variants, homologues, fragments or derivatives thereof having at least 75%, 80%, 85%, 90%, 95%, 97%, 98%, or 99% identity to any one of SEQ ID NOs 77 to 152.

As described herein, the composition or the pharmaceutical composition comprises at least two, three, four, five or six polynucleotide sequences selected from the group consisting of SEQ ID NOs 77 to 152 and variants, homologues, fragments or derivatives thereof having at least 75%, 80%, 85%, 90%, 95%, 97%, 98%, or 99% identity to any one of SEQ ID NOs 77 to 152.

For example, the composition or the pharmaceutical composition comprises at least two, three, four or five polynucleotide sequences selected from the group consisting of SEQ ID NOs 152, 109, 93, 95 and 134 and homologues thereof having at least 75%, 80%, 85%, 90%, 95%, 97%, 98%, or 99% identity to any one of SEQ ID NOs 152, 109, 93, 95 and 134.

As described herein, the composition or the pharmaceutical composition comprises at least two, three, four or five polynucleotide sequences selected from the group consisting of SEQ ID NOs 152, 109, 93, 154, and 156.

In some embodiments, the compositions or the pharmaceutical composition of the present invention further comprise at least one nucleotide sequence encoding a known EIAV peptide.

As described herein, the compositions or the pharmaceutical composition comprise a combination of at least one peptide as described herein and at least one nucleotide sequence of the present invention. In further embodiments the compositions or the pharmaceutical composition further comprise at least one known EAIV peptide and/or at least one nucleotide sequence encoding a known EIAV peptide.

### Fusion proteins

In any of the above combinations of peptides, the peptides may be combined in the form of a fusion protein - that is- the peptides of the invention may be covalently linked together.

In the fusion protein, the peptides of the invention may be directly fused together, or may be separated by a linker peptide.

Thus, one or more peptides selected from the group consisting of SEQ ID NOs: 1 to 76 or variants, homologues, fragments or derivatives thereof having at least 75%, 80%, 85%, 90%, 95%, 97%, 98%, or 99% identity to any of SEQ ID NOs 1 to 76 may be linked together as a fusion protein.

As described herein, any two peptides selected from the group consisting of SEQ ID NOs: 1 to 76 are linked together as a fusion protein.

For example, said two peptides may be SEQ ID NO: 18 and SEQ ID NO: 20, or variants, homologues, fragments or derivatives thereof having at least 75%, 80%, 85%, 90%, 95%, 97%, 98%, or 99% identity to SEQ ID NO: 18 and SEQ ID NO: 20.

For example, said two peptides may be SEQ ID NO: 18 and SEQ ID NO: 153, or variants, homologues, fragments or derivatives thereof having at least 75%, 80%, 85%, 90%, 95%, 97%, 98%, or 99% identity to SEQ ID NO: 18 and SEQ ID NO: 153.

In one embodiment, one or more peptides of the invention are linked together as a fusion protein by a linker peptide.

By "linker peptide" it is meant any collection or chain of amino acids that are covalently linked together. The linker peptide is also covalently linked to one or more peptides of the invention.

By way of example, a generic representation of a fusion protein of the invention comprising two peptides of the invention is as follows:
Peptide of the invention - linker peptide - peptide of the invention
where "-" represent a covalent bond.

In a preferred embodiment, the linker peptide is a continuous, unbranched chain of amino acids. Thus, linker peptides may be any length, for example 1, 2, 3, 4, 5, 6, 7, 8, 9, 10, 11, 12, 13, 14, 15, 16, 17, 18, 19, 20 or more amino acids in length.

In a preferred embodiment, the linker peptide is between 2 and 10 amino acid residues in length.

In a particularly preferred embodiment, said linker peptide is 3 amino acids in length.

The linker peptide may consist of or comprise any type of amino acid. For example, the linker peptide may consist of or comprise non-polar amino acids. In another embodiment, the linker peptide may consist of or comprise amino acid residues having aliphatic side chains.

In yet another embodiment, the linker peptide comprises a repetitive amino acid sequence. In a preferred embodiment, the linker peptide consists of alanine residues. In a particularly preferred embodiment, the linker peptide is AAA.

As described herein, the fusion protein may be represented by one or more of the following structures:
SEQ ID NO: 20 - linker peptide - SEQ ID NO: 18;
SEQ ID NO: 18 - linker peptide - SEQ ID NO: 20;
SEQ ID NO: 18 - linker peptide - SEQ ID NO: 153;
SEQ ID NO: 153 - linker peptide - SEQ ID NO: 18;
SEQ ID NO: 20 - AAA - SEQ ID NO: 18;
SEQ ID NO: 18 - AAA - SEQ ID NO: 20;
SEQ ID NO: 18 - AAA - SEQ ID NO: 153;
SEQ ID NO: 153 - AAA - SEQ ID NO: 18;

As described herein, the fusion protein consists of or comprises the following sequence:
EALDKIEEIQNKNKQKAAAQEQNPPPSVSLRSLFGNDPL (SEQ ID NO: 157).

As described herein, the fusion protein consists of or comprises the following sequence:
QEQNPPPSVSLRSLFGNDPLAAAEALDKIEEIQNKNKQK (SEQ ID NO: 158).

As described herein, the peptides as described herein which form part of the fusion protein may be truncated by any number of amino acids at either the N-terminus or the C-terminus. For example, the peptides may be truncated by 1, 2, 3, 4, 5, 6, 7, 8, 9 or 10 amino acids.

As described herein, the fusion protein consists of or comprises the following sequence:
EALDKIEEIQNKNKQKAAAQEQNPPPSVSLRSLFG (SEQ ID NO: 159).

As described herein, the composition of the invention may comprise a fusion protein consisting of or comprising the sequence set forth in SEQ ID NO: 157, SEQ ID NO: 158 or SEQ ID NO: 159.

Described herein a polynucleotide sequence encoding a fusion protein as described herein.

### Diagnostic kits

Described herein diagnostic methods and kits for the detection and measurement of one or more NEV peptides as described herein in a sample, the measurement of one or more antibodies of the present invention, the measurement of one or more polynucleotide sequences of the present invention.

The peptides as described herein may be used in a diagnostic method and kit to permit detection of circulating equine infectious anaemia virus (EIAV) and/or new equine virus (NEV) which, in certain situations, may indicate the progression of a disease state.

An antibody of the present invention that, for example, possesses high binding specificity can be used to establish easy to use kits for rapid, reliable, sensitive, and specific measurement of NEV peptides of the present invention in samples such as plasma, urine, and in cell culture media. The antibody of the present invention may also be used in a diagnostic method and kit to permit detection of circulating EIAV and/or NEV which, in certain situations, may indicate the progression of a disease state.

The polynucleotide sequences of the present invention may be used in a diagnostic method and kit to permit detection of circulating EIAV and/or NEV which, in certain situations, may indicate the progression of a disease state.

In one embodiment, the diagnostic method according to the present invention determines the presence or absence of an EIAV and/or NEV in an animal.

In one embodiment, the diagnostic method according to the present invention determines the viral titre of an equine infectious anaemia virus (EIAV) and/or NEV in an animal. This may enable the progression of EIAV/NEV infection and/or EIAV/NEV disease progression to be monitored.

The viral titre (e.g. NEV viral titre) may be determined in a sample from an animal such as a blood sample, a blood serum sample, a plasma sample, a saliva sample, a sputum sample, a urine sample, a semen sample, a biopsy sample, a fecal sample, a milk sample, a lymph node biopsy sample, and/or a sweat sample.

The kits and diagnostic methods of the present invention may include to the following techniques; competitive and non-competitive assays, radioimmunoassay, bioluminescence and chemiluminescence assays, fluorometric assays, infrared assays, sandwich assays, immunoradiometric assays, dot blots, enzyme linked assays including ELISA, microtiter plates, antibody coated strips, or dipsticks for rapid monitoring of urine or blood, and immunocytochemistry. For each kit the range, sensitivity, precision, reliability, specificity and reproducibility of the assay are established. Intraassay and interassay variation is established at 20%, 50% and 80% points on the standard curves of displacement or activity. The presence or absence in a sample from an animal of antibodies which are capable of binding to one or more NEV peptides of the present invention can be determined using, for example, enzyme-linked immunosorbent assays (ELISA).

The presence or absence in a sample from an animal of one or more NEV peptides according to the present invention can be determined using, for example, enzyme-linked immunosorbent assays (ELISA).

The ELISA assay may be an indirect ELISA in which peptides of the present invention are immobilized onto, for example, a microtitre plate.

The ELISA assay may be a sandwich ELISA in which antibodies of the present invention are immobilized onto, for example, a microtitre plate. The antibodies may be obtained from the antisera of equines (e.g. horses) which have been infected with NEV peptides of the present invention and/or NEV viruses as mentioned herein. Alternatively, the antibodies may be obtained from animals, such as mice, which have been infected with NEV peptides of the present invention and/or NEV viruses as mentioned herein.

The presence or absence in a sample of one or more polynucleotide sequence encoding one or more peptides according to the present invention can be determined by using, for example, PCR (such as quantitative PCR, digital PCR), nucleic acid isothermal amplification, (such as Loop mediated isothermal amplification), and/or hybridisation techniques (such as Southern blotting).

A primer for use, for example, in a PCR assay may be based on a polynucleotide sequence encoding a peptide as described herein and/or a polynucleotide sequence of the present invention.

Typically primers are between 15 to 40 nucleotides in length. As described herein, the primers are between 18 to 26 nucleotides in length.

A probe for use, for example, in a hybridisation assay (such as Southern hybridisation) may be based on a polynucleotide sequence encoding a peptide as described herein and/or a polynucleotide sequence of the present invention.

Typically probes are at least 15 nucleotides in length. As described herein, the primers are at least 20, 30 or 40 nucleotides in length. As described herein, the primers are between 15 and 200 nucleotides in length.

A combination of primers and/or probes may be used to determine the presence or absence in a sample of one or more polynucleotide sequence encoding one or more peptides as described herein.

The sample as referred to herein is obtained/obtainable from an animal. In one embodiment, the sample is obtained/obtainable from an equine such as a horse, a donkey, a mule, a hinny, or a zebra.

The sample may be blood, blood serum, plasma, saliva, sputum, urine, fecal biopsy, lymph node biopsy, milk, semen, and/or sweat.

By determining the presence of NEV peptides of the present invention and/or the presence of NEV antibodies of the present invention and/or the presence of polynucleotide sequences encoding peptides of the present invention in a sample from an animal it can be determined that an animal has been infected with an equine infectious anaemia virus (EIAV) and/or NEV.

The diagnostic methods of the present invention are typically carried out *ex vivo* or *in vitro.*

Compositions according to the invention and/or a vector according to the invention, and/or a pharmaceutical composition according to the invention, and/or an antibody according to the invention may also be used in a kit or assay for the diagnosis or prevention of EIAV and/or NEV, and/or the diagnosis or prevention of an infection by an EIAV and/or NEV in an animal.

### Polynucleotide sequence

Described herein are polynucleotide sequences encoding NEV peptides.

The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be double-stranded or single-stranded whether representing the sense or anti-sense strand.

The term "nucleotide sequence" in relation to the present invention includes genomic DNA, cDNA, synthetic DNA, and RNA. In one embodiment it means cDNA sequence coding for the present invention.

In a preferred embodiment, the nucleotide sequence of the present invention does not include the native nucleotide sequence when in its natural environment and when it is linked to its naturally associated sequence(s) that is/are also in its/their natural environment. For ease of reference, herein this embodiment is called the "non-native nucleotide sequence". In this regard, the term "native nucleotide sequence" means an entire nucleotide sequence that is in its native environment and when operatively linked to an entire promoter with which it is naturally associated, which promoter is also in its native environment. However, the amino acid sequence as described herein can be isolated and/or purified post expression of a nucleotide sequence in its native organism. However, the amino acid sequence as described herein may be expressed by a nucleotide sequence in its native organism but wherein the nucleotide sequence is not under the control of the promoter with which it is naturally associated within that organism.

Typically, the nucleotide sequence of the present invention is prepared using recombinant DNA techniques (i.e. recombinant DNA). However, in an alternative embodiment of the invention, the nucleotide sequence of the present invention could be synthesised, in whole or in part, using chemical methods well known in the art (see Caruthers MH et al., (1980) Nuc Acids Res Symp Ser 215-23 and Horn T et al., (1980) Nuc Acids Res Symp Ser 225-232).

### Preparation of the nucleotide sequence

A nucleotide sequence encoding either a peptide as described herein may be identified and/or isolated and/or purified from any cell or organism producing said peptide. Various methods are well known within the art for the identification and/or isolation and/or purification of nucleotide sequences. By way of example, DNA amplification techniques to prepare more of a sequence may be used once a suitable sequence has been identified and/or isolated and/or purified.

By way of further example, a genomic DNA and/or cDNA library may be constructed using chromosomal DNA or messenger RNA from the organism producing the peptide. If the amino acid sequence is known, labelled oligonucleotide probes may be synthesised and used to identify clones from the genomic library prepared from the organism. Alternatively, a labelled oligonucleotide probe containing sequences homologous to a similar known gene could be used to identify clones. In the latter case, hybridisation and washing conditions of lower stringency are used.

Alternatively, clones comprising the peptides of the present invention could be identified by inserting fragments of genomic DNA into an expression vector, such as a plasmid, transforming bacteria with the resulting genomic DNA library, and then plating the transformed bacteria onto agar plates containing a substrate for the peptide thereby allowing clones expressing the peptide to be identified.

In a yet further alternative, the nucleotide sequence encoding the peptide may be prepared synthetically by established standard methods, e.g. the phosphoroamidite method described by Beucage S.L. et al., (1981) Tetrahedron Letters 22, p 1859-1869, or the method described by Matthes et al., (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al., (Science (1988) 239, pp 487-491).

### Amino acid sequences

The amino acid sequence as described herein may be prepared/isolated from a suitable source, or it may be made synthetically or it may be prepared by use of recombinant DNA techniques.

The peptide as described herein may be used in conjunction with other peptide (e.g. known EIAV peptides). Thus described herein is a combination of peptides wherein the combination comprises a peptide as described herein and another peptide, which may be another peptide as described herein.

Preferably the amino acid sequence as described herein is not a native peptide. In this regard, the term "native peptide" means an entire peptide that is in its native environment and when it has been expressed by its native nucleotide sequence.

### Antibodies

Described herein amino acids that are immunologically reactive with the NEV peptides described herein.

Antibodies may be produced by standard techniques, such as by immunisation with the peptide of the invention or by using a phage display library.

For the purposes of this invention, the term "antibody", unless specified to the contrary, includes, polyclonal, monoclonal, chimeric, single chain, Fab fragments, fragments produced by a Fab expression library, as well as mimetics thereof. Such fragments include fragments of whole antibodies which retain their binding activity for a target substance, Fv, F(ab') and F(ab')₂ fragments, as well as single chain antibodies (scFv), fusion proteins and other synthetic proteins which comprise the antigen-binding site of the antibody. Furthermore, the antibodies and fragments thereof may be humanised antibodies. Neutralising antibodies, i.e., those which inhibit biological activity of the substance polypeptides, are especially preferred for diagnostics and therapeutics.

If polyclonal antibodies are desired, a selected mammal (e.g., mouse, rabbit, goat, horse, llama, etc.) and/or avian is immunised with the sequence of the present invention (or a sequence comprising an immunological epitope thereof). Depending on the host species, various adjuvants may be used to increase immunological response.

Serum from the immunised animal is collected and treated according to known procedures. If serum containing polyclonal antibodies to the peptide of the present invention (or a sequence comprising an immunological epitope thereof) contains antibodies to other antigens, the polyclonal antibodies can be purified by immunoaffinity chromatography. Techniques for producing and processing polyclonal antisera are known in the art. In order that such antibodies may be made, the invention also provides polypeptides of the invention or fragments thereof haptenised to another polypeptide for use as immunogens in animals or humans.

Monoclonal antibodies directed against the sequence of the present invention (or a sequence comprising an immunological epitope thereof) can also be readily produced by one skilled in the art and include the hybridoma technique Koehler and Milstein (1975 Nature 256:495-497), the human B-cell hybridoma technique (Kosbor et al., (1983) Immunol Today 4:72; Cote et al., (1983) Proc Natl Acad Sci 80:2026-2030) and the EBV-hybridoma technique (Cole et al., (1985) Monoclonal Antibodies and Cancer Therapy, Alan Rickman Liss Inc, pp 77-96).

In addition, techniques developed for the production of "chimeric antibodies", the splicing of mouse antibody genes to human antibody genes to obtain a molecule with appropriate antigen specificity and biological activity may be used (Morrison et al., (1984) Proc Natl Acad Sci 81:6851-6855; Neuberger et al., (1984) Nature 312:604-608; Takeda et al., (1985) Nature 314:452-454).

Alternatively, techniques described for the production of single chain antibodies (US Patent No. 4,946,779) can be adapted to produce the substance specific single chain antibodies.

Antibody fragments which contain specific binding sites for the substance may also be generated. For example, such fragments include, but are not limited to, the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed to allow rapid and easy identification of monoclonal Fab fragments with the desired specificity (Huse WD et al., (1989) Science 256:1275-128 1).

### Sequence Identity or Sequence Homology

The terms "peptide", "polypeptide", "polypeptide sequence", "protein" and "amino acid sequence" are used interchangeably herein.

The terms "polynucleotide sequence" and "nucleotide sequence" are used interchangeably herein.

Described herein is the use of sequences having a degree of sequence identity or sequence homology with amino acid sequence(s) of a polypeptide described herein (e.g. variants, homologues and derivatives) or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

In the present context, a homologous sequence is taken to include an amino acid or a nucleotide sequence which may be at least 50, 60, 70, 75, 80, 85 or 90% identical, in some embodiments at least 95, 96, 97, 98 or 99% identical to the subject sequence. Although homology can also be considered in terms of similarity (i.e. amino acid residues having similar chemical properties/functions), in the context of the present invention it is preferred to express homology in terms of sequence identity.

In some embodiments, a homologous sequence is taken to include an amino acid sequence or nucleotide sequence which has one or several additions, deletions and/or substitutions compared with the subject sequence.

Described herein is the use of a protein whose amino acid sequence is represented herein or a protein derived from this (parent) protein by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein.

Described herein is the use of a nucleic acid sequence (or gene) encoding a protein whose amino acid sequence is represented herein or encoding a protein derived from this (parent) protein by substitution, deletion or addition of one or several amino acids, such as 2, 3, 4, 5, 6, 7, 8, 9 amino acids, or more amino acids, such as 10 or more than 10 amino acids in the amino acid sequence of the parent protein and having the activity of the parent protein.

In the present context, a homologous sequence is taken to include a nucleotide sequence which may be at least 50, 60, 70, 75, 85 or 90% identical, in some embodiments at least 95, 96, 97, 98 or 99% identical to a nucleotide sequence encoding a polypeptide described herein (the subject sequence). Typically, the homologues will comprise the same or equivalent sequences that code for the domain(s) etc. as the subject sequence. Although homology can also be considered in terms of similarity (i.e. amino acid residues having similar chemical properties/functions), in the context of the present invention it is preferred to express homology in terms of sequence identity.

The homologous amino acid sequence and/or nucleotide sequence may provide and/or encode a polypeptide which retains the functional activity and/or enhances the activity of the polypeptide.

As described herein, an amino acid sequence as described herein has at least 50, 60, 70, 75, 80, 85 or 90% identity, in some embodiments at least 95, 96, 97, 98 or 99% identity to the subject sequence.

As described herein, a nucleotide sequence as described herein has at least 50, 60, 70, 75, 80, 85 or 90% identity, in some embodiments at least 95, 96, 97, 98 or 99% identity to the subject sequence.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalizing unduly the overall homology score. This is achieved by inserting "gaps" in the sequence alignment to try to maximize local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimized alignments with fewer gaps. Most alignment programs allow the gap penalties to be modified. Typically the default values are used when using such software for sequence comparisons.

Calculation of maximum % homology therefore firstly requires the production of an optimal alignment, taking into consideration gap penalties. A suitable computer program for carrying out such an alignment is the Vector NTI (Invitrogen Corp.). Examples of software that can perform sequence comparisons include the BLAST package (see Ausubel et al 1999 Short Protocols in Molecular Biology, 4th Ed - Chapter 18), BLAST 2 (see FEMS Microbiol Lett 1999 174(2): 247-50; FEMS Microbiol Lett 1999 177(1): 187-8 and tatiana@ncbi.nlm.nih.gov). FASTA (Altschul et al 1990 J. Mol. Biol. 403-410) and AlignX for example. At least BLAST, BLAST 2 and FASTA are available for offline and online searching (see Ausubel *et al* 1999, pages 7-58 to 7-60).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. Vector NTI programs generally use either the public default values or a custom symbol comparison table if supplied (see user manual for further details). For some applications, it is preferred to use the default values for the Vector NTI package.

Alternatively, percentage homologies may be calculated using the multiple alignment feature in Vector NTI (Invitrogen Corp.), based on an algorithm, analogous to CLUSTAL (Higgins DG & Sharp PM (1988), Gene 73(1), 237-244).

Once the software has produced an optimal alignment, it is possible to calculate % homology, for example % sequence identity. The software typically does this as part of the sequence comparison and generates a numerical result.

Should Gap Penalties be used when determining sequence identity, then the following parameters can be used for pairwise alignment for example:

| FOR BLAST | |
|---|---|
| GAP OPEN | 0 |
| GAP EXTENSION | 0 |

| FOR CLUSTAL | DNA | PROTEIN | |
|---|---|---|---|
| WORD SIZE | 2 | 1 | K triple |
| GAP PENALTY | 15 | 10 | |
| GAP EXTENSION | 6.66 | 0.1 | |

In one embodiment, CLUSTAL may be used with the gap penalty and gap extension set as defined above.

In one embodiment, the degree of identity with regard to a nucleotide sequence is determined over at least 20 contiguous nucleotides, for example over at least 30 contiguous nucleotides, for example over at least 40 contiguous nucleotides, for example over at least 50 contiguous nucleotides, for example over at least 60 contiguous nucleotides, for example over at least 100 contiguous nucleotides, for example over at least 200 contiguous nucleotides, for example over at least 300 contiguous nucleotides.

In one embodiment, the degree of identity with regard to a nucleotide sequence may be determined over the whole sequence.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The present invention also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, B-alanine*, L-α-amino butyric acid*, L-γ-amino butyric acid*, L-α-amino isobutyric acid*, L-ε-amino caproic acid^{#}, 7-amino heptanoic acid*, L-methionine sulfone^{#*}, L-norleucine*, L-norvaline*, p-nitro-L-phenylalanine*, L-hydroxyproline^{#}, L-thioproline*, methyl derivatives of phenylalanine (Phe) such as 4-methyl-Phe*, pentamethyl-Phe*, L-Phe (4-amino)^{#}, L-Tyr (methyl)*, L-Phe (4-isopropyl)*, L-Tic (1,2,3,4-tetrahydroisoquinoline-3-carboxyl acid)*, L-diaminopropionic acid ^{#} and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The nucleotide sequences for use in the present invention may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide sequences of the present invention.

Described herein is the use of nucleotide sequences that are complementary to the sequences presented herein, or any derivative or fragment thereof. If the sequence is complementary to a fragment thereof then that sequence can be used as a probe to identify similar coding sequences in other organisms etc.

Polynucleotides which are not 100% homologous to the sequences of the present invention but are described herein can be obtained in a number of ways. Other variants of the sequences described herein may be obtained for example by probing DNA libraries made from a range of individuals, for example individuals from different populations. In addition, other homologues may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the invention.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

Polynucleotides (nucleotide sequences) described herein may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides described herein.

Polynucleotides such as DNA polynucleotides and probes according to the invention may be produced recombinantly, synthetically, or by any means available to those of skill in the art. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. The primers may be designed to contain suitable restriction enzyme recognition sites so that the amplified DNA can be cloned into a suitable cloning vector.

### Hybridisation

The term "hybridisation" as used herein shall include "the process by which a strand of nucleic acid joins with a complementary strand through base pairing" as well as the process of amplification as carried out in polymerase chain reaction (PCR) technologies.

In one embodiment, nucleotide sequences can hybridise to a probe as described herein under stringent conditions (e.g. 50°C and 0.2xSSC).

In another embodiment, nucleotide sequences can hybridise to a probe as described herein under high stringent conditions (e.g. 65°C and 0.1xSSC).

### EXAMPLES

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Col d Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; and E. M. Shevach and W. Strober, 1992 and periodic supplements, Current Protocols in Immunology, John Wiley & Sons, New York, NY.

The inventors have developed a peptide based immunodiagnostic assay that has been validated by comparing results on reference and field samples with the immunoblot test. The peptide ELISA has similar sensitivity and specificity of the immunoblot techniques for the detection of EIAV infected horses. The ELISA is based in peptides identified in purified viral particles obtained from EIAV AGID negative field samples. The purified viral particles were obtained from primary macrophages cell lines established from seropositive horses. The EIAV AGID negative strains were also considered negative in ELISAs directed to p26 core protein as also to the dual antigen gp45 ELISAs (Eradikit ELISA, In3 Diagnostics, Italy).

The retrieved peptides were short peptides with generally less than 30 amino acids. These peptides had a good sensitivity and specificity. More than 62 peptides were identified from EIAV AGID-negative viral purified strains by mass spectrometry; these peptide were screened against public viral databases (UNIPROT KB or NCBI) and twenty three peptides of interest were selected which were found that were present in a EIAV AGID negative gene bank that belongs to Equigerminal - these peptides are shown in Table 1.

None of the identified peptides belonged to the EIAV proteome. 45 of the 62 peptides were found to have sequence identity with retroviral peptides. 26 of the 62 peptides were found to have sequence identity to part of the HIV-1 proteome,

**Table 1 - Table 1 details peptide sequences and the polynucleotide sequence which encodes the peptide. For example, the polynucleotide sequence shown as SEQ ID NO 77 encodes the peptide shown as SEQ ID NO 1.**

| **Peptide** | **Polynucleotide sequence** |
|---|---|
| SRLLESRGPTTSEK (SEQ ID NO 1) | AGCAGGCTGCTGGAGAGCAGGGGCCCCACCACCAGCGAGA AG (SEQ ID NO 77) |
| TLKEVLGGEAAVR (SEQ ID NO 2) | ACCCTGAAAGAGGTTCTTGGTGGTGAAGCAGCCGTGCGC (SEQ ID NO 78) |
| PCRSKNILPV (SEQ ID NO 3) | CCCTGCCGGAGCAAGAACATCCTGCCCGTG (SEQ ID NO 79) |
| DPCVHSVDVLLR (SEQ ID NO 4) | GACCCCTGCGTGCACAGCGTGGACGTGCTGCTGAGG (SEQ ID NO 80) |
| STFPPTPV (SEQ ID NO 5) | TCCACATTTCCTCCCACTCCCGTC (SEQ ID NO 81) |
| NAPLLSKVSP (SEQ ID NO 6) | AATGCCCCATTACTCTCAAAAGTGTCCCCA (SEQ ID NO 82) |
| MAQCIVNR (SEQ ID NO 7) | ATGGCACAGTGTATCGTTAACCGC (SEQ ID NO 83) |
| KPNVEGRYGLSRSETNK (SEQ ID NO 8) | AAGCCCAACGTGGAGGGCAGGTACGGCCTGAGCAGGAGCG AGACCAACAAG(SEQ ID NO 84) CAGCAAGGCCCAGAGGCGCCACTGCCCAGCCTGCAGGTTG CAGAAGTGCCTAAA (SEQ ID NO 85) |
| QQGPEAPLPSLQVAEVPK (SEQ ID NO 9) | |
| PHAGSTQTEWPK (SEQ ID NO 10) | CCCCATGCGGGCTCCACTCAGACAGAGTGGCCCAAA (SEQ ID NO 86) |
| PIQINACK (SEQ ID NO 11) | CCAATACAGATCAATGCTTGCAAA (SEQ ID NO 87) |
| GGMRESWDGGQV (SEQ ID NO 12) | GGTGGAATGCGTGAGTCATGGGATGGAGGACAAGTT (SEQ ID NO 88) |
| ESLVSKGFR (SEQ ID NO 13) | GAATCTCTGGTGTCAAAAGGGTTTCGG (SEQ ID NO 89) |
| CSLVLGEMQIK (SEQ ID NO 14) | TGCTCATTAGTCCTTGGGGAGATGCAAATCAAA (SEQ ID NO 90) |
| LDKWEKIR (SEQ ID NO 15) | CTGGACAAGTGGGAGAAGATCAGG (SEQ ID NO 91) |
| QMMIAASEK (SEQ ID NO 16) | CAGATGATGATCGCCGCCAGCGAGAAG(SEQ ID NO 92) |
| QPSLPTGSEELK (SEQ ID NO 17) | CAGCCCAGCCTGCCCACCGGCAGCGAGGAGCTGAAG (SEQ ID NO |
| EALDKIEEIQNKNKQK (SEQ ID NO 18) | GAGGCCCTGGACAAGATCGAGGAGATCCAGAACAAGAACAA GCAGAAG (SEQ ID NO 152) |
| PPIPVGGIYK (SEQ ID NO 19) | CCCCCCATCCCCGTGGGCGGCATCTACAAG (SEQ ID NO 94) |
| QEQNPPPSVSLRSLFGNDPL( SEQ ID NO 20) | CAGGAGCAGAACCCCCCCCCAGCGTGAGCCTGAGGAGCC TGTTCGGCAACGACCCCCTG(SEQ ID NO 95) |
| SEQ 20) EKIEQLR (SEQ ID NO 21) | GAGAAGATCGAGCAGCTGAGG (SEQ ID NO 96) |
| DLLLIVAR (SEQ ID NO 22) | GACCTGCTGCTGATCGTGGCCAGG (SEQ ID NO 97) |
| IVELLGR (SEQ ID NO 23) | ATCGTGGAGCTGCTGGGCAGG (SEQ ID NO 98) |
| IWGNVTWMEWER (SEQ ID NO 24) | ATCTGGGGCAACGTGACCTGGATGGAGTGGGAGAGG (SEQ ID NO 99) |
| LKDLFPNK (SEQ ID NO 25) | CTGAAGGACCTGTTCCCCAACAAG (SEQ ID NO 100) |
| AKLEESFPGK (SEQ ID NO 26) | GCCAAGCTGGAGGAGAGCTTCCCCGGCAAG (SEQ ID NO 101) |
| NGSLAGESIIIR (SEQ ID NO 27) | AACGGCAGCCTGGCCGGCGAGAGCATCATCATCAGG (SEQ ID NO 102) |
| NITFNSSAGGDLEIT (SEQ ID NO 28) | AACATCACCTTCAACAGCAGCGCCGGCGGCGACCTGGAGAT CACC (SEQ ID NO 103) |
| AVILLLDRLR (SEQ ID NO 29) | GCCGTGATCCTGCTGCTGGACAGGCTGAGG (SEQ ID NO 104) |
| GPGIHIGKR (SEQ ID NO 30) | AAAA (SEQ ID NO 105) |
| VIICSASK (SEQ ID NO 31) | GTGATCATCTGCAGCGCCAGCAAG (SEQ ID NO 106) |
| ESFPNK (SEQ ID NO 32) | GAGAGCTTCCCCAACAAG (SEQ ID NO 107) |
| FGNKTTIIFTK (SEQ ID NO 33) | TTCGGCAACAAGACCACCATCATCTTCACCAAG (SEQ ID NO 108) |
| LLNGSLAGESIIIR (SEQ ID NO 34) | CTGCTGAACGGCAGCCTGGCCGGCGAGAGCATCATCATCA GG (SEQ ID NO 109) GTGAACGTGAGCGTGACCAACAACAACACCACCACCAACGT G (SEQ ID NO 110) |
| VNVSVTNNNTTTNV (SEQ ID NO 35) | |
| AILHILRR (SEQ ID NO 36) | GCCATCCTGCACATCCTGAGGAGG (SEQ ID NO 111) |
| DLLALDK (SEQ ID NO 37) | GACCTGCTGGCCCTGGACAAG (SEQ ID NO 112) |
| IGCQHSRIGITLPR (SEQ ID NO 38) | |
| TLQYLALTALVTPK (SEQ ID NO 39) | ATCGGCTGCCAGCACAGCAGGATCGGCATCACCCTGCCCA GG (SEQ ID NO 113) ACCCTGCAGTACCTGGCCCTGACCGCCCTGGTGACCCCCAA G (SEQ ID NO 114) CCCACCACCCCCGTGACCCCCGCCCCCGGCGTGGGCGAGA TCAGCAAGGAGCTGGCCCAG GGCAAG (SEQ ID NO 115) |
| PTTPVTPAPGVGEISKELAQG K (SEQ ID NO 40) | |
| VVSSALQYLIPR (SEQ ID NO 41) | GTGGTGAGCAGCGCCCTGCAGTACCTGATCCCCAGG (SEQ ID NO 116) |
| PVWAEPVK (SEQ ID NO 42) | CCCGTGTGGGCCGAGCCCGTGAAG (SEQ ID NO 117) |
| VKGNDLLK (SEQ ID NO 43) | GTGAAGGGCAACGACCTGCTGAAG (SEQ ID NO 118) |
| EDLNLQDWK (SEQ ID NO 44) | GAGGACCTGAACCTGCAGGACTGGAAG (SEQ ID NO 119) |
| LFVSVLQR (SEQ ID NO 45) | CTGTTCGTGAGCGTGCTGCAGAGG (SEQ ID NO 120) |
| AAEQKASPPSLTPK (SEQ ID NO 46) | GCCGCCGAGCAGAAGGCCAGCCCCCCCAGCCTGACCCCCA AG (SEQ ID NO 121) |
| PLSLPLKI (SEQ ID NO 47) | CCCCTGAGCCTGCCCCTGAAGATC (SEQ ID NO 122) |
| FPSIVGRPR (SEQ ID NO 48) | TTCCCCAGCATCGTGGGCAGGCCCAGG (SEQ ID NO 123) |
| IWHHTFYNELR (SEQ ID NO 49) | ATCTGGCACCACACCTTCTACAACGAGCTGAGG (SEQ ID NO 124) |
| MTQIMFETF (SEQ ID NO 50) | ATGACCCAGATCATGTTCGAGACCTTC (SEQ ID NO 125) |
| EEEVAALVIDNGSGMCK (SEQ ID NO 51) | |
| VAPEEHPVLLTEAPLNPK (SEQ ID NO 52) | GAGGAGGAGGTGGCCGCCCTGGTGATCGACAACGGCAGCG GCATGTGCAAG (SEQ ID NO 126) GTGGCCCCCGAGGAGCACCCCGTGCTGCTGACCGAGGCCC CCCTGAACCCCAAG (SEQ ID NO 127) |
| AVFPSIVGRPR (SEQ ID NO 53) | GCCGTGTTCCCCAGCATCGTGGGCAGGCCCAGG (SEQ ID NO 128) |
| YPIEHGIVTNWDDMEK (SEQ ID NO 54) | TACCCCATCGAGCACGGCATCGTGACCAACTGGGACGACAT GGAGAAG (SEQ ID NO 129) |
| AVFPSIVGR (SEQ ID NO 55) | GCCGTGTTCCCCAGCATCGTGGGCAGG(SEQ ID NO 130) |
| IGRKDAERQLLSPGNAR (SEQ ID NO 56) | ATCGGCAGGAAGGACGCCGAGAGGCAGCTGCTGAGCCCCG GCAACGCCAGG (SEQ ID NO 131) |
| DSYVGDEAQSKR (SEQ ID NO 57) | GACAGCTACGTGGGCGACGAGGCCCAGAGCAAGAGG (SEQ ID NO 132) |
| RGILTLK (SEQ ID NO 58) | AGGGGCATCCTGACCCTGAAG (SEQ ID NO 133) |
| WGIAHTTGIPGNSQGQAMVE R (SEQ ID NO 59) | TGGGGCATCGCCCACACCACCGGCATCCCCGGCAACAGCC AGGGCCAGGCCATGGTGGAG AGG (SEQ ID NO 134) |
| HLVDQLIRDLK (SEQ ID NO 60) | CACCTGGTGGACCAGCTGATCAGGGACCTGAAG (SEQ ID NO 135) |
| YEQLQLQAR (SEQ ID NO 61) | TACGAGCAGCTGCAGCTGCAGGCCAGG (SEQ ID NO 136) |
| TITLEVEPSDTIENVK (SEQ ID NO 62) | ACCATCACCCTGGAGGTGGAGCCCAGCGACACCATCGAGA ACGTGAAG (SEQ ID NO 137) |
| INRELLK (SEQ ID NO 63) | ATCAACAGGGAGCTGCTGAAG (SEQ ID NO 138) |
| QTIIPTNKDVDEK (SEQ ID NO 64) | CAGACCATCATCCCCACCAACAAGGACGTGGACGAGAAG (SEQ ID NO 139) |
| KNYGKLDK (SEQ ID NO 65) | AAGAACTACGGCAAGCTGGACAAG (SEQ ID NO 140) |
| TTISFSK (SEQ ID NO 66) | ACCACCATCAGCTTCAGCAAG (SEQ ID NO 141) |
| QDRTTISFSK (SEQ ID NO 67) | CAGGACAGGACCACCATCAGCTTCAGCAAG (SEQ ID NO 142) |
| AQGRAIAHK (SEQ ID NO 68) | GCCCAGGGCAGGGCCATCGCCCACAAG (SEQ ID NO 143) |
| MAQTQLVPVK (SEQ ID NO 69) | ATGGCCCAGACCCAGCTGGTGCCCGTGAAG (SEQ ID NO 144) |
| QELIPPCK (SEQ ID NO 70) | CAGGAGCTGATCCCCCCCTGCAAG (SEQ ID NO 145) |
| DIVLLENGK (SEQ ID NO 71) | GACATCGTGCTGCTGGAGAACGGCAAG (SEQ ID NO 146) |
| LPPLSILK (SEQ ID NO 72) | CTGCCCCCCCTGAGCATCCTGAAG (SEQ ID NO 147) |
| IFLINLAFLIK (SEQ ID NO 73) | ATCTTCCTGATCAACCTGGCCTTCCTGATCAAG (SEQ ID NO 148) |
| NRLEILK (SEQ ID NO 74) | AACAGGCTGGAGATCCTGAAG (SEQ ID NO 149) |
| ADDVAVLQDALGR (SEQ ID NO 75) | GCCGACGACGTGGCCGTGCTGCAGGACGCCCTGGGCAGG (SEQ ID NO 150) |
| LNKSLEQLR (SEQ ID NO 76) | CTGAACAAGAGCCTGGAGCAGCTGAGG (SEQ ID NO 151) |

The inventors carried out ELISA assays using the above identified short synthetic peptides in Table 1, which are easy to produce, and control and which can be used as target antigens for the detection of anti-NEV antibodies.

This trend towards smaller antigens however is accompanied by a risk that the synthesized epitope is not able to assume a rigid conformation that is recognized by the antibody. Although the number of serum samples tested in each of these cases is very limited, specificity was found to be very high (95%-100%) with small synthetic peptides but the overall sensitivity varied between 80 and 100%. In the only example where 100% sensitivity was attained only a few samples had been tested.

Also, the use of a synthetic peptide based ELISA can also overcome the use of the impurities present in the antigens preparations resultant from protein production in bacterial or mammalian cells that are also responsible for unacceptably high levels of false positive results which can cause healthy horse to be eliminated by common policies of EIAV control.

### Material and Methods

### EIAV seropositive and AGID negative sera.

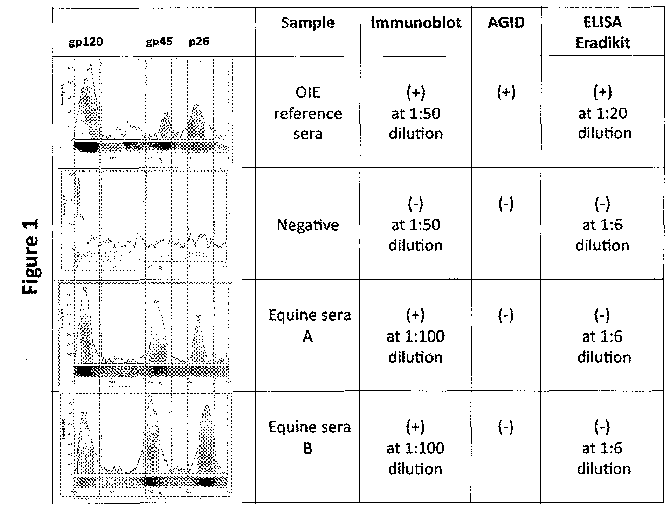

Equine infectious anemia (EIAV) seropositiveness was first determined by immunoblot (Figure 1).

EIAV Wyoming (EIAV_{WYO} viral strain (ATCC VR-778) Malmquist et al., 1973) was cultured in a permissive macrophage-like cell line EML-3C cell line (ATCC CRL-2996) previously established for EIAV replication studies (Fidalgo-Carvalho et al., 2009). The cells were seeded in T75cm² flasks prior to infection. After two days cell cultures were infected with EIAV_{WYO}. Tissue culture supernatants were screened for Reverse Transcriptase (RT) activity by using the Enzcheck reverse transcriptase kit (Molecular Probes, Invitrogen) according to manufacturer instructions. RT positive cell culture supernatants were submitted to sequential centrifugations steps. To remove cell debris supernatants were centrifuged at 500 rcf for 10 minutes, supernatants moved to a clean tube and centrifuged again at 3200 rcf for 20 minutes. The RT positive supernatants were then filtered by a syringe minisart 0.45 micron filter (Sartorius) and viral particles concentrated to 0.5-1 mL in a Vivaspin 6 >100,000 MWCO PES (Sartorius) accordingly manufacturers instructions.

EIA viral particles were then lysed and submitted to SDS-PAGE gel system by using 10% Bis-Tris gels Novex NuPAGE in a 2D well lane and run in a MES buffer. After separation the gels were transferred to nitrocellulose membranes in 7 minutes by using the iBlot dry blotting system (Invitrogen) accordingly manufacturer instructions.

After blotting the membranes were blocked with a non-animal blocking buffer (Nap-Blocker, G-Biosciences) for two hours at room temperature. Membrane Strips were cut and incubated with horse sera at 1:50 or 1:100 dilution in blocking buffer for 1 hour at 37°C. Strips were washed three times with TBS 0.005% Tween20 and incubated, for 30 minutes at 37°C, with goat secondary anti-horse IgG (H+L) antibody labelled with Horseradish peroxidase in a 1:4000 dilution.

The secondary antibody was washed out three times, strips passed in a PBS solution and revealed with Ampliflu Red (Sigma-aldrich) fluorescent substrate and visualized with a 570 nm filter at in a Gel doc EZ (Biorad). Results were analysed by using the Image lab software (Biorad). Four different EIAV positive reference sera were used in the immunoblot: the World organization for animal health (OIE) EIAV positive reference sera kindly provided by the European Reference Center for Equine diseases (ANSES, France), and the EIA strong, medium and weak reference sera purchased to VMRD diagnostic (Pullman, Washington DC).

As a positive control seropositive EIAV AGID negative were also tested in EIAV_{PV} blotted membranes kindly provided by Doctor Charles Issel (University of Kentucky). The results obtained with the 'in house' EIAV_{WYO} blotted membranes were similar to the results obtained with UK EIAV_{PV} membranes. The EIAV_{PV} viral strain is a strain derived from EIAV_{WYO} (Lichtenstein *et al.,* 2006).

In Figure 1 the inventors compared the pattern of the OIE reference sera and the EIAV AGID negative samples. In this Figure the immunoblot pattern of EIAV AGID positive samples, such as the OIE reference sera, can be visualised. This pattern it is usually composed of a three-band pattern in the region of the p26, the gp45 and gp120 proteins that correspond to the binding of anti-p26, anti- gp45, and to the anti-gp120 specific antibodies. However, with the VMRD reference sera, a more broad reactivity against different EIAV proteins (not shown) can be visualized. In Figure 1 the pattern of EIAV AGID positive and negative reactors can be compared. The EIAV AGID negative, like the EIAV AGID positive reactors, possesses the three-band pattern characteristic of EIAV seropositiveness. However, these reactors are considered negative in the AGID assay and/or in the ELISA (Eradikit, ln3 Diagnostic, Italy).

### EIAV AGID and ELISA negative sera

The EIAV immunoblot (IB) positive sera were submitted to AGID testing by using an ID.Vet EIAV AGID kit (ID.Vet, France). The AGID was performed accordingly the manufacturer instructions. Moreover, 1:2 and 1:3 dilutions of the EIAV antigen (p26 recombinant protein) and positive control were performed in order to visualize weak reactivity against the p26 antigen in EIAV IB positive samples. As results none of the seropositives detected in the immunoblot were positive in AGID, neither with the non-diluted nor diluted reagents.

Moreover, EIAV AGID negative sera were analysed by EIAV commercially available ELISAs using antibodies raised against the p26 antigen, or against the dual antigen p26 and gp45, the Eradikit (IN3.Diagnostic, Italy). The sera were serial diluted from 1:6 to 1:20 dilution. The results showed that EIAV AGID negative sera were also considered as negative in all the dilutions tested in this ELISA.

### Establishment of NEV macrophage-iike cell lines from EIAV AGID negative horses.

For isolation and viral propagation of EIAV AGID negative strains, three different macrophage cell lines were established from EIAV AGID negative infected horses. Peripheral blood was collect from 3 different EIAV seropositive horses and processed according to Fidalgo-Carvalho et al., (2009).

The macrophage-like cells were able to divide, being passaged and remained in culture for 3-4 months. The macrophage-like cells obtained were similar to the EML-3C reported in Fidalgo-Carvalho et al., (2009), but during the first weeks of culture showed syncytia cell formation and cell death (Figure 2A1 and Figure 2A2). After 3 weeks the cells were able to recover (Figure 2B) and showed low levels of reverse transcriptase activity measured in cell supernatants by the Enzcheck reverse transcriptase assay (Invitrogen) (Figure 2.D1 and 2.D2).

Viral particles (vp) were isolated from RT positive cell culture supernatants from NEV macrophage-like cell lines A (NEV_MaA), and/or NEV_MaB and/or NEV_MaC. No evident signs of cell stress or death were visualized. The viral particles were isolated as described in the above section.

### Viral transfer

Further the inventors performed viral transfer among the NEV cells lines generated. Viral particles of MaA cells were used to infect NEV-MaB cells, or NEV-MaC cells and vice-versa. Surprisingly, the cell cultures of NEV-MaC infected with vpB, and NEV-MaA infected with vpC showed an increased reverse transcription activity (in cell culture supernatants) and marked cythopathic events after 7-10 days of infections (Figure 2C) and all cells died 15 days post-infection. The viral particles obtained from NEV-MaC-VpB and/or NEV-MaA-vpC infection were isolated, centrifuged, filtered in a 0.2µm and concentrated in Vivaspin6 100 kda as described above.

Moreover these viral particles were used for further analysis of the proteome by mass spectrometry. The purified viral particles were also used for viral genome analysis.

### Viral RNA extraction

Viral RNA was extracted from isolated and purified viral particles by using the Trizol LS method, or by using the Pure-link viral RNA/DNA mini-kit (Invitrogen) according to the manufacturer instructions (Figure 3A).

### EIAV AGID negative proteome

The purified viral particles were run in a 10% SDS-PAGE and different bands were digested and injected for mass spectrometry analysis. The results showed a wide diverse spectrum and several retroviral peptides were identified in the EIAV AGID negative viral particles. None of the peptides retrieved belonged to the known EIAV AGID positive strains (Figure 3B).

### Serial passages of NEV virus and gain of virulence

To propagate the undescribed virus, the inventors performed *in vitro* serial passages of NEV virus into MaC cell line as described above (viral transfer). MaC monolayers cells in T75cm² or T175cm² flasks were infected with 1 ml of NEV virus and incubated at 37°C, 5%CO2 in a humid chamber for 7 days in DMEM high glucose medium without phenol red (Gibco, Life Technologies), supplemented with 10% of fetal bovine serum, 2% glutamax (Gibco, Life Technologies) and 1% of Penicilin-Streptomicin (Gibco, Life Technologies). The supernatant medium of named passage 1 was serially passaged in MaC cells as described above for 10 times. After serial passage #10, virulence become more marked. In Figure 4 the inventors compared cell micrograph of day 7 non-infected cells (Fig 4A) versus NEV infected cells. The infected cells showed an increased number of induced syncytia cell formation and cell death. (Fig. 4B - MaC cells). These cytopathic effects begin to appear at day 4 post-infection (p.i), cell monolayers were partially destroyed at day 5-7 p.i. and completely destroyed at 10-12 days p.i.

### NEV is a highly cytopathic virus in Equine Dermal cells.

Viral propagation of the NEV virus was further tested in Equine Dermal Cells (ED) (NB-6, ATCC CCL-57) and its cytopathogenicity compared to EIAV_Wyoming.

EIAV-Wyoming (ATCC-VR-778) is reported as an *in vivo* pathogenic virus that can replicate in Equine Dermal cells with slight cytopathic effects (CPE) (Malmquist WA et al., 1973, Orego A, et al, 1982).

ED cells were seeded 24 to 72 hours prior to infection in T75cm² flasks. Highly confluent (80-90%) cells were infected with 1ml of 0.2 µm filtered NEV viral supernatants or 1 MOI of EIAV-Wyoming, and incubated at 37°C 5% CO2 for 5 or more days.

At day 5-7, cell death was evident with visible cell monolayer destruction. In ED cells the NEV induced CPE (Fig. 4B - ED cells) were very similar to those described above for MaC cells (Fig. 4B - MaC cells). However, the ED cells infected with EIAV- Wyoming failed to induce marked CPE and cell death at day 7 or day 12 (Fig 4C - ED cells).

Moreover, to quantify EIAV viral replication activity we developed a reverse transcriptase and qPCR assay by using the gene specific primers EIA (3595-3615) forward primer 5'-GGGGTGGTATTATTCGTGGCT-3' (SEQ ID NO: 161) and EIA (4060-4088) reverse primer 5'- TTTTTCTAATTTGTGTGCCTTGATATGCT 3' (SEQ ID NO: 162) directed to the pol region. The assay performed with Superscript III Reverse transcriptase (Life Technologies) and SsoFast™ EvaGreen® supermix (Bio-Rad) according to the manufacturer's instructions was run in a CFX96 Bio-Rad apparatus. The amplified 494 bp of the EIAV pol gene was previously cloned into Topo TA pCR4 vector to obtain the plasmid construct pCR4.EIAVpol. This plasmid was further used to construct a standard curve by performing 10-fold serial dilutions in duplicates. The retrieved standard curve had a R² of 0.998 and was able to detect 4 viral particles. According to the obtained standard curve, viral supernatant obtained from day 6 cell cultures of ED cells infected with EIAV-Wyoming (Fig.4C) contained 9.18x10⁻⁴ viral particles/ml. The results showed that EIAV Wyoming virus replicates at high levels in ED cells without significant CPE. Moreover, the RT-qPCR assay failed to amplify the NEV genome.

To better evaluate the pathogenicity of NEV in ED cells, Presto Blue cell viability assays (Molecular Probes, Life Technologies) were performed in cells infected with NEV or EIAV Wyoming (ATCC-VR-778). Presto blue cellular viability assays were performed in quintuplicates following the manufacturer's instructions. ED cells were seeded into 96-well plates 24-48 hours before infection. Highly confluent cell monolayers were infected with 20µl of NEV viral supernatant of day 7, or 1 MOI of EIAV Wyoming strain and allowed to incubate for 11 days. Presto blue reagent was added to the cultures, incubated for 16 hours and absorbance read at 570 nm in a Multiskan Go ELISA reader (Thermo Scientific).

The results showed that the viability of NEV infected cells was decreased to less than one third of the absorbance values of mock cells. The viability of NEV infected cells showed a statistically significant difference (Mann Whitney test) when compared to non-infected or EIAV-Wyoming infected cells (Fig.4D). Moreover, cell viability of EIAV Wyoming infected cells and mock cells showed similar absorbance (Fig.4D) confirming the microscopic data (Fig. 4C) and indicating that contrary to NEV, the EIAV-Wyoming doesn't induce evident CPE on ED cells at day 11.

### NEV virus possesses Aspartic protease activity

To assess for *in vitro* proteolytic cleavage activity, cell infections were performed in serum free media conditions. Day 5-7 p.i. viral particles were concentrated and extensively washed with HBSS in Vivaspin 100kDa (Sartorius) columns. Concentrated viral particles were lysed in 0.1% SDS and submitted to analytical size-exclusion chromatography on a Superdex 200 5/150 GL (GE Healthcare Life Sciences) column connected to a Prominence HPLC system (Shimadzu Corporation, Tokyo, Japan). The column was equilibrated in 20 mM phosphate buffer pH 7.5 containing 150 mM NaCl.

The protease activity was determined by fluorescence assays in 96-well plates in a Gemini EM Fluorescence Microplate Reader, using the HIV Protease Substrate 1 Arg-Glu(EDANS)-Ser-Gln-Asn-Tyr-Pro-Ile-Val-Gln-Lys(DABCYL)-Arg (SEQ ID NO: 160) (Sigma Aldrich) or the Phe-Phe substrate. For determination of the pH profile, viral particles were assayed for activity at 37°C in buffers ranging between pH 3 and 6 (50 mM sodium acetate pH 3.0, 4.0, 6.0; 50 mM Tris-HCl pH) containing 100 mM NaCl. To test the specificity of enzymatic cleavage, Indinavir (Sigma Aldrich), a specific inhibitors of HIV protease, or pepstatin were used. The protease was pre-incubated in the presence of the inhibitor. The Pesptatin or Indinavir (10 µM) was again incubated with viral particles for 10 minutes at room temperature in 50 mM sodium acetate pH 5 or pH 6.0 containing 100 mM NaC. The rate of substrate hydrolysis was monitored for 3 hours by the increase of intensity with excitation/emission wavelengths of 328/393 nm.

Our results demonstrated that fraction 12, 15 and 16 of NEV viral particles were able to cleave HIV substrate 1 (Fig 5C) at pH=5 and Phe-Phe substrate at pH=3 and 4 (Fig. 5A and B). Moreover, pepstatin or Indinavir were able to inhibit the protease activity of NEV fractions (Fig. 5A, B and C).

### Example 1

### Immunoenzyme Assay Using the Peroxidase-NEV Peptide Conjugates

The detection of antibodies directed against theNEV viruses is based, in the example below, on the principle of the immunoenzyme technique of the "double-antigen sandwich" type. The test is based on the use, on the one hand, of a microplate (solid phase) sensitized with purified antigens, and, on the other hand, of a conjugate constituted by a peptide according to the invention labelled with peroxidase, which take an NEV antibody to be detected into a sandwich.

The assay protocol used is as follows: 100 µl of each serum sample tested, diluted to [3/4], are distributed in a well of the sensitized microplate and the mixture is homogenized. After incubation of the mixture under adhesive film for 60 minutes at 37°C., followed by washing of the microplate by means of a Tris NaCl buffer, 100 µl of labelled conjugate (peroxidase-labelled NEV peptide according to the invention) are added to each well. The mixture is incubated under adhesive film for 30 minutes at 18-30°C.

After removal (by washing with the above-mentioned Tris NaCl buffer) of the conjugate fraction that has remained free, the presence of the immobilized enzyme on the complexes is revealed. To that end, 80 µl of a revelation solution (containing H₂O₂ substrate in a solution of citric acid and sodium acetate, and a chromogenic compound, tetramethylbenzidine or TMB) are added to each well. After incubation of the mixture again in darkness for 30 minutes at 18-30°C., the revelation is stopped by addition of 100 µl of 1N sulfuric acid. The optical density is read on a spectrophotometer at 450/620 nm.

The presence or absence of anti-NEV antibodies is determined by comparing, for each sample tested, the recorded optical density ("OD") with that of the calculated cut-off (cut-off=average of the OD of negative samples+0.100 OD unit).

A sample is considered to be positive (carrier of anti-NEV antibodies) if the OD obtained is greater than that of the cut-off, and negative if its OD is lower than that of the cut-off.

### Example 2

### Indirect Immunoassay for the NEV Detection of Specific Antibodies

Synthesis of the Peptides According to the Invention was carried out in accordance with Barani, G. and Merrifield, R. B. in The Peptides, Analysis, Synthesis and Biology, Vol. 2, Academic Press, Ed. Erhard Gross, Johannes Meyerhofer. The crude peptide was purified by HPLC.

### Example 2a

### Preparation of Peptide Solutions and Coating of Micro-Titration Plates with These Peptides

The peptides were dissolved in 50% (v/v) acetic acid, water, or DMSO, at a concentration of 5 mg/ml. The stock solutions were diluted in 0.10 M sodium bicarbonate (pH 9.6) such that the concentrations of the peptides are 1 µg/ml. 100 µl of the dilute solution were added to each of the wells of type B microtitration plates from Nunc, Denmark. The filled test plates were incubated at 20°C for 18 hours. The solutions were then sucked off and the wells were rinsed 3-4 times with 300 µl of a 10 g/l solution of bovine serum albumin in phosphate-buffered physiological sodium chloride solution (PBS, pH 7.4), and the test plates were then dried over silica gel at 20°C.

### Example 2b

### Preparation of a Peroxidase-Labelled Antibody Against Horse Immunoglobulin of the IgG Class (h-IgG), and also TMB Substrate for Detection

Monoclonal antibodies against h-IgG were prepared in accordance with the method of Koehler and Milstein, Nature 256: 495, 1975, with different monoclonal antibodies having the same antigen specificity being identified by the method described by Stahli et al., J. of Immunological Methods 32: 297-304 (1980). Following purification by gel chromatography and dialysis against PBS buffer, pH 7.4, the monoclonal antibody fraction (4 mg of protein/ml) was reacted with N-gammamaleimidobutyloxysuccinimide (GMBS) in accordance with Tanamori et al., J. Immunol. Meth. 62: 123-131 (1983). In parallel with this, 2-iminothiolane hydrochloride (from Sigma, Cat. No. 1 6256) was reacted with horseradish peroxidase (POD, from Boehringer Mannheim, Cat. No. 413470) in accordance with King et al. Biochem. 17: 1499-1506 (1978). An antibody/POD conjugate was prepared from the GMBS/antibody conjugate and the iminothiolane/POD conjugate as described by Tanamori et al., supra. The resulting solution of the IgG/POD conjugate had a protein content of 360 µl/ml. The ratio of POD to IgG was 2.8. The solution was subsequently diluted to 500 ng/ml IgG/POD using a solution of 50 ml/l fetal calf serum. (FCS, from Biochrom KG, Berlin) and 5 g/l polyoxyethylene (20) sorbitan monolaurate (Tween 20) in PBS, and was given the designation anti-IgG/POD conjugate. For use in the ELISA, the anti-IgG/POD conjugate was diluted 1:100 to 1:20,000 with Tris buffer (pH 7.4, containing 0.5% Tween 20), and then a series of 1:26 final dilutions in conjugate buffer (0.1 M1-amino-2-(hydroxymethyl)-1,3-propanediol (Tris), 0.1 M sodium chloride (NaCl) and 0.1% Tween 20, pH 8.4) is prepared. For detecting anti-human IgG/POD, the present inventors used a substrate system, or a substrate preparation, composed of hydrogen peroxide and tetramethylbenzidine (TMB), which was prepared from two stock solutions as follows:
Stock solution 1: TMB dihydrochloride was dissolved with stirring in double-distilled water at a concentration of 5 g/l (16 mmol/1), and this solution was adjusted to pH 1.5 using 5 N hydrochloric acid. Penicillin G was added to this solution with stirring, up to a final concentration of 200 mg/l (0.56 mmol/l).
Stock solution 2:1.4 ml of glacial acetic acid, 1.5 ml of 1 N NaOH and 250 mg (3 mmol) of H₂O₂, as a urea/hydrogen peroxide adduct, were added to 900 ml of double-distilled water. After these substances had dissolved completely, the solution was made up to 1 litre using double-distilled water.

TMB substrate preparation: One part by volume of stock solution 1 and 10 parts by volume of stock solution 2 were mixed together.

### Example 2c

### Determination of Horse Antibodies of the Immunoglobulin G Class Against peptides in an ELISA Using the Peptide According to the Invention

50 µl of serum or plasma were added to 50 µl of sample buffer, containing 0.3 M Tris, 0.3 M NaCl, 20% bovine serum and 0.1% Tween 20, in wells of coated microtiter plates which were prepared in accordance with the Example 2b. After the plates had been incubated at 37°C. for 30 minutes, the test solutions were aspirated and the wells were in each case washed five times with washing buffer containing 1 g/l Tween 20 in PBS. After that, 100 µl of conjugate were added to each of the wells, a preliminary dilution of 1:3000 in Tris buffer (pH 7.4, 0.5% Tween 20) and a final dilution of 1:26 in conjugate buffer preferably being selected. After the plates had been incubated at 37°C. for 30 minutes, the contents of the wells were aspirated and the wells were once again in each case washed five times. Subsequently, 100 µl of TMB substrate preparation were added to each well and the plates were incubated at 20-22°C for 30 minutes; the reaction was then stopped by adding 100 µl of 1 normal sulfuric acid. The extinction of the colored solution was measured at a wavelength of 450 nm (E450) against a blank value of PBS.

The reactivities of Western-blot anti-NEV negative and Western-blot anti-NEV positive samples are compared on microtitration plates which are coated, on the one hand, with the synthetic peptide.

### Example 3

### Immunoassay for Detection of Antibodies Induced by NEV.

The magnetic particle reagents are to be prepared according to the manufacturers recommended protocol. Dynal AS, is the manufacturer of the Dynabeads, which are employed. The magnetic particles coated with ligand are called Reagent 1. A peptide according to the invention is covalently coupled to the pre-activated surface of the magnetic particles. It is also possible to physically absorb the peptide to the surface of the magnetic particles. The concentration of particles in Reagent 1 is within the range from 1 mg/ml to 15 mg/ml. The particle size varies between 0.2 µm to 15 µm. The concentration of peptides is within the range from 1 ng/mg particle to 1 mg/mg particle. For example the concentration of peptides may be within the range from 1 ng/mg particle to 0.01 mg/mg particle, or preferably within the range from 0.01 mg/mg particle to 1 mg/mg particle. The anti horse Ig Alkaline Phosphatase (AP) conjugated antibody reagent is prepared according to the recommended protocol of Dako AS. This protocol is a standard procedure in this field. This reagent is called Reagent 2.

The substrate solution phenolphtaleine-monophosphate is to be prepared according to the recommended protocol of Fluka AG. This protocol is a standard procedure in this field. The substrate solution is called Reagent 3.

The washing and incubation buffer which is used is standard 0.05M tris-base buffer with the following additional compounds; Tween 20 (0.01% to 0.1%), glycerol (0.1% to 10%) and sodium chloride (0.2% to 0.1%).

The assay procedure comprises an incubation step wherein 1 drop of Reagent 1 is mixed with 2 drops of washing buffer in each well. After mixing, 30 µl of sample is added and the solution is incubated for 5 minutes. The magnetic particles can be trapped by a magnet and the liquid removed, before the magnet is separated. Then the wells are washed twice in 4 drops of washing solution, before incubation with Reagent 2. 1 drop of Reagent 2 is added with 2 drops of washing buffer and the solution is incubated for 5 minutes. The magnetic particles can be trapped by a magnet and the liquid removed, before the magnet is separated. Then the washing step is repeated before incubation with Reagent 3. Two drops of Reagent 3 are added to each well and the solution is incubated for 3 minutes. The results can be read against a white background. Positive results are red (3+=strong red) whereas negative results are clearly light yellow/brown solutions as obtained in the negative control. The immunoassay kit could be used in detection of antibodies, induced either by NEV virus or NEV-specific peptides or proteins, for instance the peptides of the present invention.

### Example 4

### Immunomodulator (e.g. therapeutic or prophylactic vaccine).

At least one of the peptides of the invention, selected from the group of sequences SEQ ID NO: 1 to SEQ ID NO: 76 can form antigens and constitute the active principle of an immunomodulator e.g. a prophylactic or therapeutic vaccine intended to provide protection against the equine retrovirus (EIAV) or NEV. The immunomodulator may include compounds having beneficial effects in protecting or stimulating the hosts immune system for instance interleukins, interferons, granulocyte macrophage growth factors, haematopoietic growth factors or similar. In one embodiment the immunomodulator further contains an adjuvant or vehicle; for example, the adjuvant or vehicle is Monophosphoryl Lipid A (MPL(R)) possibly with alum, Freund's adjuvant (complete or incomplete) or aluminium hydroxyd. The optimal amount of adjuvant/vehicle will depend on the type(s) which is chosen.

The peptides of the present invention may be modified by C-terminal addition of a single fatty acid such as a single palmitoyl chain to form a lipopeptide vaccine. Further the lipopeptides can be introduced into liposome membranes by the freeze-thaw method resulting in liposomes bearing the peptide ligands on their surface.

The peptides of the present invention or compositions of the present invention or pharmaceutical compositions of the present invention or immunomodulator formulation can be freeze-dried prior to storage. The freeze-dried peptides can be dissolved in sterile water to a final concentration of 0.1-100 mg/ml. The peptides of the present invention or compositions of the present invention or pharmaceutical compositions of the present invention or the immunomodulator may be stored, for example, at low temperature, in ampoules containing one or more dosage units, ready for use.

A typical dosage unit of a peptide according to the invention is within the concentration range: 0.05 µg-1 mg per kg bodyweight, preferably within 0, 15 µg-0.15 mg per kg body weight. Persons skilled in the art will appreciate that a suitable dose will depend on the body weight of the animal, the type of disease, severity of condition, administration route and several other factors.

The peptides of the present invention or compositions of the present invention or pharmaceutical compositions of the present invention may be used as a therapeutic vaccine.

When used as a therapeutic vaccine, the vaccine might be administered up to 12 times, through injections. Further boosters might follow and can in some cases take place throughout the animal life. In preparation of an injection solution the peptides are typically dissolved in sterile water at a final concentration of 1 mg/ml per peptide. Typically an injection volume is 100 µl to 200 µl (2x100 µl). The peptide is preferably co-administered with a suitable adjuvant and/or a granulocyte-macrophage growth factor for instance Leucomax(R) (Schering Plough) made within a concentration range of from 0.1 mg/ml to 1 mg/ml, or according to the manufacturers recommendations for use.

These peptides may be administered simultaneously or sequentially. Suitable administration may be intracutaneous, subcutaneous, intravenous, peroral, intramuscular, intranasal, mucosal or any other suitable route. Booster administrations may be required in order to maintain protection. For persons skilled in the art it will be understood that the immunomodulators according to the invention may be useful not only in prevention of infection, but also in treatment of infection.

### Example 5

An immunomodulator comprising one or more peptides selected from the group consisting of SEQ ID NO: 1 to and 76 are prepared. The freeze-dried peptides are dissolved in sterile water at a final concentration of 4 mg/ml. A preparation of a granulocyte-macrophage-colony stimulating factor (GM-CSF) is also prepared, according to the manufacturers directions for use, to a final concentration of 0.3 mg/ml. The solutions are administered intracutaneously or intramuscularly or intradermal. A typical injection dose is 100 µl.

### Example 6

An antigen solution or suspension is mixed with equal parts of Freund's adjuvant of Behring, complete or incomplete, and is then finely emulsified by being drawn up into, and vigorously pressed out of, an injection syringe, or with a homogenator. The emulsion should remain stable for at least 30 minutes. The antigen-adjuvant emulsions is best injected subcutaneously, or intramuscularly or intradermal as a depot.

### Example 7

Development of a fast and sensitive peptide based c-ELISA to identify NEV infected animals A highly sensitive peptide based competitive enzyme-linked immunosorbent (c-ELISA) procedure was developed to detect sera antibodies against NEV.

Mouse monoclonal antibodies (mab) were produced against SEQ ID NO: 18 or SEQ ID NO: 20.

A monoclonal antibody mab#4G6E4 was directed against SEQ ID NO: 20.. Furthermore this mab was tested against SEQ ID NO: 20. or the fusion peptide SEQ ID NO: 159. by ELISA procedures.

For that, serial dilutions of peptides in duplicates (1:1.3 or 1:1.4 in 0.05M carbonate buffer pH=9.3) ranging between 5 to 500 ng/mL were covalently linked to 96 microwell Poylsorp amino plates (Nunc, Thermofisher) for two hours at room temperature. To remove unbound peptide, plates were washed three times with PBS-Tween20 (0.005%) in an automated Wellwasher apparatus (Thermoscientific). After washing, plates were blocked by adding 300 microliters/well of Superblock T20 in PBS (Thermoscientific, Pierce). Superblock T20 was immediately removed and plates inverted till drying out. After blocked and dried plates were ready for c-ELISA or to be stored at 4-8°C for further testing.

To compare the affinity of mab#4G6E4 for the peptide SEQ ID NO: 20 or for the fusion peptide SEQ ID NO: 159 mab#4G6E4 was diluted in the antibody dilution buffer, 3% BSA blocker (Thermoscientific).

A rapid (2 hours) c-ELISA procedure was performed as follows: 70 ng/mL of mab#4G6E4 alone (50 microliters) diluted in dilution buffer was added to peptide-coated wells and incubated for 1hour at 37°C. After 1 hour of incubation plates were extensively washed with 300 microliters of PBS-Tween20 (0.05%) for three times. The plate was after that incubated with a secondary goat anti-mouse IgG- HRP (Sigma Aldrich) at 1:2500 dilution and incubated for 30 minutes at 37°C. Plates were washed again four times with 300 microliters of PBS-Tween20 (0.05%) and signal developed by adding 50 microliters of fresh TMB reagent (Sigma Aldrich) for 15 minutes at room temperature. After colour development the reaction was stopped by adding 50 microliters of 1N H₂SO₄ (Sigma Aldrich). Determination of absorbance was obtained by reading plates at 450 nm in a Multiskan Go ELISA reader (Thermoscientific).

### Dose responses curves and inhibition rate

Dose responses curves were analysed by GraphPad Prism 6.0 software by using the four parameter function log(agonist) vs response with a variable slope. In these conditions the EC₅₀ of peptide SEQ ID NO: 159 was 163.5 ng/mL and 193.7 ng/mL for the SEQ ID NO: 20 peptide. The Hill slope was very high for both peptides 7.2 for SEQ ID NO: 159 and 6.6 for SEQ ID NO: 20. The results are indicating high affinity between the mab and the peptide sequences (Figure 6A1).

Furthermore dose response curves were optimized in order to decrease the Hill Slope and turn the assay more sensitive. For that the dilution buffer was replaced by 0.025% of casein blocking (instead of 3%BSA). The results showed (Figure 6A2) that in these conditions the mab#4G6E4 (70ng/mL) is highly specific and able to bind less than 25 ng/mL of the fusion peptide. In these conditions the EC50 was decreased to 59 ng/mL and Hill Slope to 2.52, the R2 was 0.9997.

In Figure 6A1 we show dose response curves for SEQ ID NO: 20 and SEQ ID NO: 159 and mab#4G6E4 in 3% BSA. In Figure 6A2 we show dose responses curves for mab#4G6E4 diluted in 0.025% of casein.

### Competitive ELISA

To develop a competitive ELISA the mab#4G6E4 was incubated in the presence of a NEV negative control sera or positive control sera and incubated as previously described in wells coated with serial dilutions of the fusion peptide set forth as SEQ ID NO: 159. For that, 25 microliters of mab#4G6E4 and 25 microliters of diluted equine sera were added in coated wells and incubated together for 1hour at 37°C. Serum or mab#4G6E4 were diluted in 0.025% Casein blocking buffer in PBS (Thermoscientific, Pierce).

Data were analysed by using GraphPad PRISM6.0 version software. Different dose response curves were generated by the nonlinear regression, log(agonist)vs response with a variable slope - four parameter function.

The inhibition of mAb#4G6E4 binding was calculated per each peptide dilution as a percent inhibition of the mAb with reference negative serum, using the following formula: % inhibition (%I) = 100 - [(test serum ODx100)/(mean negative control OD)]. The test serum was considered to be antibody-positive if the %I was ≥ 30%.

Moreover in the presence of seropositive equine sera (diluted 1:10 or 1:100) mab#4G6E4 binding to SEQ ID NO: 159 was evident with decreased signal due to inhibition/competition per the peptide. The same effect was not shown in the presence of seronegative sera. The EC₅₀, Hill slope, top and bottom values of the dose curve response were calculated for different dose curve responses in the presence of seropositive or seronegative sera. Different dose responses curves were analysed and compared with each other using the extra sum of squares F test. Results showed EC₅₀, Hill slope, bottom and top values of mixtures of mab#4G6E4 and seronegative sera were statistically different (p<0.0001) from the EC₅₀, Hill slope, bottom and top values of mixtures of mab#4G6E4 and seropositive sera. The R² retrieved for each dose response curve were always higher than 0.980.

In Figure 6B we present dose responses curve and inhibition rates of NEV seronegative and seropositives samples.

In Figure 6C we show representative inhibition rates (%I) of NEV seropositive and one seronegative. Inhibition rates were obtained for a SEQ ID NO: 159 peptide concentration of 96 ng/mL of peptide and mab#4G6E4 IgG concentration of 70ng/mL.

### SEQUENCES

Below are the polypeptide and polynucleotide sequences referred to in the present application:
SRLLESRGPTTSEK (SEQ ID NO: 1);
TLKEVLGGEAAVR (SEQ ID NO: 2);
PCRSKNILPV (SEQ ID NO: 3);
DPCVHSVDVLLR (SEQ ID NO: 4);
STFPPTPV (SEQ ID NO: 5);
NAPLLSKVSP (SEQ ID NO: 6);
MAQCIVNR (SEQ ID NO: 7);
KPNVEGRYGLSRSETNK (SEQ ID NO: 8);
QQGPEAPLPSLQVAEVPK (SEQ ID NO: 9);
PHAGSTQTEWPK (SEQ ID NO: 10);
PIQINACK (SEQ ID NO: 11);
GGMRESWDGGQV (SEQ ID NO: 12);
ESLVSKGFR (SEQ ID NO: 13);
CSLVLGEMQIKRIR (SEQ ID NO: 14);
LDKWEKIR (SEQ ID NO 15);
QMMIAASEK (SEQ ID NO 16);
QPSLPTGSEELK (SEQ ID NO 17);
EALDKIEEIQNKNKQK (SEQ ID NO 18);
PPIPVGGIYK (SEQ ID NO 19);
QEQNPPPSVSLRSLFGNDPL (SEQ ID NO 20);
EKIEQLR (SEQ ID NO 21);
DLLLIVAR (SEQ ID NO 22);
IVELLGR (SEQ ID NO 23);
IWGNVTWMEWER (SEQ ID NO 24);
LKDLFPNK (SEQ ID NO 25);
AKLEESFPGK (SEQ ID NO 26);
NGSLAGESIIIR (SEQ ID NO 27);
NITFNSSAGGDLEIT (SEQ ID NO 28);
AVILLLDRLR (SEQ ID NO 29);
GPGIHIGKR (SEQ ID NO 30);
VIICSASK (SEQ ID NO 31);
ESFPNK (SEQ ID NO 32);
FGNKTTIIFTK (SEQ ID NO 33);
LLNGSLAGESIIIR (SEQ ID NO 34);
VNVSVTNNNTTTNV (SEQ ID NO 35);
AILHILRR (SEQ ID NO 36);
DLLALDK (SEQ ID NO 37);
IGCQHSRIGITLPR (SEQ ID NO 38);
TLQYLALTALVTPK (SEQ ID NO 39);
PTTPVTPAPGVGEISKELAQGK (SEQ ID NO 40);
VVSSALQYLIPR (SEQ ID NO 41);
PVWAEPVK (SEQ ID NO 42);
VKGNDLLK (SEQ ID NO 43);
EDLNLQDWK (SEQ ID NO 44);
LFVSVLQR (SEQ ID NO 45);
AAEQKASPPSLTPK (SEQ ID NO 46);
PLSLPLKI (SEQ ID NO 47);
FPSIVGRPR (SEQ ID NO 48);
IWHHTFYNELR (SEQ ID NO 49);
MTQIMFETF (SEQ ID NO 50);
EEEVAALVIDNGSGMCK (SEQ ID NO 51);
VAPEEHPVLLTEAPLNPK (SEQ ID NO 52);
AVFPSIVGRPR (SEQ ID NO 53);
YPIEHGIVTNWDDMEK (SEQ ID NO 54);
AVFPSIVGR (SEQ ID NO 55);
IGRKDAERQLLSPGNAR (SEQ ID NO 56);
DSYVGDEAQSKR (SEQ ID NO 57);
RGILTLK (SEQ ID NO 58);
WGIAHTTGIPGNSQGQAMVER (SEQ ID NO 59);
HLVDQLIRDLK (SEQ ID NO 60);
YEQLQLQAR (SEQ ID NO 61);
TITLEVEPSDTIENVK (SEQ ID NO 62);
INRELLK (SEQ ID NO 63);
QTIIPTNKDVDEK (SEQ ID NO 64);
KNYGKLDK (SEQ ID NO 65);
TTISFSK (SEQ ID NO 66);
QDRTTISFSK (SEQ ID NO 67);
AQGRAIAHK (SEQ ID NO 68);
MAQTQLVPVK (SEQ ID NO 69);
QELIPPCK (SEQ ID NO 70);
DIVLLENGK (SEQ ID NO 71);
LPPLSILK (SEQ ID NO 72);
IFLINLAFLIK (SEQ ID NO 73);
NRLEILK (SEQ ID NO 74);
ADDVAVLQDALGR (SEQ ID NO 75);
LNKSLEQLR (SEQ ID NO 76);
AGCAGGCTGCTGGAGAGCAGGGGCCCCACCACCAGCGAGAAG (SEQ ID NO 77);
ACCCTGAAAGAGGTTCTTGGTGGTGAAGCAGCCGTGCGC (SEQ ID NO 78);
CCCTGCCGGAGCAAGAACATCCTGCCCGTG (SEQ ID NO 79);
GACCCCTGCGTGCACAGCGTGGACGTGCTGCTGAGG (SEQ ID NO 80);
TCCACATTTCCTCCCACTCCCGTC (SEQ ID NO 81);
AATGCCCCATTACTCTCAAAAGTGTCCCCA (SEQ ID NO 82);
ATGGCACAGTGTATCGTTAACCGC (SEQ ID NO 83);
AAGCCCAACGTGGAGGGCAGGTACGGCCTGAGCAGGAGCGAGACCAACAAG (SEQ ID NO 84); 
[?]);
CAGCAAGGCCCAGAGGCGCCACTGCCCAGCCTGCAGGTTGCAGAAGTGCCTA
AA (SEQ ID NO 85);
CCCCATGCGGGCTCCACTCAGACAGAGTGGCCCAAA (SEQ ID NO 86);
CCAATACAGATCAATGCTTGCAAA (SEQ ID NO 87);
GGTGGAATGCGTGAGTCATGGGATGGAGGACAAGTT (SEQ ID NO 88);
GAATCTCTGGTGTCAAAAGGGTTTCGG (SEQ ID NO 89);
TGCTCATTAGTCCTTGGGGAGATGCAAATCAAA (SEQ ID NO 90);
CTGGACAAGTGGGAGAAGATCAGG (SEQ ID NO 91);
CAGATGATGATCGCCGCCAGCGAGAAG (SEQ ID NO 92);
CAGCCCAGCCTGCCCACCGGCAGCGAGGAGCTGAAG (SEQ ID NO 93);
CCCCCCATCCCCGTGGGCGGCATCTACAAG (SEQ ID NO 94); 
CAGGAGCAGAACCCCCCCCCAGCGTGAGCCTGAGGAGCCTGTTCGGCAACG
ACCCCCTG (SEQ ID NO 95);
GAGAAGATCGAGCAGCTGAGG (SEQ ID NO 96);
GACCTGCTGCTGATCGTGGCCAGG (SEQ ID NO 97);
ATCGTGGAGCTGCTGGGCAGG (SEQ ID NO 98);
ATCTGGGGCAACGTGACCTGGATGGAGTGGGAGAGG (SEQ ID NO 99);
CTGAAGGACCTGTTCCCCAACAAG (SEQ ID NO 100);
GCCAAGCTGGAGGAGAGCTTCCCCGGCAAG (SEQ ID NO 101);
AACGGCAGCCTGGCCGGCGAGAGCATCATCATCAGG (SEQ ID NO 102);
AACATCACCTTCAACAGCAGCGCCGGCGGCGACCTGGAGATCACC (SEQ ID NO 103);
GCCGTGATCCTGCTGCTGGACAGGCTGAGG (SEQ ID NO 104);
GGCCCCGGCATCCACATCGGCAAGAGG (SEQ ID NO 105);
GTGATCATCTGCAGCGCCAGCAAG (SEQ ID NO 106);
GAGAGCTTCCCCAACAAG (SEQ ID NO 107);
TTCGGCAACAAGACCACCATCATCTTCACCAAG (SEQ ID NO 108);
CTGCTGAACGGCAGCCTGGCCGGCGAGAGCATCATCATCAGG (SEQ ID NO 109);
GTGAACGTGAGCGTGACCAACAACAACACCACCACCAACGTG (SEQ ID NO 110);
GCCATCCTGCACATCCTGAGGAGG (SEQ ID NO 111);
GACCTGCTGGCCCTGGACAAG (SEQ ID NO 112);
ATCGGCTGCCAGCACAGCAGGATCGGCATCACCCTGCCCAGG (SEQ ID NO 113);
ACCCTGCAGTACCTGGCCCTGACCGCCCTGGTGACCCCCAAG (SEQ ID NO 114); 
CCCACCACCCCCGTGACCCCCGCCCCCGGCGTGGGCGAGATCAGCAAGGAGC
TGGCCCAGGGCAAG (SEQ ID NO 115);
GTGGTGAGCAGCGCCCTGCAGTACCTGATCCCCAGG (SEQ ID NO 116);
CCCGTGTGGGCCGAGCCCGTGAAG (SEQ ID NO 117);
GTGAAGGGCAACGACCTGCTGAAG (SEQ ID NO 118);
GAGGACCTGAACCTGCAGGACTGGAAG (SEQ ID NO 119);
CTGTTCGTGAGCGTGCTGCAGAGG (SEQ ID NO 120);
GCCGCCGAGCAGAAGGCCAGCCCCCCCAGCCTGACCCCCAAG (SEQ ID NO 121);
CCCCTGAGCCTGCCCCTGAAGATC (SEQ ID NO 122);
TTCCCCAGCATCGTGGGCAGGCCCAGG (SEQ ID NO 123);
ATCTGGCACCACACCTTCTACAACGAGCTGAGG (SEQ ID NO 124);
ATGACCCAGATCATGTTCGAGACCTTC (SEQ ID NO 125);
GAGGAGGAGGTGGCCGCCCTGGTGATCGACAACGGCAGCGGCATGTGCAAG (SEQ ID NO 126);
GTGGCCCCCGAGGAGCACCCCGTGCTGCTGACCGAGGCCCCCCTGAACCCCA AG (SEQ ID NO 127);
GCCGTGTTCCCCAGCATCGTGGGCAGGCCCAGG (SEQ ID NO 128);
TACCCCATCGAGCACGGCATCGTGACCAACTGGGACGACATGGAGAAG (SEQ ID NO 129);
GCCGTGTTCCCCAGCATCGTGGGCAGG (SEQ ID NO 130);
ATCGGCAGGAAGGACGCCGAGAGGCAGCTGCTGAGCCCCGGCAACGCCAGG (SEQ ID NO 131);
GACAGCTACGTGGGCGACGAGGCCCAGAGCAAGAGG (SEQ ID NO 132);
AGGGGCATCCTGACCCTGAAG (SEQ ID NO 133); 
TGGGGCATCGCCCACACCACCGGCATCCCCGGCAACAGCCAGGGCCAGGCCA
TGGTGGAGAGG (SEQ ID NO 134);
CACCTGGTGGACCAGCTGATCAGGGACCTGAAG (SEQ ID NO 135);
TACGAGCAGCTGCAGCTGCAGGCCAGG (SEQ ID NO 136);
ACCATCACCCTGGAGGTGGAGCCCAGCGACACCATCGAGAACGTGAAG (SEQ ID NO 137);
ATCAACAGGGAGCTGCTGAAG (SEQ ID NO 138);
CAGACCATCATCCCCACCAACAAGGACGTGGACGAGAAG (SEQ ID NO 139);
AAGAACTACGGCAAGCTGGACAAG (SEQ ID NO 140);
ACCACCATCAGCTTCAGCAAG (SEQ ID NO 141);
CAGGACAGGACCACCATCAGCTTCAGCAAG (SEQ ID NO 142);
GCCCAGGGCAGGGCCATCGCCCACAAG (SEQ ID NO 143);
ATGGCCCAGACCCAGCTGGTGCCCGTGAAG (SEQ ID NO 144);
CAGGAGCTGATCCCCCCCTGCAAG (SEQ ID NO 145);
GACATCGTGCTGCTGGAGAACGGCAAG (SEQ ID NO 146);
CTGCCCCCCCTGAGCATCCTGAAG (SEQ ID NO 147);
ATCTTCCTGATCAACCTGGCCTTCCTGATCAAG (SEQ ID NO 148);
AACAGGCTGGAGATCCTGAAG (SEQ ID NO 149);
GCCGACGACGTGGCCGTGCTGCAGGACGCCCTGGGCAGG (SEQ ID NO 150);
CTGAACAAGAGCCTGGAGCAGCTGAGG (SEQ ID NO 151);
GAGGCCCTGGACAAGATCGAGGAGATCCAGAACAAGAACAAGCAGAAG (SEQ ID NO 152);
QEQNPPPSVSLRSLFG (SEQ ID No 153);
CAGGAGCAGAACCCCCCCCCCAGCGTGAGCCTGAGGAGCCTGTTCGGC (SEQ ID No 154);
WGIAHTTGIPGNSQGQAM (SEQ ID No 155); 
TGGGGCATCGCCCACACCACCGGCATCCCCGGCAACAGCCAGGGCCAGGCCA
TG (SEQ ID No 156);
EALDKIEEIQNKNKQKAAAQEQNPPPSVSLRSLFGNDPL (SEQ ID NO: 157);
QEQNPPPSVSLRSLFGNDPLAAAEALDKIEEIQNKNKQK (SEQ ID NO: 158);
EALDKIEEIQNKNKQKAAAQEQNPPPSVSLRSLFG (SEQ ID NO: 159).

### REFERENCES

Lignee, M., 1843, Memoire et observation sur une maladie du sang, connue sous le nom d'anhemie hydrohemie cachexie aqueuse du cheval. Rec. Med. Vet. 20, 30.

Spyrou V, Papanastassopoulou M, Koumbati M, Nikolakaki SV, Koptopoulos G. Molecular analysis of the proviral DNA of equine infectious anemia virus in mules in Greece. Virus Res. 2005 Jan;107(1):63-72. PubMed PMID: 15567035.

Mooney J, Flynn O, Sammin D. Equine infectious anaemia in Ireland: characterisation of the virus. Vet Rec. 2006 Oct 21;159(17):570. PubMed PMID: 17056658.

Quinlivan M, Cook F, Kenna R, Callinan JJ, Cullinane A. Genetic characterization by composite sequence analysis of a new pathogenic field strain of equine infectious anemia virus from the 2006 outbreak in Ireland. J Gen Virol. 2013 Mar;94(Pt 3):612-22. doi: 10.1099/vir.0.047191-0. Epub 2012 Nov 21. PubMed PMID: 23175240.

Cappelli K, Capomaccio S, Cook FR, Felicetti M, Marenzoni ML, Coppola G, Verini-Supplizi A, Coletti M, Passamonti F. Molecular detection, epidemiology, and genetic characterization of novel European field isolates of equine infectious anemia virus. J Clin Microbiol. 2011 Jan;49(1):27-33. doi: 10.1128/JCM.01311-10. Epub 2010 Nov 17. PubMed PMID: 21084503; PubMed Central PMCID: PMC3020406.

Quinlivan M, Cook RF, Cullinane A. Real-time quantitative RT-PCR and PCR assays for a novel European field isolate of equine infectious anaemia virus based on sequence determination of the gag gene. Vet Rec. 2007 May 5;160(18):611-8. PubMed PMID: 17483378.

Cappelli K, Capomaccio S, Cook FR, Felicetti M, Marenzoni ML, Coppola G, Verini-Supplizi A, Coletti M, Passamonti F. Molecular detection, epidemiology, and genetic characterization of novel European field isolates of equine infectious anemia virus. J Clin Microbiol. 2011 Jan;49(1):27-33. doi: 10.1128/JCM.01311-10. Epub 2010 Nov 17. PubMed PMID: 21084503; PubMed Central PMCID: PMC3020406.

Capomaccio S, Cappelli K, Cook RF, Nardi F, Gifford R, Marenzoni ML, Passamonti F. Geographic structuring of global EIAV isolates: a single origin for New World strains? Virus Res. 2012 Feb;163(2):656-9. doi:10.1016/j.virusres.2011.11.011. Epub 2011 Nov 22. PubMed PMID: 22119901.

Kuhar U, Završ̌̌nik J, Toplak I, Malovrh T. Detection and molecular characterisation of equine infectious anaemia virus from field outbreaks in Slovenia. Equine Vet J. 2014 May;46(3):386-91. doi: 10.1111/evj.12138. Epub 2013 Sep 9. PubMed PMID: 23834226.

Scicluna MT, Issel CJ, Cook FR, Manna G, Cersini A, Rosone F, Frontoso R, Caprioli A, Antonetti V, Autorino GL. Is a diagnostic system based exclusively on agar gel immunodiffusion adequate for controlling the spread of equine infectious anaemia? Vet Microbiol. 2013 Jul 26;165(1-2):123-34. doi:10.1016/j.vetmic.2013.02.027. Epub 2013 Mar 28. PubMed PMID: 23618837.

Quinlivan M, Cook F, Kenna R, Callinan JJ, Cullinane A. Genetic characterization by composite sequence analysis of a new pathogenic field strain of equine infectious anemia virus from the 2006 outbreak in Ireland. J Gen Virol. 2013 Mar;94(Pt 3):612-22. doi: 10.1099/vir.0.047191-0. Epub 2012 Nov 21. PubMed PMID: 23175240.

Issel CJ, Scicluna MT, Cook SJ, Cook RF, Caprioli A, Ricci I, Rosone F, Craigo JK, Montelaro RC, Autorino GL. Challenges and proposed solutions for more accurate serological diagnosis of equine infectious anaemia. Vet Rec. 2013 Feb 23;172(8):210. doi: 10.1136/vr-2012-100735. Epub 2012 Nov 16. PubMed PMID: 23161812; PubMed Central PMCID: PMC3593188.

Capomaccio S, Willand ZA, Cook SJ, Issel CJ, Santos EM, Reis JK, Cook RF. Detection, molecular characterization and phylogenetic analysis of full-length equine infectious anemia (EIAV) gag genes isolated from Shackleford Banks wild horses. Vet Microbiol. 2012 Jun 15;157(3-4):320-32. doi: 10.1016/j.vetmic.2012.01.015. Epub 2012 Jan 18. PubMed PMID: 22310073.

Murakami K, Konishi M, Kameyama K, Shibahara T. Detection of equine infectious anaemia virus in native Japanese ponies. Vet Rec. 2012 Jul 21;171(3):72. doi: 10.1136/vr.100459. Epub 2012 Jul 10. PubMed PMID: 22781341.

Craigo JK, Barnes S, Zhang B, Cook SJ, Howe L, Issel CJ, Montelaro RC. An EIAV field isolate reveals much higher levels of subtype variability than currently reported for the equine lentivirus family. Retrovirology. 2009 Oct 20;6:95. doi: 10.1186/1742-4690-6-95. PubMed PMID: 19843328; PubMed Central PMCID: PMC2770520.

Boldbaatar B, Bazartseren T, Koba R, Murakami H, Oguma K, Murakami K, Sentsui H. Amplification of complete gag gene sequences from geographically distinct equine infectious anemia virus isolates. J Virol Methods. 2013 Apr;189(1):41-6. doi: 10.1016/j.jviromet.2012.12.010. Epub 2013 Jan 11. PubMed PMID: 23318370.

Dong JB, Zhu W, Cook FR, Goto Y, Horii Y, Haga T. Identification of a novel equine infectious anemia virus field strain isolated from feral horses in southern Japan. J Gen Virol. 2013 Feb;94(Pt 2):360-5. doi: 10.1099/vir.0.047498-0. Epub 2012 Oct 24. PubMed PMID: 23100364.

Murakami K, Konishi M, Kameyama K, Shibahara T. Detection of equine infectious anaemia virus in native Japanese ponies. Vet Rec. 2012 Jul 21;171(3):72. doi: 10.1136/vr.100459. Epub 2012 Jul 10. PubMed PMID: 22781341.

Pagamjav O, Kobayashi K, Murakami H, Tabata Y, Miura Y, Boldbaatar B, Sentsui H. Serological survey of equine viral diseases in Mongolia. Microbiol Immunol. 2011 Apr;55(4):289-92. doi: 10.1111/j.1348-0421.2011.00312.x. PubMed PMID: 21447048.

Sentsui H, Inoshima Y, Murakami K, Akashi H, Purevtseren B, Pagmajav O, Sugiura T. Cross reaction of recombinant equine infectious anemia virus antigen to heterologous strains and application for serological survey among horses in the field. Microbiol Immunol. 2001;45(1):45-50. PubMed PMID: 11270606.

Clabough DL, Gebhard D, Flaherty MT, Whetter LE, Perry ST, Coggins L, Fuller FJ. Immune-mediated thrombocytopenia in horses infected with equine infectious anemia virus. J Virol. 1991 Nov;65(11):6242-51. PubMed PMID: 1717720; PubMed Central PMCID: PMC250322.

Crawford TB, Wardrop KJ, Tornquist SJ, Reilich E, Meyers KM, McGuire TC. A primary production deficit in the thrombocytopenia of equine infectious anemia. J Virol. 1996 Nov;70(11):7842-50. PubMed PMID: 8892906; PubMed Central PMCID: PMC190855.

Oaks JL, Long MT, Baszler TV. Leukoencephalitis associated with selective viral replication in the brain of a pony with experimental chronic equine infectious anemia virus infection. Vet Pathol. 2004 Sep;41(5):527-32. PubMed PMID: 15347829.

## Claims

1. A composition comprising one or more peptides selected from the group consisting of SEQ ID NOs: 18 and 20 and variants thereof having at least 80% identity to SEQ ID NO: 20; and/or comprising one or more polynucleotide sequences selected from the group consisting of SEQ ID NOs: 95 and 152, and variants thereof having at least 80% identity to SEQ ID NO: 95.

2. The composition according to claim 1, wherein said one or more peptides selected from the group consisting of SEQ ID NOs: 18 and 20 and said variants thereof are linked together as a fusion protein, preferably wherein any two peptides selected from the group consisting of SEQ ID NO: 18 and SEQ ID NO: 20, and said variants thereof are linked together as a fusion protein.

3. The composition according to claim 2, further wherein said peptides are linked together as a fusion protein by a linker peptide, preferably wherein said linker peptide is between 2 and 10 amino acid residues in length, more preferably wherein said linker peptide is 3 amino acids in length.

4. The composition according to any one of claims 2 to 3, wherein said fusion protein comprises the sequence QEQNPPPSVSLRSLFGNDPLAAAEALDKIEEIQNKNKQK (SEQ ID NO: 158) or EALDKIEEIQNKNKQKAAAQEQNPPPSVSLRSLFG (SEQ ID NO: 159).

5. A polynucleotide sequence encoding the fusion protein of any one of claims 2 to 4.

6. A vector that encodes one or more peptides as defined in any one of claims 1 to 4, or a vector comprising one or more polynucleotide sequences as defined in claim 1 or claim 5.

7. An antibody that binds to SEQ ID NO: 20.

8. A pharmaceutical composition comprising:
one or more peptides as defined in any one of claims 1 to 4; or
one or more polynucleotide sequences as defined in claim 1 or claim 5; or
one or more vectors according to claim 6; or
one or more antibodies according to claim 7;
and a pharmaceutically acceptable carrier, vehicle, diluent or excipient.

9. A kit comprising:
a composition according to any one of claims 1 to 4; and/or
a vector according to claim 6; and/or
an antibody according to claim 7; and/or
a pharmaceutical composition according to claim 8;
and optionally instructions for administration to an animal.

10. A diagnostic method comprising providing a sample obtained from an animal and determining the presence or absence in said sample of antibodies that bind to one or more peptides selected from the group consisting of SEQ ID NOs: 18 and 20;
and/or determining the presence or absence in said sample of one or more of said peptides; and/or determining the presence or absence in said sample of one or more polynucleotide sequences as defined in claim 1 or claim 5, preferably wherein: said method determines the presence or absence of an EIAV and/or NEV in said animal, and/or the diagnostic method determines the viral titre of an EIAV and/or NEV in said animal; wherein NEV is deposited under ECAAC accession number 14120201; preferably wherein said animal is an equine, more preferably a horse.

11. Use of:
(a) a composition comprising one or more peptides selected from the group consisting of SEQ ID NOs: 18 and 20 and variants thereof having at least 80% identity to SEQ ID NO: 20; and/or comprising one or more polynucleotide sequences selected from the group consisting of SEQ ID NOs: 95 and 152, and variants thereof having at least 80% identity to SEQ ID NO: 95, preferably wherein said one or more peptides selected from the group consisting of SEQ ID Nos: 18 and 20 are linked together as a fusion protein; and/or
(b) a vector encoding said one or more peptides or said fusion protein; or comprising said one or more polynucleotide sequences; and/or
(c) a pharmaceutical composition comprising said one or more peptides and/or said fusion protein and/or said one or more polynucleotide sequences and/or said vector and/or an antibody that binds to said one or more peptides and a pharmaceutically acceptable carrier, vehicle, diluent or excipient; and/or
(d) an antibody that binds to said one or more peptides in a kit or assay for the *in vitro* or *ex vivo* diagnosis an infection by an EIAV and/or NEV in an animal; wherein NEV is deposited under ECAAC accession number 14120201.

## Patentansprüche

1. Eine Zusammensetzung, umfassend ein oder mehrere Peptide, ausgewählt aus der Gruppe bestehend aus SEQ ID NOs: 18 und 20 und Varianten davon mit mindestens 80% Identität zu SEQ ID NO 20; und / oder umfassend eine oder mehrere Polynukleotidsequenzen, ausgewählt aus der Gruppe bestehend aus SEQ ID NOs: 95 und 152 und Varianten davon mit mindestens 80% Identität zu SEQ ID NO:95.

2. Zusammensetzung nach Anspruch 1, wobei sagte eine oder die mehreren Peptide ausgewählt aus der Gruppe bestehend aus SEQ ID NOs: 18 und 20 und sagte Varianten davon als Fusionsprotein miteinander verbunden sind, vorzugsweise wobei zwei beliebige Peptide ausgewählt aus der Gruppe bestehend aus SEQ ID NOs: 18 und 20, und sagte Varianten davon sind als Fusionsprotein miteinander verbunden.

3. Zusammensetzung nach Anspruch 2, ferner wobei sagte Peptide als Fusionsprotein durch ein Linkerpeptid miteinander verbunden sind, wobei das Linkerpeptid vorzugsweise zwischen 2 und 10 Aminosäurereste lang ist, bevorzugter wobei das Linkerpeptid 3 Amino beträgt Säuren in der Länge.

4. Zusammensetzung nach einem der Ansprüche 2 bis 3, wobei sagte Fusionsprotein die Sequenz QEQNPPPSVSLRSLFGNDPLAAAEALDKIEEIQNKNKQK (SEQ ID NO: 158) oder EALDKIEEIQNKNKQKAAAQEQNPPPSVSLRSLFG (SEQ ID) umfasst.

5. Eine Polynukleotidsequenz, die das Fusionsprotein nach einem der Ansprüche 2 bis 4 codiert.

6. Ein Vektor, der ein oder mehrere Peptide codiert, wie in einem der Ansprüche 1 bis 4 definiert, oder ein Vektor, der eine oder mehrere Polynukleotidsequenzen umfasst, wie in Anspruch 1 oder Anspruch 5 definiert.

7. Ein Antikörper, der an SEQ ID NO: 20 bindet.

8. Pharmazeutische Zusammensetzung, umfassend:
ein oder mehrere Peptide, wie in einem der Ansprüche 1 bis 4 definiert; oder
eine oder mehrere Polynukleotidsequenzen gemäß Anspruch 1 oder Anspruch 5;
einen oder mehrere Vektoren nach Anspruch 6; oder
ein oder mehrere Antikörper nach Anspruch 7;
und ein pharmazeutisch verträglicher Träger, Vehikel, Verdünnungsmittel oder Hilfsstoff.

9. Ein Kit bestehend aus:
eine Zusammensetzung nach einem der Ansprüche 1 bis 4; oder
einen Vektor nach Anspruch 6; und / oder
ein Antikörper nach Anspruch 7; und / oder
ein pharmazeutische Zusammensetzung nach Anspruch 8;
und gegebenenfalls Anweisungen zur Verabreichung an ein Tier.

10. Diagnoseverfahren, umfassend das Bereitstellen einer von einem Tier erhaltenen Probe und das Bestimmen der Anwesenheit oder Abwesenheit in der sagte Probe von Antikörpern, die an ein oder mehrere Peptide binden, ausgewählt aus der Gruppe bestehend aus SEQ ID NOs: 18 und 20;
und / oder Bestimmen der Anwesenheit oder Abwesenheit in der sagte Probe eines oder mehrerer der sagte Peptide;
und / oder Bestimmen der Anwesenheit oder Abwesenheit einer oder mehrerer Polynukleotidsequenzen in der sagte Probe, wie in Anspruch 1 oder Anspruch 5 definiert, vorzugsweise wobei: sagte Verfahren das Vorhandensein eines EIAV und / oder NEV in besagten Tier; wobei NEV unter der ECAAC-Zugangsnummer 14120201 hinterlegt ist, vorzugsweise wobei das Tier ein Pferde- ist, bevorzugter ein Pferd.

11. Verwendung von:
a) eine Zusammensetzung, umfassend ein oder mehrere Peptide, ausgewählt aus der Gruppe bestehend aus SEQ ID NOs: 18 und 20 und Varianten davon mit mindestens 80% Identität zu SEQ ID NO: 20; und / oder umfassend eine oder mehrere Polynukleotidsequenzen, ausgewählt aus der Gruppe bestehend aus SEQ ID NOs: 95 und 152, und Varianten davon mit mindestens 80% Identität zu SEQ ID NO: 95, vorzugsweise wobei sagte eine oder die mehreren Peptide ausgewählt aus der Gruppe bestehend von SEQ ID NOs: 18 und 20 sind als Fusionspeptid miteinander verbunden ; und / oder
b) einen Vektor, der sagte eine oder die mehreren Peptide oder sagte Fusionsprotein codiert sagte oder umfassend die eine oder mehreren Polynukleotidsequenzen; und / oder
c) eine pharmazeutische Zusammensetzung, umfassend sagte eine oder die mehreren Peptide und/ oder sagte Fusionsprotein, und/oder sagte eine oder mehreren Polynukleotidsequenzen, und / oder sagte Vektor und / oder ein Antikörper, der an sagte eine oder die mehreren Peptide bindet und ein pharmazeutisch verträglichen Träger, Vehikel, Verdünnungsmittel oder Hilfsstoff bindet; und / oder
d) ein Antikörper, der an sagte eine oder die mehreren Peptide bindet in einem Kit oder Assay für die In-vitro- oder Ex-vivo-Diagnose eine Infektion durch ein EIAV und / oder NEV bei einem Tier wobei NEV unter der ECAAC-Zugangsnummer 14120201 hinterlegt ist.

## Revendications

1. Une composition comprenant un ou plusieurs peptides sélectionnés dans le groupe constitué des SEQ ID NO: 18 et 20 et leurs variants ayant au moins 80% d'identité avec SEQ ID NO: 20; et / ou comprenant une ou plusieurs séquences polynucléotidiques sélectionnées dans le groupe consistant en SEQ ID NO: 95 et 152, et leurs variantes ayant au moins 80% d'identité avec SEQ ID NO: 95.

2. Composition selon la revendication 1, dans laquelle lesdits un ou plusieurs peptides sélectionnés dans le groupe consistant en SEQ ID NO: 18 et 20 et lesdits variants de ceux-ci sont liés ensemble en tant que protéine de fusion, de préférence dans laquelle deux peptides quelconques sélectionnés dans le groupe constitué des SEQ ID NO: 18 et 20, et lesdits variants de ceux-ci sont liés ensemble en tant que protéine de fusion.

3. Composition selon la revendication 2, dans laquelle en outre lesdits peptides sont liés ensemble en tant que protéine de fusion par un peptide de liaison, de préférence dans lequel ledit peptide de liaison a une longueur comprise entre 2 et 10 résidus d'acides aminés, plus préférablement dans lequel ledit peptide de liaison a une longueur de 3 acides aminés.

4. Composition selon l'une quelconque des revendications 2 à 3, dans laquelle ladite protéine de fusion comprend la séquence QEQNPPPSVSLRSLFGNDPLAAAEALDKIEEIQNKNKQK (SEQ ID NO: 158) ou EALDKIEEIQNKNKQKAAAQEQNPPPSVSLRSLFG (SEQ ID NO: 159).

5. Séquence polynucléotidique codant pour la protéine de fusion selon l'une quelconque des revendications 2 à 4.

6. Vecteur qui code pour un ou plusieurs peptides tels que définis dans l'une quelconque des revendications 1 à 4, ou un vecteur comprenant une ou plusieurs séquences polynucléotidiques telles que définies dans la revendication 1 ou la revendication 5.

7. Un anticorps qui se lie à SEQ ID NO: 20.

8. Une composition pharmaceutique comprenant:
un ou plusieurs peptides tels que définis dans l'une quelconque des revendications 1 à 4; ou
une ou plusieurs séquences polynucléotidiques telles que définies dans la revendication 1 ou la revendication 5; ou
un ou plusieurs vecteurs selon la revendication 6; ou
un ou plusieurs anticorps selon la revendication 7;
et un support, véhicule, diluant ou excipient pharmaceutiquement acceptable.

9. Un kit comprenant:
une composition selon l'une quelconque des revendications 1 à 4; ou
un vecteur selon la revendication 6; et / ou
un anticorps selon la revendication 7; et / ou
une composition pharmaceutique selon la revendication 8;
et éventuellement des instructions pour l'administration à un animal.

10. Procédé de diagnostic comprenant la fourniture d'un échantillon obtenu à partir d'un animal et la détermination de la présence ou de l'absence dans ledit échantillon d'anticorps qui se lient à un ou plusieurs peptides sélectionnés dans le groupe constitué par les SEQ ID NO: 18 et 20;
et / ou déterminer la présence ou l'absence dans ledit échantillon d'un ou plusieurs desdits peptides; et / ou déterminer la présence ou l'absence dans ledit échantillon d'une ou plusieurs séquences polynucléotidiques telles que définies dans la revendication 1 ou la revendication 5, de préférence dans lequel: ladite méthode détermine la présence d'un EIAV et / ou NEV dans ledit animal; dans lequel NEV est déposé sous le numéro d'accès ECAAC 14120201, de préférence dans lequel ledit animal est un équidé, plus préférablement un cheval.

11. Utilisation de:
a) une composition comprenant un ou plusieurs peptides choisis dans le groupe consistant en SEQ ID NO: 18 et 20 et leurs variantes ayant au moins 80% d'identité avec SEQ ID NO: 20; et / ou comprenant une ou plusieurs séquences polynucléotidiques choisies dans le groupe consistant en SEQ ID NO: 95 et 152, et leurs variantes ayant au moins 80% d'identité avec SEQ ID NO: 95, de préférence dans laquelle ledit un ou plusieurs peptides sont choisis dans le groupe consistant en SEQ ID NO: 18 et 20 sont liés ensemble en tant que peptide de fusion; et / ou
b) un vecteur codant pour lesdits un ou plusieurs peptides ou ladite protéine de fusion; ou comprenant lesdites une ou plusieurs séquences polynucléotidiques; et / ou
c) une composition pharmaceutique comprenant lesdits un ou plusieurs peptides et / ou ladite protéine de fusion et / ou ladite une ou plusieurs séquences polynucléotidiques et / ou ledit vecteur et / ou un anticorps qui se lie auxdits un ou plusieurs peptides et un support pharmaceutiquement acceptable, véhicule, diluant ou excipient; et / ou
d) un anticorps qui se lie auxdits un ou plusieurs peptides
dans un kit ou test pour le diagnostic in vitro ou ex vivo d'une infection par un EIAV et / ou NEV chez un animal; dans lequel NEV est déposé sous le numéro d'accès ECAAC 14120201.
